# EUROPEAN PATENT APPLICATION

(11) **EP 4 843 612 A2**
(43) Date of publication of application: **25.11.2026**
(21) Application number: 26210091.0
(22) Date of filing: 17.10.2018
(51) Int. Cl.: A61N 1/375

(54) **HIS BUNDLE AND BUNDLE BRANCH PACING ADJUSTMENT**

(30) Priority: 17.10.2017 US 201762573685 P; 03.11.2017 US 201762581486 P; 12.01.2018 US 201862617059 P
(62) Document number(s) of the earlier application(s) in accordance with Art. 76 EPC: 24197884.0 / 4 445 945; 18800375.0 / 3 697 495
(71) Applicant: Medtronic, Inc., Minneapolis, MN 55432-5604 (US)
(72) Inventor: ZHOU, Xiaohong, Minneapolis, 55432 (US)
(74) Representative: Zimmermann & Partner Patentanwälte mbB

(57) **Abstract**

The present disclosure relates generally to pacing of cardiac tissue, and more particularly to adjusting delivery of His bundle or bundle branch pacing in a cardiac pacing system to achieve synchronized ventricular activation. Bundle pacing may be delivered in response to determining whether the QRS parameter or activation interval is greater than a threshold. A set of AV delays may be generated, and an optimal AV delay may be selected from the stored set of AV delays. His-bundle or bundle-branch pacing may be selectively delivered based on RV or LV activation time. Pacing may also be adjusted based on dyssynchrony detected or the type of bundle branch block pattern detected.

## Description

### CROSS-REFERENCE TO RELATED APPLICATIONS

This application claims the benefit of U.S. Provisional Application No. 62/573,685, filed October 17, 2017, the benefit of U.S. Provisional Application No. 62/581,486, filed November 3, 2017, and the benefit of U.S. Provisional Application No. 62/617,059, filed January 12, 2018, which are incorporated herein in their entireties.

The present disclosure relates generally to pacing of cardiac tissue, and more particularly, to pacing using the conduction system of the heart, such as the His bundle.

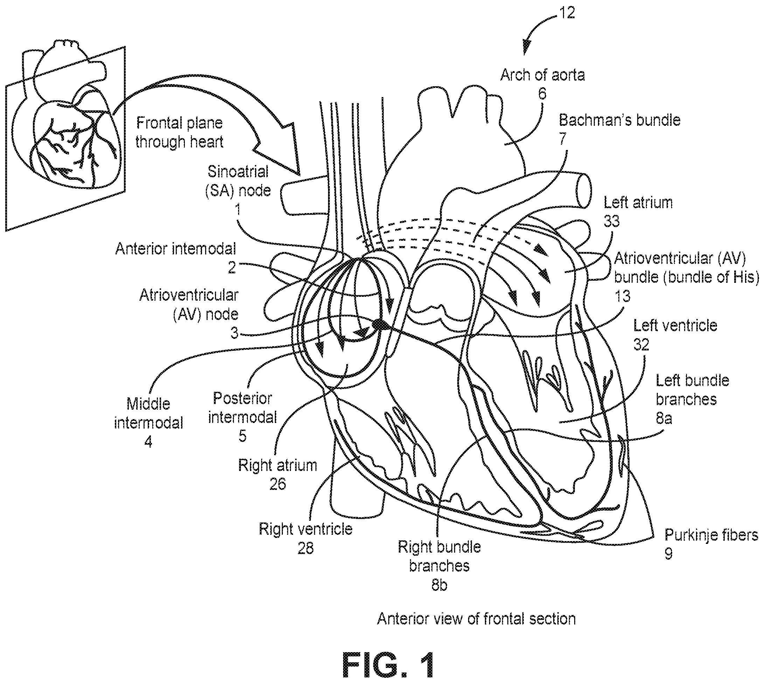

Implantable medical devices (IMDs), such as cardiac pacemakers or implantable cardioverter defibrillators, deliver therapeutic stimulation to patients' hearts thereby improving the lives of millions of patients living with heart conditions. Conventional pacing techniques involve pacing one or more of the four chambers of patient's heart **12**-left atrium (LA) **33,** right atrium (RA) **26,** left ventricle (LV) **32** and right ventricle (RV) **28,** all of which are shown in **FIG. 1****.** One common conventional therapeutic pacing technique that treats a slow heart rate, referred to as Bradycardia, involves delivering an electrical pulse to a patient's right ventricular tissue. In response to the electrical pulse, both the right and left ventricles contract. However, the heart beat process may be significantly delayed because the pulse travels from the right ventricle through the left ventricle. The electrical pulse passes through the muscle cells that are referred to as myocytes. Myocyte-to-myocyte conduction may be very slow. Delayed electrical pulses can cause the left ventricle to be unable to maintain synchrony with the right ventricle.

Over time, the left ventricle can become significantly inefficient at pumping blood to the body. In some patients, heart failure can develop such that the heart is too weak to pump blood to the body. Heart failure may be a devastating diagnosis since, for example, fifty percent of the heart failure patients have a life expectancy of five years. To avoid the potential development of heart failure, some physicians have considered alternative pacing methods that involve the cardiac conduction system. The cardiac conduction system, like a "super highway," may be described as quickly conducting electrical pulses whereas pacing cardiac muscle tissue may slowly conduct electrical pulses, like "traveling on a dirt road."

The cardiac conduction system includes sinoatrial node (SA node) **1,** atrial internodal tracts **2, 4, 5** (i.e., anterior internodal **2,** middle internodal **4,** and posterior internodal **5),** atrioventricular node (AV node) **3,** His bundle **13** (also known as atrioventricular bundle or bundle of His), and right and left bundle branches **8a, 8b.** **FIG. 1** also shows the arch of aorta **6** and Bachman's bundle **7.** The SA node, located at the junction of the superior vena cava and right atrium, is considered to be the natural pacemaker of the heart since it continuously and repeatedly emits electrical impulses. The electrical impulse spreads through the muscles of right atrium **26** to left atrium **33** to cause synchronous contraction of the atria. Electrical impulses are also carried through atrial internodal tracts to atrioventricular (AV) node **3** - the sole connection between the atria and the ventricles. Conduction through the AV nodal tissue takes longer than through the atrial tissue, resulting in a delay between atrial contraction and the start of ventricular contraction. The AV delay, which is the delay between atrial contraction and ventricular contractor, allows the atria to empty blood into the ventricles. Then, the valves between the atria and ventricles close before causing ventricular contraction via branches of the bundle of His. His bundle **13** is located in the membranous atrioventricular septum near the annulus of the tricuspid valve. His bundle **13** splits into right and left bundle branches **8a, 8b** and are formed of specialized fibers called "Purkinje fibers" **9.** Purkinje fibers **9** may be described as rapidly conducting an action potential down the ventricular septum (VS), spreading the depolarization wavefront quickly through the remaining ventricular myocardium, and producing a coordinated contraction of the ventricular muscle mass.

While His bundle pacing is increasingly used as an alternative to traditional pacing techniques, His bundle pacing has not been widely adopted for a variety of reasons. For example, His pacing electrodes should be positioned within a precise target location (e.g., within about 1 millimeter) of the His bundle, which may be difficult. Further, some physicians insert the pacing electrode in a manner that damages the His bundle thereby causing complete block or bundle branch block timing of delivery of pacing pulses not being optimal. Additionally, some physicians place the His lead in a proximal His bundle position that requires a much higher pacing output to be delivered by the pacemaker to effectively pace the His bundle. It is desirable to develop new methods and systems of His bundle pacing that overcome some of the disadvantages associated with His bundle pacing alone or in combination with other pacing therapies.

### SUMMARY

The techniques of this disclosure generally relate to pacing the special cardiac conductive system, e.g., the His-Purkinje system, including His bundle and bundle branches. A conduction block can either exist due to disease progression, such as AV block or infra-Hisian block, or appear during the lead implantation procedure that causes bundle injury or otherwise affects bundle conduction. In such cases, conventional His bundle pacing may not work properly. For example, activation can propagate via one bundle branch but not the other one. Thus, this disclosure provides bundle branch pacing by bypassing the block region and algorithms that allow better pacing timing and output to provide a better ventricular synchronization than conventional His bundle pacing. For example, if a pacing lead is placed near the left bundle branch and a low pacing output only excites the left bundle branch but not the right bundle branch, an algorithm of the present disclosure will adjust pacing timing to synchronize the activation generated by pacing with intrinsic activation via the right bundle branch, which may result in a normal ventricular synchronization. In another example, if the lead placement damages or otherwise affects the right bundle branch and can only stimulate the left bundle branch, which may lead to an electrocardiogram (ECG) representing a right bundle branch block, or a sign of ventricular dyssynchrony, then a new lead of the present disclosure may be used to stimulate both left and right bundle branches at the same time to achieve synchronized right and left ventricular activation.

In one aspect, the present disclosure provides a method of using a pacemaker for His-bundle or bundle-branch pacing of a patient's heart. The method includes sensing heart activity using a set of electrodes associated with the pacemaker. The method also includes using a processor in the pacemaker to determine whether one of a QRS parameter and an activation interval acquired from the sensed heart activity is greater than a first threshold. The method also includes, in response to determining whether the QRS parameter or activation interval is greater than the first threshold, using the processor to determine whether bundle pacing should be delivered earlier or later. Further, the method includes, in response to determining whether bundle pacing should be delivered earlier or later, using the processor to adjust a time interval for bundle pacing.

In another aspect, the present disclosure provides a method of using an implantable medical device having a processor and an electrical pulse generator. The method includes using the processor to control the electrical pulse generator to adjust delivery of bundle pacing. The processor is configured to measure a QRS parameter and determine whether the QRS parameter is larger than a threshold. The processor is further configured to, in response to determining that the QRS parameter is greater than or equal to the threshold, adjust a bundle-pacing to ventricular-activation interval or a bundle pacing output level. The processor is further configured to deliver bundle pacing using the adjusted bundle-pacing to ventricular-activation interval or using the adjusted bundle pacing output level.

In another aspect, the present disclosure provides an implantable medical device for His-bundle or bundle-branch pacing of a patient's heart. The device includes a sensing circuit configured to sense an atrial event (As) and a ventricular event (Vs). The device also includes an electrical pulse generator configured to generate and deliver electrical His-bundle or bundle-branch stimulation pulses (Vp). The device also includes a processor coupled to the sensing circuit and the electrical pulse generator and configured to control the electrical pulse generator to deliver said Vp on expiration of a defined AV interval or Vp-Vs interval following a said As or Vs. The processor is further configured to determine whether Vp timing should be adjusted. The processor is also configured to, in response to determining that the Vp timing should be adjusted, determine whether Vp should be delivered earlier or later. The processor is also configured to, in response to determining whether the Vp should be delivered earlier or later, decrease an AV delay if the Vp should be delivered earlier or increase the AV delay in response to determining Vp should be delivered later. Further, the processor is configured to generate a set of AV delays that are stored in memory based on iterating through decreases or increases in the AV delay. The processor is further configured to select an optimal AV delay from the set of AV delays stored in memory. The processor is also configured to use the optimal AV delay when delivering His-bundle or bundle-branch pacing.

In another aspect, the present disclosure provides an implantable medical device for His-bundle or bundle-branch pacing of a patient's heart. The device includes a sensing circuit configured to sense an atrial event (As) and a ventricular event (Vs). The device also includes an electrical pulse generator configured to generate and deliver electrical His-bundle or bundle-branch stimulation pulses (Vp). The device further includes a processor coupled to the sensing circuit and the electrical pulse generator and configured to control the electrical pulse generator to deliver said Vp on expiration of a defined AV interval or Vp-Vs interval following a said As or Vs. The processor is further configured to determine whether RV activation occurs earlier than LV activation. The processor is also configured to, in response to determining RV activation occurs earlier than LV activation, adjust Vp timing to deliver His-bundle or bundle-branch pacing based on an LV activation time.

In another aspect, the present disclosure provides an implantable medical device for His-bundle or bundle-branch pacing of a patient's heart. The device includes a sensing circuit configured to sense an atrial event (As) and a ventricular event (Vs). The device also includes an electrical pulse generator configured to generate and deliver electrical His-bundle or bundle-branch stimulation pulses (Vp). The device also includes a processor coupled to the sensing circuit and the electrical pulse generator and configured to control the electrical pulse generator to deliver said Vp on expiration of a defined AV interval or Vp-Vs interval following a said As or Vs. The processor is further configured to determine whether RV activation occurs earlier than LV activation. The processor is also configured to, in response to determining that RV activation does not occur earlier than LV activation, determine whether RV activation occurs later than LV activation. The processor is also configured to, in response to determining RV activation occurs later than LV activation, adjust Vp timing to deliver His-bundle or bundle-branch pacing based on an RV activation time.

In another aspect, the present disclosure provides a method of using a pacemaker for His-bundle or bundle-branch pacing of a patient's heart. The method includes sensing electrical activity of the patient's heart including an atrial event (As) and a ventricular event (Vs). The method also includes detecting dyssynchrony based on the sensed electrical activity of the patient's heart during the patient's intrinsic rhythm or during pacing. The method further includes adjusting delivery of His-bundle or bundle-branch stimulation pulses (Vp) based on the dyssynchrony detected.

In another aspect, the present disclosure provides, a method of using a pacemaker for His-bundle or bundle-branch pacing of a patient's heart. The method includes sensing electrical activity of the patient's heart including an atrial event (As) and a ventricular event (Vs). The method also includes detecting a right bundle branch block (RBBB) pattern or left bundle branch block (LBBB) pattern in the sensed electrical activity of the patient's heart during the patient's intrinsic rhythm or during pacing. The method further includes adjusting the delivery of His-bundle or bundle-branch stimulation pulses (Vp) based on the detected bundle-branch-block pattern.

In another aspect, the present disclosure provides an implantable medical device for bundle-branch pacing of a patient's heart. The device includes a plurality of electrodes. The plurality of electrodes includes a left bundle branch (LBB) cathode electrode positionable, when implanted, on a left side of the septum of the patient's heart proximate to the LBB. The plurality of electrodes also includes a right bundle branch (RBB) cathode electrode positionable, when implanted, implantable on a right side of the septum of the patient's heart proximate to the RBB. The device includes an electrical pulse generating circuit coupled to the plurality of electrodes to generate and deliver electrical bundle-branch stimulation pulses (Vp). The device also includes a processor coupled to the electrical pulse generating circuit. The processor is configured to control the electrical pulse generating circuit to deliver synchronized left and right bundle-branch Vp using the plurality of electrodes based on one or both of an atrial event (As) and a ventricular event (Vs).

In another aspect, the present disclosure provides a method of using a pacemaker for bundle-branch pacing of a patient's heart. The method includes sensing electrical activity of the patient's heart including an atrial event (As) and a ventricular event (Vs). The method also includes delivering synchronized left and right bundle-branch stimulation pulses (Vp) using left and right bundle-branch cathode electrodes based on one or both of As and Vs. The left bundle branch (LBB) cathode electrode is implantable on a left side of the septum of the patient's heart proximate to the LBB of the patient's heart. The right bundle branch (RBB) cathode electrode is implantable on a right side of the septum of the patient's heart proximate to the RBB.

In another aspect, the present disclosure provides a method of positioning a bundle pacing electrode in a patient's heart. The method includes monitoring electrical activity of the patient's heart using one or both of an external electrode and a bundle pacing electrode on a bundle pacing lead or leadless device. The method also includes rotating or advancing the bundle pacing lead or leadless device toward the His bundle or bundle branch of the patient's heart while intermittently applying pacing with the bundle pacing electrode. The method also includes determining whether an RBBB pattern is present based on the monitored electrical activity. Further, the method includes determining whether the patient's heart has a right bundle branch block (RBBB). The method also includes, in response to determining that an RBBB pattern is present during applied pacing and that the patient's heart does not have a RBBB, fixing the bundle pacing lead or leadless device for left bundle branch pacing.

In another aspect, the present disclosure provides a leadless pacing device (LPD) for His-bundle or bundle-branch pacing in a patient's heart. The LPD includes an intracardiac housing and a plurality of electrodes. The plurality of electrodes includes a bundle pacing electrode leadlessly connected to the housing and implantable proximate to or in the His bundle or bundle branch of the patient's heart. The LPD also includes a sensing circuit operably coupled to the plurality of electrodes and configured to sense one or both of an atrial event (As) and a ventricular event (Vs) using at least one of the plurality of electrodes. Further, the LPD includes an electrical pulse generator coupled to the bundle pacing electrode, the electrical pulse generator configured to generate and deliver electrical His-bundle or bundle-branch stimulation pulses (Vp) based on one or both of As and Vs to the patient's heart using the bundle pacing electrode.

In another aspect, the present disclosure provides a leadless pacing device (LPD) for His-bundle or bundle-branch pacing in a patient's heart. The LPD includes an intracardiac housing. The LPD also includes a communication interface disposed in the housing configured to receive signals from a subcutaneously implanted device (SD). The LPD further includes a controller disposed in the housing operatively coupled to at least one electrode configured to deliver His-bundle or bundle-branch pacing pulses to the patient's heart in response to the received signals.

In another aspect, the present disclosure provides a method of His-bundle or bundle-branch pacing. The method includes using a subcutaneously implanted device (SD) to sense far-field electrical signals. The method also includes determining a QRS parameter or activation based on the far-field electrical signals. The method also includes communicating a timing to a leadless pacing device (LPD) to deliver His-bundle or bundle-branch pacing using an atrial-activation to bundle-pacing interval.

### BRIEF DESCRIPTION

**FIG. 1** is a schematic diagram of a heart of patient (prior art).
**FIG. 2A** is a conceptual diagram illustrating an example therapy system (e.g., triple-chamber implantable medical device) that is configured to provide therapy to a heart of patient through a His-bundle or bundle-branch pacing lead and lead placed either in the right ventricle or the right atrium using an implantable medical device (IMD).
**FIG. 2B** is a schematic diagram illustrating an example His-bundle or bundle-branch pacing lead positioned in bundle of the His in a cross-sectional view of the heart.
**FIG. 3A** is a conceptual diagram illustrating an example therapy system (e.g., dual-chamber implantable medical device) that is configured to provide therapy to a heart of patient suffering from atrial fibrillation through a His-bundle or bundle-branch pacing lead and lead placed in the left ventricle using an IMD.
**FIG. 3B** is a schematic diagram illustrating an example of a His-bundle or bundle-branch pacing lead positioned in bundle of the His in a cross-sectional view of the heart using an IMD.
**FIG. 4** is a conceptual diagram illustrating an example of a therapy system (e.g., dual chamber implantable medical device) that is configured to provide therapy to a heart of patient through a His-bundle or bundle-branch pacing lead and lead placed in the left ventricle using an IMD.
**FIG. 5** is a functional block diagram illustrating an example of a configuration of an implantable medical device of **FIG. 2A****, 3A-4,** or **15-18B.**
**FIG. 6** is a functional block diagram illustrating an example of a configuration of a programmer used during the procedure of implanting a medical device in a patient or interrogating the implantable device during a follow-up visit at a hospital.
**FIG. 7** is a functional block diagram illustration an example configuration of a system including a network for use with, for example, the IMD of **FIG. 5****.**
**FIG. 8A** depicts the His bundle and left and right bundle branches in which intrinsic rhythm (also referred to as native rhythm) is acquired from a set of electrodes without His bundle pacing being delivered.
**FIG. 8B** depicts proximal His bundle pacing being delivered proximal to the block.
**FIG. 8C** depicts distal His bundle pacing being delivered distal to the block.
**FIG. 8D** depicts low output bundle pacing being delivered to the left bundle branch to correct left bundle branch block (LBBB) and/or right bundle branch block (RBBB).
**FIG. 9A** graphically depicts unipolar ECG signals of intrinsic conduction (native rhythm) acquired from a set of electrodes disposed over cardiac tissue including the His bundle.
**FIG. 9B** graphically depicts unipolar ECG signals, acquired from a set of electrodes, in response to proximal His bundle pacing being delivered at a low pacing output.
**FIG. 9C** graphically depicts unipolar ECG signals, acquired from a set of electrodes, in response to proximal His bundle pacing being delivered with a high or increased pacing output compared to the pacing output delivered in **FIG. 9B****.**
**FIG. 10A** graphically depicts unipolar ECG signals of intrinsic conduction (native rhythm) acquired from a set of electrodes disposed over cardiac tissue including the His bundle.
**FIG. 10B** graphically depicts unipolar ECG signals, acquired from a set of electrodes, in response to distal His bundle pacing being delivered at low pacing output.
**FIG. 11A** graphically depicts unipolar ECG signals of intrinsic conduction (native rhythm) acquired from a set of electrodes disposed over cardiac tissue including the His bundle.
**FIG. 11B** graphically depicts unipolar ECG signals, acquired from a set of electrodes, responsive to bundle pacing being delivered to the left bundle branch using an atrioventricular delay of 40 milliseconds (ms).
**FIG. 11C** graphically depicts unipolar ECG signals acquired from a set of electrodes responsive to bundle pacing being delivered to the left bundle branch using an atrioventricular delay of 100 ms.
**FIG. 12** is a flow diagram of an illustrative process of determining an optimal atrioventricular (AV) delay for delivering bundle pacing with an implantable medical device.
**FIG. 13** is a flow diagram of another illustrative process of determining an optimal AV delay for delivering bundle pacing with an implantable medical device.
**FIG. 14** is a flow diagram of an illustrative process of determining an optimal interventricular (VV) delay for delivering bundle pacing with an implantable medical device.
**FIG. 15** is a conceptual drawing illustrating an example system that includes a subcutaneous implantable cardioverter defibrillator (SICD) implanted exterior to the rib cage of a patient and a leadless pacing device (LPD) implanted within a cardiac chamber of the patient.
**FIG. 16** is a conceptual drawing illustrating the example LPD of **FIG. 15****.**
**FIG. 17A** is a front view of a patient implanted with an implantable cardiac system that includes a subcutaneous device and a leadless pacing device.
**FIG. 17B** is a side view of a patient with the implantable cardiac system shown in **FIG. 17A****.**
**FIG. 18A** is a cross-sectional view of a patient's heart implanted with an implantable medical electrical lead to deliver bundle branch pacing.
**FIG. 18B** is a close-up view of the lead in the patient's heart of **FIG. 18A****.**
**FIGS. 19A-D** graphically depict electrogram (EGM) signals (at the top) and unipolar ECG signals (at the bottom) representing conduction using various intrinsic or pacing configurations.
**FIG. 19E** depicts a portion of the patient's heart having a left bundle branch block (LBBB) with intrinsic conduction (native rhythm).
**FIG. 19F** depicts a portion of the patient's heart in which pacing is applied distal to the block with an AV delay of 40 ms.
**FIG. 19G** depicts a portion of the patient's heart in which pacing is applied distal to the block with an AV delay of 90 ms.
**FIG. 19H** depicts a portion of the patient's heart in which pacing is applied distal to the block with an AV delay of 160 ms.
**FIG. 20** graphically depicts unipolar ECG signals, acquired from a set of electrodes, in response to various intrinsic or pacing configurations for a patient with an LBBB and resulting QRS duration.
**FIG. 21** graphically depicts unipolar ECG signals, acquired from a set of electrodes, in response to various intrinsic or pacing configurations for a patient with an RBBB and resulting QRS durations.
**FIG. 22A** schematically depicts a lead configured to deliver bundle branch pacing through a helical tip electrode (i.e., cathode) and a ring electrode (i.e., cathode) while the anode is remotely positioned on the housing of the implantable medical device (i.e., pacemaker, ICD).
**FIG. 22B** schematically depicts a lead configured to deliver bundle branch pacing with a shield, proximal to the helical tip electrode, to prevent the helical tip from being "over screwed" into tissue.
**FIG. 23** is a flow diagram showing one illustrative method for implanting an IMD for His bundle or bundle branch pacing.
**FIG. 24** schematically depicts an implantable medical device (IMD) including a first and second pacing electrodes (i.e., cathodes) near a distal end portion of the IMD and a ring electrode (i.e., anode) located at a proximal end portion of the IMD.
**FIGS. 25-27** are end views of a distal end of various IMDs, such as the IMD of **FIG. 24****.**
**FIGS. 28A-B** depict a cross-sectional view of a lead connection to an implantable medical device that shows a lead connector before and after placement in the connector.
**FIG. 29** is a flow diagram for a method of His bundle pacing adjustment post-implantation.
**FIG. 30** is a flow diagram for a method of His bundle pacing adjustment post-implantation.
**FIG. 31** is a flow diagram for a method of bundle branch pacing adjustment post-implantation.

### DETAILED DESCRIPTION

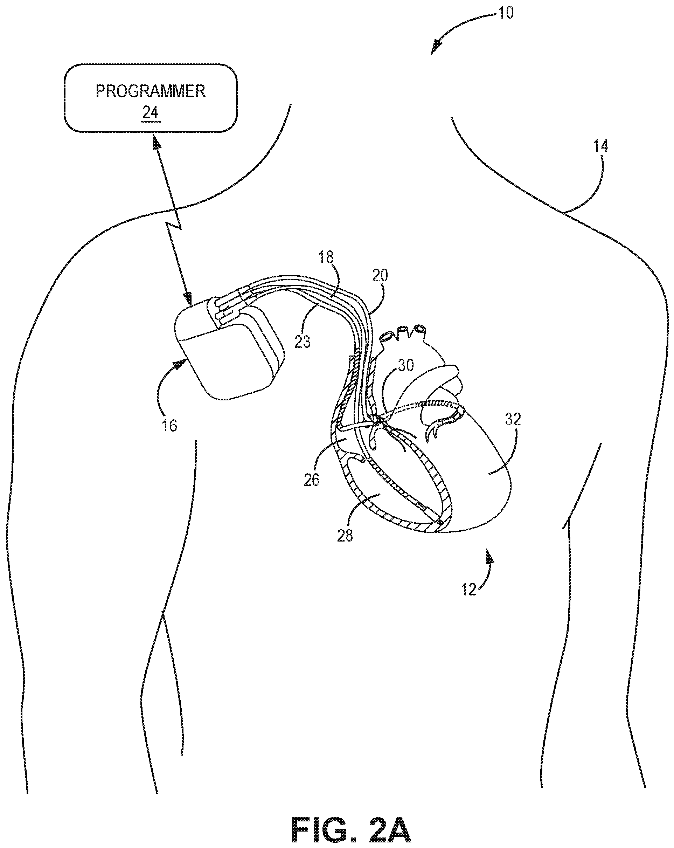

**FIGS. 2A-B** are conceptual diagrams illustrating one example therapy system **10** that may be used to provide therapy to heart **12** of patient **14.** Patient **14** ordinarily, but not necessarily, will be a human. Therapy system **10** includes IMD **16,** which is coupled to leads **18, 20, 23** and programmer **24.** IMD **16** may be, for example, an implantable pacemaker, cardioverter, and/or defibrillator that provides electrical signals to heart **12** via electrodes coupled to one or more of leads **18, 20, 23.** Further non-limiting examples of IMD **16** include: a pacemaker with a medical lead, an implantable cardioverter-defibrillator (ICD), an intracardiac device, a leadless pacing device (LPD), a subcutaneous ICD (S-ICD), and a subcutaneous medical device (e.g., nerve stimulator, inserted monitoring device, etc.).

Leads **18, 20, 23** extend into heart **12** of patient **14** to sense electrical activity of heart **12** and/or deliver electrical stimulation to heart **12.** In the example shown in **FIG. 2A****,** right ventricular (RV) lead **18** extends through one or more veins (not shown), the superior vena cava (not shown), and right atrium (RA) **26,** and into right ventricle **28.** Left ventricular (LV) coronary sinus lead **20** extends through one or more veins, the vena cava, right atrium **26,** and into the coronary sinus **30** to a region adjacent to the free wall of left ventricle **32** of heart **12.** Bundle pacing lead **23** (e.g., His-bundle or bundle-branch pacing lead) extends through one or more veins and the vena cava, and into the right atrium **26** of heart **12** to pace the His bundle or one or both bundle branches. In some embodiments, bundle pacing lead **23** may be positioned within about 1 millimeter of the His bundle or one or both bundle branches.

As used herein, the term "bundle pacing" refers to His bundle pacing, bundle branch pacing, or both His bundle pacing and bundle branch pacing. Bundle branch pacing refers to pacing one or both of the left and right bundle branches of the patient's heart **12.**

As used herein, the term "activation" refers to a sensed or paced event. For example, an atrial activation may refer to an atrial sense or event (As) or an atrial pace or artifact of atrial pacing (Ap). Similarly, a ventricular activation may refer to a ventricular sense or event (Vs) or a ventricular pace or artifact of ventricular pacing (Vp), which may be described as ventricular stimulation pulses. In some embodiments, activation interval can be detected from As or Ap to Vs or Vp, as well as Vp to Vs. In particular, activation intervals may include a pacing (Ap or Vp) to ventricular interval (LV or RV sense) or an atrial-sensing (As) to ventricular-sensing interval (LV or RV sense).

One example of bundle pacing lead **23** (e.g., a His lead) can be the SELECTSURE^{™} 3830. A description of the SELECTSURE^{™} 3830 is found in the Medtronic model SELECTSURE^{™} 3830 manual (2013), incorporated herein by reference in its entirety. The SELECTSURE^{™} 3830 includes two or more conductors with or without lumens.

An elongated conductor of the lead may extend through a hermetic feedthrough assembly (not shown), and within an insulative tubular member of the lead, and may electrically couple an electrical pulse generator (contained within housing) to the helical tip of bundle pacing lead **23.** The conductor may be formed by one or more electrically conductive wires, for example, MP35N alloy known to those skilled in the art, in a coiled or cabled configuration, and the insulative tubular member may be any suitable medical grade polymer, for example, polyurethane, silicone rubber, or a blend thereof. According to an illustrative embodiment, the flexible lead body extends a pre-specified length (e.g., about 10 centimeters (cm) to about 20 cm, or about 15 to 20 cm) from a proximal end of housing to the other end. The lead body is less than about **7** French (FR) but typically in the range of about 3 to 4 FR in size. In one or more embodiments, about 2 to about 3 FR size lead body is employed.

His bundle pacing can be performed by other leads. Another illustrative lead, including two or more pacing electrodes, can be used to deliver multisite pacing pulses to the bundle of His or one or both bundle branches. Multisite pacing can be delivered simultaneously or sequentially, as described and shown by U.S. Patent Publication No. 2016/0339248, filed on April 21, 2016, entitled EFFICIENT DELIVERY OF MULTI-SITE PACING, the disclosure of which is incorporated by reference in its entirety.

Multi-site or multipoint pacing may be delivered by simultaneous or sequential stimulation of two or more electrodes located in the same chamber (e.g., LV). Simultaneous pacing is defined as pacing from two or more electrodes where the timing delay between electrodes is less than about 50, 30, 10, or even 5 ms. In some embodiments, the timing delay between electrodes is greater than 1 ms. In one or more embodiments, the timing delay between electrodes is about 5 ms.

Since the electrodes in multi-site or multi-point stimulation are in close proximity to each other, it may be important to detect and verify effective capture of individual electrodes during delivery of such therapy. Delivering multisite pacing pulses may include delivering pacing pulses to a first tissue site and a second tissue site through first and second pacing electrodes, respectively, all of which may occur within the same cardiac cycle.

In particular, a lead configured to perform multi-site pacing, which is different than LV coronary sinus lead **20,** can be placed in the ventricular septum with the first (distal) electrode on the left side of the ventricular septum for left bundle branch pacing and with the second electrode (proximal) on the right side of the septum for pacing the right bundle branch. An interelectrode distance may be defined as the distance between the first and second electrodes, or the distance that the electrodes are apart. In some embodiments, the interelectrode distance is at least about 3, 4, 5, 6, 7, or 8 millimeters (mm). In some embodiments, the interelectrode distance is at most about 15, 14, 13, 12, 11, or 10 mm. For example, the interelectrode distance may be in a range from about 6 to 12 mm apart. Once the pacing is delivered, both the left bundle branch and the right bundle branch may be stimulated such that both ventricles are naturally or near-naturally synchronized. In contrast, in traditional CRT, the ventricles may be described as not naturally synchronized.

A single lead, including two (or more) pacing electrodes (e.g., cathodes) may deliver cathode pacing outputs at two separate locations (e.g., left and right bundle branches), so both bundle branches can be excited at the same time.

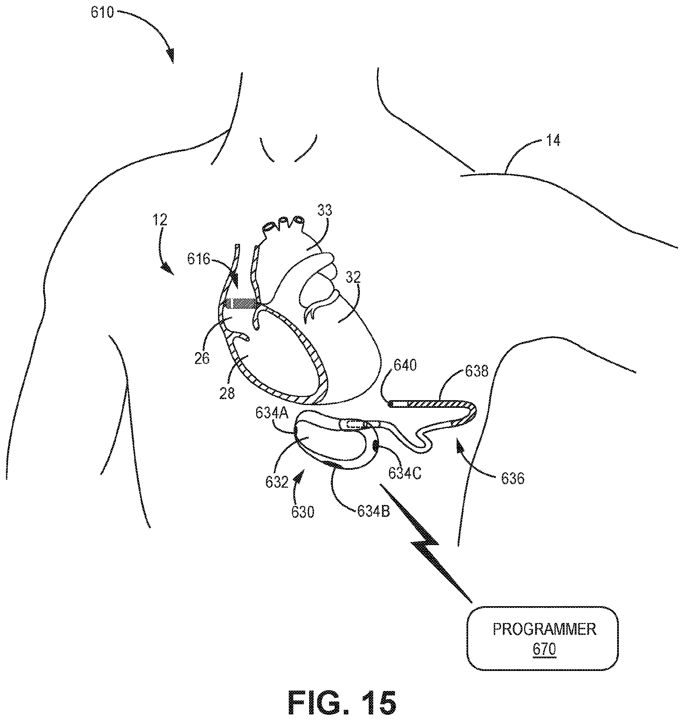

His bundle pacing, though a leading candidate for physiological pacing, may be hard to implant, may have a relatively high pacing threshold, and may have an unstable long-term pacing threshold in patients with conduction disease. Bundle branch pacing may bypass the pathological region and may have a low and stable pacing threshold. In some embodiments, only one bundle branch may be paced by using pacing leads. One aspect of this disclosure provides pacing of both bundle branches at the same time (e.g., dual bundle branch pacing), which may mimic intrinsic activation propagation via the His bundle-Purkinje conduction system, e.g., paced activation propagates via both bundle branches to both ventricles for synchronized contraction. Traditional His bundle pacing, on the other hand, typically paces the His bundle proximal to the bundle branches. In some embodiments, IMD **16** may include one, two, or more electrodes located in one or more bundle branches configured for bundle branch pacing. In some embodiments, IMD **16** may be an intracardiac pacemaker or leadless pacing device (LPD), such as LPD **616 (****FIGS. 15-16****).**

As used herein, "leadless" refers to a device being free of a lead extending out of patient's heart **12.** In other words, a leadless device may have a lead that does not extend from outside of the patient's heart to inside of the patient's heart. Some leadless devices may be introduced through a vein, but once implanted, the devices are free of, or may not include, any transvenous lead and may be configured to provide cardiac therapy without using any transvenous lead. In one or more embodiments, an LPD for bundle pacing does not use a lead to operably connect to an electrode disposed proximate to the septum when a housing of the device is positioned in the atrium. A leadless electrode may be leadlessly coupled to the housing of the medical device without using a lead between the electrode and the housing.

IMD **16** may sense electrical signals attendant to the depolarization and repolarization of heart **12** via electrodes (not shown in **FIGS. 2A-B)** coupled to at least one of leads **18, 20, 23.** In some examples, IMD **16** provides pacing pulses to heart **12** based on the electrical signals sensed within heart **12.** The configurations of electrodes used by IMD **16** for sensing and pacing may be unipolar or bipolar. IMD **16** may also provide defibrillation therapy and/or cardioversion therapy via electrodes located on at least one of leads **18, 20, 23.** IMD **16** may detect arrhythmia of heart **12,** such as fibrillation of ventricles **28** and **32,** and deliver defibrillation therapy to heart **12** in the form of electrical pulses. In some examples, IMD **16** may be programmed to deliver a progression of therapies, e.g., pulses with increasing energy levels, until a fibrillation of heart **12** is stopped. IMD **16** may detect fibrillation employing one or more fibrillation detection techniques known in the art.

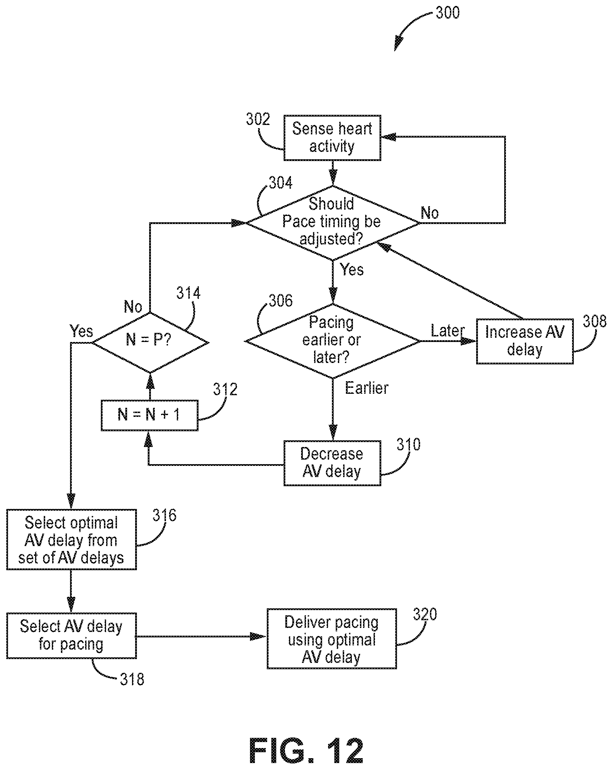

In some examples, programmer **24 (****FIG. 1****)** may be a handheld computing device or a computer workstation or a mobile phone. Programmer **24** may include a user interface that receives input from a user. The user interface may include, for example, a keypad and a display, which may for example, be a cathode ray tube (CRT) display, a liquid crystal display (LCD) or light emitting diode (LED) display. The keypad may take the form of an alphanumeric keypad or a reduced set of keys associated with particular functions. Programmer **24** can additionally or alternatively include a peripheral pointing device, such as a mouse, via which a user may interact with the user interface. In some embodiments, a display of programmer **24** may include a touch screen display, and a user may interact with programmer **24** via the display. For example, methods **300-500** shown in **FIGS. 12-14** may be used to optimize pacing parameters. Through the graphical user interface on programmer **24,** a user may select one or more optimized parameters. For example, methods **300-500** can optimize the AV delay and/or VV delay. The computer instructions can present the best AV delay to be used for a patient or a user may be presented with a set of optimal AV delays and the user can select one of the optimal AV delays.

In addition, the user may select which pacing criterion to use when determining whether pacing timing needs to be adjusted. As described further herein with respect to **FIG. 12****,** block **304** in method **300** uses QRS duration to determine whether pacing timing needs to be adjusted. QRS complex involves a set of waves that occur in quick succession and is referred to as the QRS complex. In some embodiments, the QRS complex is detected using far-field electrical signals. For example, the far-field electrical signals may be sensed in a far-field electrogram (EGM) monitored by IMD **16** and a corresponding lead or a separate device, such as a subcutaneously implanted device. QRS duration is the time from which the Q wave is detected until the S wave ends. While QRS duration can be employed in any of the methods described herein, other criteria may also be used including R-wave timing, pacing-RV or -LV sensing, and/or VV delay. Any one of these criteria may be selected by, for example, processor **80** or by a user through a GUI on programmer **24.**

As used herein, the term "far-field" electrical signal refers to the result of measuring cardiac activity using a sensor, or electrode, positioned outside of an area of interest. For example, an ECG signal measured from an electrode positioned outside of the patient's heart is one example of a far-field electrical signal of the patient's heart. As another example, a far-field electrical signal representing electrical activity of a chamber of the patient's heart may be measured from a sensor, or electrode, positioned in an adjacent chamber.

As used herein, the term "near-field" electrical signal refers to the result of measuring cardiac activity using a sensor, or electrode, positioned near an area of interest. For example, an EGM signal measured from an electrode positioned on the left side of the patient's ventricular septum is one example of a near-field electrical signal of the patient's LV.

R-wave timing is the time in which QRS is detected. Typically, R-wave timing includes using the maximal first derivative of an R-wave upstroke (or the time of the maximal R-wave value). R-wave timing is also used in the device marker channel to indicate the time of the R-wave or the time of ventricular activation.

Pacing-RV sensing or pacing-LV sensing (e.g., pacing-to-RV sensing or pacing-to-LV sensing) is the time interval from the pacing (or pacing artifact) to the time of RV or LV sensing. For example, if pacing-RV sensing is much longer than pacing-LV sensing, this may indicate that the LV activation is occurring much earlier than RV activation (so pacing-RV sensing is longer), then RV pacing may be delivered in synchronization with bundle pacing, so RV and LV activation can occur approximately at the same time.

A user, such as a physician, technician, or other clinician, may interact with programmer **24** to communicate with IMD **16.** For example, the user may interact with programmer **24** to retrieve physiological or diagnostic information from IMD **16.** One illustrative IMD **16** is described in the Medtronic AMPLIA MRITM CRT-D SURESCAN^{™} DTMB2D1 manual, which is incorporated by reference in its entirety. A user may also interact with programmer **24** to program IMD 16, e.g., select values for operational parameters of the IMD.

For example, the user may use programmer **24** to retrieve information from IMD **16** regarding the rhythm of heart **12,** trends therein over time, or tachyarrhythmia (tachy) episodes. As another example, the user may use programmer **24** to retrieve information from IMD **16** regarding other sensed physiological parameters of patient **14,** such as intracardiac or intravascular pressure, activity, posture, respiration, or thoracic impedance. As another example, the user may use programmer **24** to retrieve information from IMD **16** regarding the performance or integrity of IMD **16** or other components of system **10,** such as leads **18, 20, 23,** or a power source of IMD **16.**

The user may use programmer **24** to program a therapy progression, select electrodes used to deliver defibrillation shocks, select waveforms for the defibrillation shock, or select or configure a fibrillation detection algorithm for IMD **16.** The user may also use programmer **24** to program aspects of other therapies provided by IMD **16,** such as cardioversion or pacing therapies (e.g., bundle pacing therapy, cardiac resynchronization therapy, anti-tachy therapy, etc.). In some examples, the user may activate certain features of IMD **16** by entering a single command via programmer **24,** such as depression of a single key or combination of keys of a keypad or a single point- and-select action with a pointing device.

IMD **16** and programmer **24** may communicate via wireless communication using any techniques known in the art. Examples of communication techniques may include, for example, low frequency or radiofrequency (RF) telemetry, but other techniques are also contemplated. In some examples, programmer **24** may include a programming head that may be placed proximate to the patient's body near the IMD **16** implant site in order to improve the quality or security of communication between IMD **16** and programmer **24.**

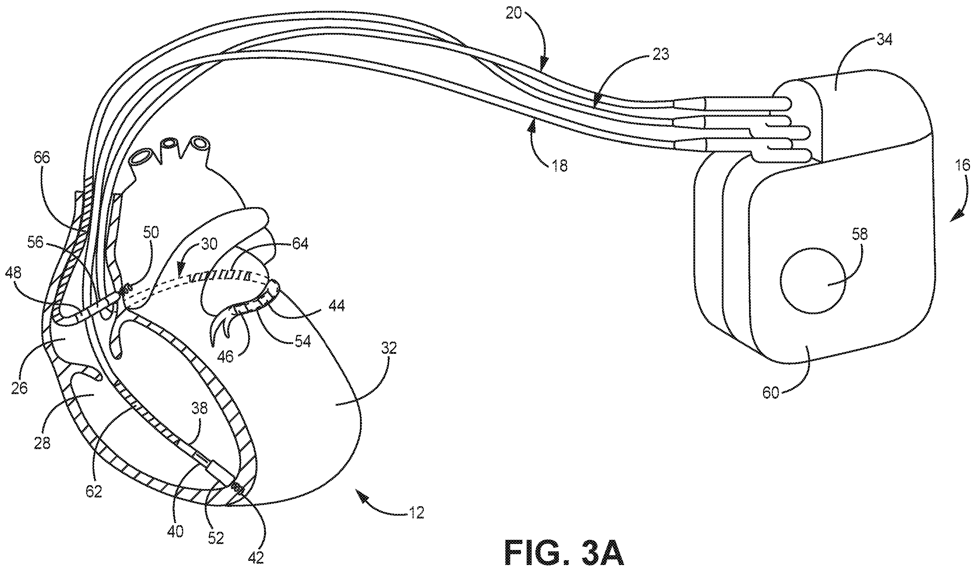

**FIG. 3A** is a conceptual diagram illustrating IMD 16 and leads **18, 20, 23** of therapy system **10** in greater detail. The triple chamber IMD **16** may be used for cardiac rhythm therapy and defibrillation or cardioversion therapy (CRT-D). Leads **18, 20, 23** may be electrically coupled to a stimulation generator, a sensing module, or other modules of IMD **16** via connector block **34.** In some examples, proximal ends of leads **18, 20, 23** may include electrical contacts that electrically couple to respective electrical contacts within connector block **34.** In addition, in some examples, leads **18, 20, 23** may be mechanically coupled to connector block **34** with the aid of set screws, connection pins, or another suitable mechanical coupling mechanism.

Each of the leads **18, 20, 23** includes an elongated, insulative lead body, which may carry any number of concentric coiled conductors separated from one another by tubular, insulative sheaths. In the illustrated example, an optional pressure sensor **38** and bipolar electrodes **40** and **42** are located proximate to a distal end of lead **18.** In addition, bipolar electrodes **44** and **46** are located proximate to a distal end of lead **20** and bipolar electrodes **48** and **50** are located proximate to a distal end of lead **23.** In **FIG. 3A****,** pressure sensor **38** is disposed in right ventricle **28.** Pressure sensor **38** may respond to an absolute pressure inside right ventricle **28,** and may be, for example, a capacitive or piezoelectric absolute pressure sensor. In other examples, pressure sensor **38** may be positioned within other regions of heart **12** and may monitor pressure within one or more of the other regions of heart **12,** or pressure sensor **38** may be positioned elsewhere within or proximate to the cardiovascular system of patient **14** to monitor cardiovascular pressure associated with mechanical contraction of the heart. Optionally, a pressure sensor in the pulmonary artery can be used that is in communication with IMD **16.**

Electrodes **40, 44** and **48** may take the form of ring electrodes, and electrodes **42, 46** and **50** may take the form of extendable and/or fixed helix tip electrodes mounted within insulative electrode heads **52, 54** and **56,** respectively. Each of electrodes **40, 42, 44, 46, 48** and **50** may be electrically coupled to a respective one of the coiled conductors within the lead body of its associated lead **18, 20, 23,** and thereby coupled to respective ones of the electrical contacts on the proximal end of leads **18, 20 23.**

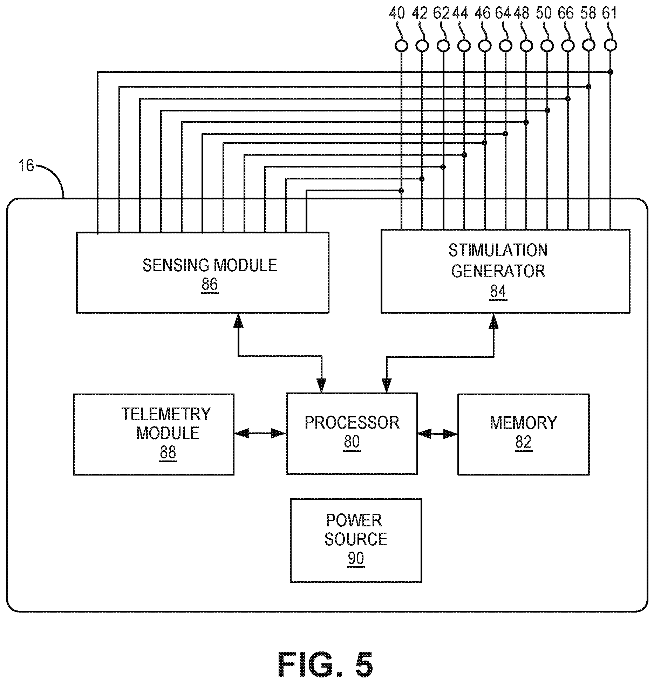

Electrodes **40, 42, 44, 46, 48** and **50** may sense electrical signals attendant to the depolarization and repolarization of heart **12.** The electrical signals are conducted to IMD **16** via the respective leads **18, 20, 23.** In some examples, IMD **16** also delivers pacing pulses via electrodes **40, 42, 44, 46, 48, 50** to cause depolarization of cardiac tissue of heart **12.** In some examples, as illustrated in **FIGS. 3A-B,** IMD **16** includes one or more housing electrodes, such as housing electrode **58,** which may be formed integrally with an outer surface of hermetically-sealed housing **60** of IMD **16** or otherwise coupled to housing **60.** In some examples, housing electrode **58** may be defined by an uninsulated portion of an outward facing portion of housing **60** of IMD **16.** Electrode **50** may be used for pacing and/or sensing of the His bundle or bundle branch tissue. Other divisions between insulated and uninsulated portions of housing **60** may be employed to define two or more housing electrodes. In some examples, housing electrode **58** includes substantially all of housing **60.** Any of the electrodes **40, 42, 44, 46, 48** and **50** may be used for unipolar sensing or pacing in combination with housing electrode **58** or for bipolar sensing with two electrodes in the same pacing lead. In one or more embodiments, housing **60** may enclose a stimulation generator (see **FIG. 5****)** that generates cardiac pacing pulses and defibrillation or cardioversion shocks, as well as a sensing module for monitoring the patient's heart rhythm.

Leads **18, 20, 23** may also include elongated electrodes **62, 64, 66,** respectively, which may take the form of a coil. IMD **16** may deliver defibrillation shocks to heart **12** via any combination of elongated electrodes **62, 64, 66,** and housing electrode **58.** Electrodes **58, 62, 64, 66** may also be used to deliver cardioversion pulses to heart **12.** Electrodes **62, 64, 66** may be fabricated from any suitable electrically conductive material, such as, but not limited to, platinum, platinum alloy or other materials known to be usable in implantable defibrillation electrodes.

Pressure sensor **38** may be coupled to one or more coiled conductors within lead **18.** In **FIG. 3A****,** pressure sensor **38** is located more distally on lead **18** than elongated electrode **62.** In other examples, pressure sensor **38** may be positioned more proximally than elongated electrode **62,** rather than distal to electrode **62.** Further, pressure sensor **38** may be coupled to another one of the leads **20, 23** in other examples, or to a lead other than leads **18, 20, 23** carrying stimulation and sense electrodes. In addition, in some examples, pressure sensor **38** may be self-contained device that is implanted within heart **12,** such as within the septum separating right ventricle **28** from left ventricle **32,** or the septum separating right atrium **26** from left atrium **33.** In such an example, pressure sensor **38** may wirelessly communicate with IMD **16.**

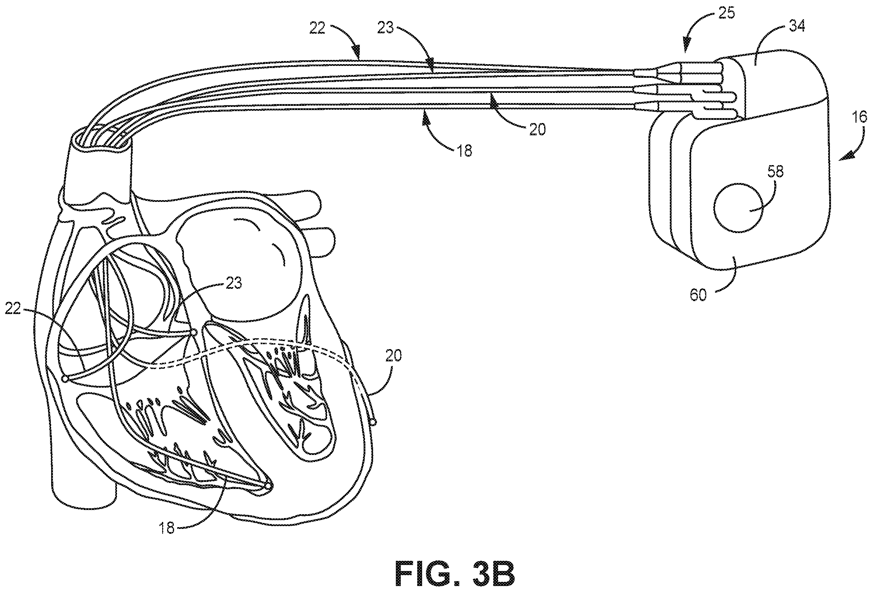

**FIG. 3B** shows IMD **16** coupled to leads **18, 20, 22, 23.** Right atrial (RA) lead **22** may extend through one or more veins and the vena cava, and into the right atrium **26** of heart **12.** RA lead **22** may be connected to triple chamber IMD **16,** e.g., using a Y-adaptor. IMD **16** may be used for cardiac rhythm therapy and defibrillation or cardioversion therapy (CRT-D). RA lead **22** may include electrodes that are the same or similar to the electrodes of lead **18, 20, 23,** such as ring electrodes **40, 44** and **48,** extendable helix tip electrodes **42, 46** and **50,** and/or elongated electrodes **62, 64, 66,** in the form of a coil.

The configuration of therapy system **10** illustrated in **FIGS. 2A-4** are merely examples. In other examples, a therapy system may include epicardial leads and/or patch electrodes instead of or in addition to the transvenous leads **18, 20, 22** and/or bundle pacing lead **23** illustrated in **FIGS. 2A-4** or other configurations shown or described herein or incorporated by reference. Further, IMD **16** need not be implanted within patient **14.** In examples in which IMD **16** is not implanted in patient **14,** IMD **16** may deliver defibrillation shocks and other therapies to heart **12** via percutaneous leads that extend through the skin of patient **14** to a variety of positions within or outside of heart **12.**

In other examples of therapy systems that provide electrical stimulation therapy to heart **12,** such therapy systems may include any suitable number of leads coupled to IMD **16,** and each of the leads may extend to any location within or proximate to heart **12.** For example, other examples of therapy systems may include three transvenous leads located as illustrated in **FIGS. 2A-4****,** and an additional lead located within or proximate to left atrium **33 (****FIG. 1****).** As another example, other examples of therapy systems may include a single lead that extends from IMD **16** into right atrium **26** or right ventricle **28,** or two leads that extend into a respective one of right ventricle **28** and right atrium **26.** An example of this type of therapy system is shown in **FIGS. 3A-3B****.** If four leads are required for therapy delivery, an IS-1 connector may be used in conjunction with Y-adaptor **25** extending from the RA port of the connector. The Y-adaptor allows two separate leads-e.g., right atrial lead and the bundle pacing bundle lead--to extend from the two separate legs of the "Y shape" while the single leg is inserted into connector block **34** on IMD **16.**

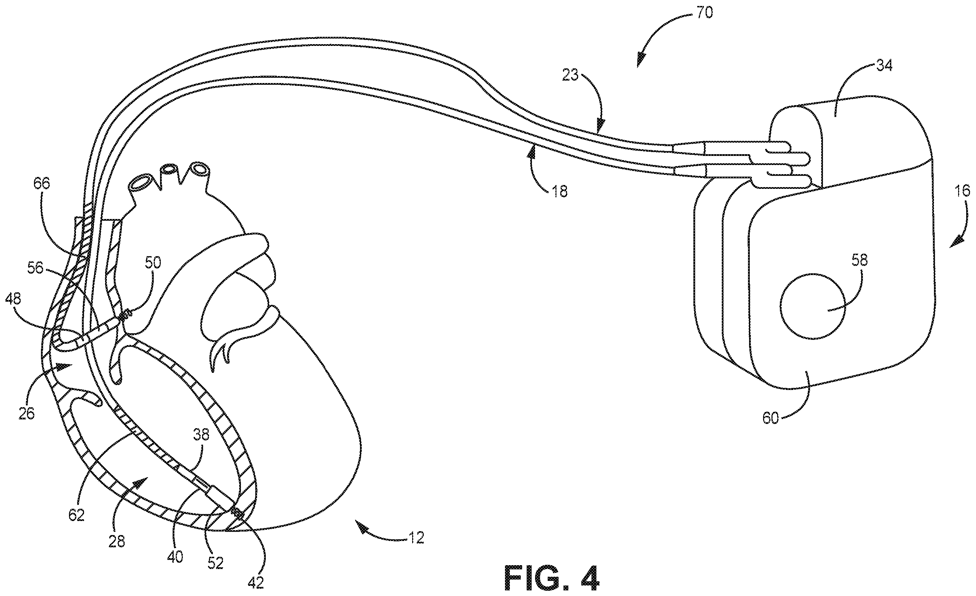

**FIG. 4** **is** a conceptual diagram illustrating another example of therapy system **70.** Therapy system **70** shown in **FIG. 4** may be useful for providing defibrillation and pacing pulses to heart **12.** Therapy system **70** is similar to therapy system **10** of **FIGS. 2A-****B** or **3A-B,** but includes two leads **18, 23,** rather than three leads. Therapy system **70** may utilize an IMD **16** configured to deliver, or perform, dual chamber pacing. Leads **18, 23** are implanted within right ventricle **28** and right atrium **26** to pace the His bundle or one or both bundle branches, respectively.

Bundle pacing lead **23** may be in the form of a helix (also referred to as a helical electrode) may be positioned proximate to, near, adjacent to, or in the His bundle or bundle branch tissue. Bundle pacing lead **23** is configured as a bipolar lead or as a quadripolar lead that may be used with a pacemaker device, a CRT-P device or a CRT-ICD.

As illustrated in **FIGS. 2A-4****,** lead configurations and connections of IMD **16** may vary in any suitable manner. In particular, lead configurations and connections of IMD **16** may be based on the presence of atrial fibrillation (AF) and/or the bundle branch block. If there is no AF present in the patient, bundle pacing lead **23,** located in the bundle of His or one or both bundle branches (e.g., bundle of His or bundle branch region) may be connected to IMD **16** ventricular connection port and atrial lead **22** in the right atrial (RA) may be connected to the atrial connection port of the connector module. If there is persistent/chronic AF, bundle pacing lead **23** may be connected to the device atrial connection port and RV lead **18** in the right ventricle (RV) may be connected to the ventricular connection port. In an ICD, RV lead **18** may be connected to connector block **34** at RV connection port, and bundle pacing lead **23** may be connected to the atrial port connector block.

In a CRT-ICD, RV lead **18** positioned in the RV may be connected to device RV connection port. If there is no atrial fibrillation (AF), atrial lead **22** positioned in the right atrium may be connected to the atrial connection port, and bundle pacing lead **23** may be connected to the LV connection port. If there is persistent/chronic AF, bundle pacing lead **23** may be connected to the device atrial connection port, RV lead **18** may be connected to the RV connection port, and LV lead **20** in LV cardiac vein may be connected to the LV connection port.

In a CRT-P device, if there is no AF, atrial lead **22** in the right atrium may be connected to the atrial connection port, bundle pacing lead **23** may be connected to the device RV connection port, and LV lead **20** in the LV cardiac vein may be connected to the LV connection port. If there is persistent/chronic AF, bundle pacing lead **23** may be connected to the atrial connection port, RV lead **18** may be connected to the RV connection port, and LV lead **20** may be connected to the LV connection port.

In some embodiments, a right atrial port may receive RA lead **22,** a left ventricular port may receive LV lead **20,** and bundle pacing lead **23** is configured to be coupled to the right atrial port or ventricular port.

In some embodiments, when the patient is experiencing atrial fibrillation, bundle pacing lead **23** may be inserted through the device right atrial port, RV lead **18** may be inserted through the device right ventricular port, and LV lead **20** may be inserted through the device left ventricular port.

In some embodiments, when a patient is not experiencing atrial fibrillation, bundle pacing lead **23** may be inserted through either the right or left ventricular port of the device, and RV or LV lead **18, 20** may be inserted through the other of the right or left ventricular port of the device.

In some embodiments, the right atrial port may receive a Y-adaptor. The Y-adaptor may receive connections to the tip and ring electrodes of bundle pacing lead **23.**

In some embodiments, RV lead **18** is inserted through right ventricular port of IMD **16** and LV lead **20** is inserted through the left ventricular port of IMD **16.**

**FIG. 5** is a functional block diagram of one example configuration of IMD **16,** which includes processor **80,** memory **82,** stimulation generator **84** (e.g., electrical pulse generator or signal generating circuit), sensing module **86** (e.g., sensing circuit), telemetry module **88,** and power source **90.** One or more components of IMD **16,** such as processor **80,** may be contained within a housing of IMD **16** (e.g., within a housing of a pacemaker). Telemetry module **88,** sensing module **86,** or both telemetry module **88** and sensing module **86** may be included in a communication interface. Memory **82** includes computer-readable instructions that, when executed by processor **80,** cause IMD **16** and processor **80** to perform various functions attributed to IMD **16** and processor **80** herein. Memory **82** may include any volatile, non-volatile, magnetic, optical, or electrical media, such as a random-access memory (RAM), read-only memory (ROM), non-volatile RAM (NVRAM), electrically-erasable programmable ROM (EEPROM), flash memory, or any other digital media.

Processor **80** may include any one or more of a microprocessor, a controller, a digital signal processor (DSP), an application specific integrated circuit (ASIC), a field-programmable gate array (FPGA), or equivalent discrete or integrated logic circuitry. In some examples, processor **80** may include multiple components, such as any combination of one or more microprocessors, one or more controllers, one or more DSPs, one or more ASICs, or one or more FPGAs, as well as other discrete or integrated logic circuitry. The functions attributed to processor **80** herein may be embodied as software, firmware, hardware or any combination thereof. Processor **80** controls stimulation generator **84** to deliver stimulation therapy to heart **12** according to a selected one or more of therapy programs (e.g., optimization of the atrial-His bundle or bundle branch-ventricle delay, VV delay etc.), which may be stored in memory **82.** Specifically, processor **80** may control stimulation generator **84** to deliver electrical pulses with amplitudes, pulse widths, frequency, or electrode polarities specified by the selected one or more therapy programs.

In some embodiments, RA lead **22** may be operably coupled to electrode **61,** which may be used to monitor or pace the RA. Stimulation generator **84** may be electrically coupled to electrodes **40, 42, 44, 46, 48, 50, 58, 61, 62, 64,** and **66,** e.g., via conductors of respective lead **18, 20, 22, 23** or, in the case of housing electrode **58,** via an electrical conductor disposed within housing **60** of IMD **16.** Stimulation generator **84** may be configured to generate and deliver electrical stimulation therapy to heart **12.** For example, stimulation generator **84** may deliver defibrillation shocks to heart **12** via at least two of electrodes **58, 62, 64, 66.** Stimulation generator **84** may deliver pacing pulses via ring electrodes **40, 44, 48** coupled to leads **18, 20, 23,** respectively, and/or helical electrodes **42, 46,** and **50** of leads **18, 20,** or **23,** respectively. Bundle pacing can be delivered through lead **23** that is connected to an atrial, RV, or LV connection port of connector block **34.** In some embodiments, His bundle or bundle branch therapy can be delivered through leads **18** or **23.** In some examples, stimulation generator **84** delivers pacing, cardioversion, or defibrillation stimulation in the form of electrical pulses. In other examples, stimulation generator **84** may deliver one or more of these types of stimulation in the form of other signals, such as sine waves, square waves, or other substantially continuous time signals.

Stimulation generator **84** may include a switch module and processor **80** may use the switch module to select, e.g., via a data/address bus, which of the available electrodes are used to deliver defibrillation shocks or pacing pulses. The switch module may include a switch array, switch matrix, multiplexer, or any other type of switching device suitable to selectively couple stimulation energy to selected electrodes.

Sensing module **86** monitors signals from at least one of electrodes **40, 42, 44, 46, 48, 50, 58, 61, 62, 64** or **66** in order to monitor electrical activity of heart **12,** e.g., via electrical signals, such as electrocardiogram (ECG) signals and/or electrograms (EGMs). Sensing module **86** may also include a switch module to select which of the available electrodes are used to sense the heart activity. In some examples, processor **80** may select the electrodes that function as sense electrodes via the switch module within sensing module **86,** e.g., by providing signals via a data/address bus. In some examples, sensing module **86** includes one or more sensing channels, each of which may include an amplifier. In response to the signals from processor **80,** the switch module may couple the outputs from the selected electrodes to one of the sensing channels.

In some examples, one channel of sensing module **86** may include an R-wave amplifier that receives signals from electrodes **40, 42,** which are used for pacing and sensing in right ventricle **28** of heart **12.** Another channel may include another R-wave amplifier that receives signals from electrodes **44, 46,** which are used for pacing and sensing proximate to left ventricle **32** of heart **12.** In some examples, the R-wave amplifiers may take the form of an automatic gain-controlled amplifier that provides an adjustable sensing threshold as a function of the measured R-wave amplitude of the heart rhythm.

In addition, in some examples, one channel of sensing module **86** may include a P-wave amplifier that receives signals from electrodes **48, 50,** which are used for pacing and sensing in right atrium **26** of heart **12.** In some examples, the P-wave amplifier may take the form of an automatic gain-controlled amplifier that provides an adjustable sensing threshold as a function of the measured P-wave amplitude of the heart rhythm. Examples of R-wave and P-wave amplifiers are described in U.S. Patent No. 5,117,824 to Keimel et al., which issued on June 2, 1992 and is entitled, "APPARATUS FOR MONITORING ELECTRICAL PHYSIOLOGIC SIGNALS," and is incorporated herein by reference in its entirety. Other amplifiers may also be used. Furthermore, in some examples, one or more of the sensing channels of sensing module **86** may be selectively coupled to housing electrode **58,** or elongated electrodes **62, 64,** or **66,** with or instead of one or more of electrodes **40, 42, 44, 46, 48** or **50,** e.g., for unipolar sensing of R-waves or P-waves in any of chambers **26, 28,** or **32** of heart **12.**

In some examples, sensing module **86** includes a channel that includes an amplifier with a relatively wider pass band than the R-wave or P-wave amplifiers or a high-resolution amplifier with relatively narrow-pass band for His bundle or bundle branch potential recording. Signals from the selected sensing electrodes that are selected for coupling to this wide-band amplifier may be provided to a multiplexer, and thereafter converted to multi-bit digital signals by an analog-to-digital converter for storage in memory **82** as an electrogram (EGM). In some examples, the storage of such EGMs in memory **82** may be under the control of a direct memory access circuit. Processor **80** may employ digital signal analysis techniques to characterize the digitized signals stored in memory **82** to detect and classify the patient's heart rhythm from the electrical signals. Processor **80** may detect and classify the heart rhythm of patient **14** by employing any of the numerous signal processing methodologies known in the art.

If IMD **16** is configured to generate and deliver pacing pulses to heart **12,** processor **80** may include pacer timing and control module, which may be embodied as hardware, firmware, software, or any combination thereof. The pacer timing and control module may include a dedicated hardware circuit, such as an ASIC, separate from other processor **80** components, such as a microprocessor, or a software module executed by a component of processor **80,** which may be a microprocessor or ASIC. The pacer timing and control module may include programmable counters which control the basic time intervals associated with DDD, VVI, DVI, VDD, AAI, DDI, DDDR, VVIR, DVIR, VDDR, AAIR, DDIR and other modes of single and dual chamber pacing. In the aforementioned pacing modes, "D" may indicate dual chamber, "V" may indicate a ventricle, "I" may indicate inhibited pacing (e.g., no pacing), and "A" may indicate an atrium. The first letter in the pacing mode may indicate the chamber that is paced, the second letter may indicate the chamber in which an electrical signal is sensed, and the third letter may indicate the chamber in which the response to sensing is provided.

Intervals defined by the pacer timing and control module may include atrial and ventricular pacing escape intervals, refractory periods during which sensed P-waves and R-waves are ineffective to restart timing of the escape intervals, and the pulse widths of the pacing pulses. As another example, the pace timing and control module may define a blanking period and provide signals from sensing module **86** to blank one or more channels, e.g., amplifiers, for a period during and after delivery of electrical stimulation to heart **12.** The durations of these intervals may be determined by processor **80** in response to stored data in memory **82.** The pacer timing and control module may also determine the amplitude of the cardiac pacing pulses.

During pacing, escape interval counters within the pacer timing/control module may be reset upon sensing of R-waves and P-waves. Stimulation generator **84** may include pacer output circuits that are coupled, e.g., selectively by a switching module, to any combination of electrodes **40, 42, 44, 46, 48, 50, 58, 61, 62,** or **66** appropriate for delivery of a bipolar or unipolar pacing pulse to one of the chambers of heart **12.** Processor **80** may reset the escape interval counters upon the generation of pacing pulses by stimulation generator **84,** and thereby control the basic timing of cardiac pacing functions, including anti-tachyarrhythmia pacing.

The value of the count present in the escape interval counters when reset by sensed R-waves and P-waves may be used by processor **80** to measure the durations of R-R intervals, P-P intervals, P-R intervals and R-P intervals, which are measurements that may be stored in memory **82.** Processor **80** may use the count in the interval counters to detect a tachyarrhythmia event, such as ventricular fibrillation event or ventricular tachycardia event. Upon detecting a threshold number of tachyarrhythmia events, processor **80** may identify the presence of a tachyarrhythmia episode, such as a ventricular fibrillation episode, a ventricular tachycardia episode, or a non-sustained tachycardia (NST) episode. Measurements in His-bundle- or bundle-branch-potential to R-wave interval, measurements in atrial to His-bundle- or bundle-branch-potential interval, and EGM morphology and duration can be used for (1) timing the delivery of His bundle or bundle branch pacing (e.g., bundle pacing), (2) determining the efficacy of His bundle or bundle branch pacing, and/or (3) determining the status of activation propagation such as the presence of left or right bundle branch block.

In some examples, processor **80** may operate as an interrupt driven device, and is responsive to interrupts from pacer timing and control module, where the interrupts may correspond to the occurrences of sensed P-waves and R-waves and the generation of cardiac pacing pulses. Any necessary mathematical calculations to be performed by processor **80** and any updating of the values or intervals controlled by the pacer timing and control module of processor **80** may take place following such interrupts. A portion of memory **82** may be configured as a plurality of recirculating buffers, capable of holding series of measured intervals, which may be analyzed by processor **80** in response to the occurrence of a pace or sense interrupt to determine whether the patient's heart **12** is presently exhibiting atrial or ventricular tachyarrhythmia.

In some examples, an arrhythmia detection method may include any suitable tachyarrhythmia detection algorithms. In one example, processor **80** may utilize all or a subset of the rule-based detection methods described in U.S. Patent No. 5,545,186 to Olson et al., entitled, "PRIORITIZED RULE BASED METHOD AND APPARATUS FOR DIAGNOSIS AND GREATMENT OF ARRHYTHMIAS," which issued on August 13, 1996, or in U.S. Patent No. 5,755,736 to Gillberg et al., entitled, "PRIORITIZED RULE BASED METHOD AND APPARATUS FOR DIAGNOSIS AND TREATMENT OF ARRHYTHMIAS," which issued on May 26, 1998, which are incorporated herein by reference in their entireties. However, other arrhythmia detection methodologies may also be employed by processor **80** in other examples.

In the examples described herein, processor **80** may identify the presence of an atrial or ventricular tachyarrhythmia episode by detecting a series of tachyarrhythmia events (e.g., R-R or P-P intervals having a duration less than or equal to a threshold) of an average rate indicative of tachyarrhythmia or an unbroken series of short R-R or P-P intervals. The thresholds for determining the R-R or P-P interval that indicate a tachyarrhythmia event may be stored within memory **82** of IMD **16.** In addition, the number of tachyarrhythmia events that are detected to confirm the presence of a tachyarrhythmia episode may be stored as a number of intervals to detect (NID) threshold value in memory **82.** In some examples, processor **80** may also identify the presence of the tachyarrhythmia episode by detecting a variable coupling interval between the R-waves of the heart signal. For example, if the interval between successive tachyarrhythmia events varies by a particular percentage or the differences between the coupling intervals are higher than a given threshold over a predetermined number of successive cycles, processor **80** may determine that the tachyarrhythmia is present.

If processor **80** detects an atrial or ventricular tachyarrhythmia based on signals from sensing module **86,** and an anti-tachyarrhythmia pacing regimen is desired, timing intervals for controlling the generation of anti-tachyarrhythmia pacing therapies by stimulation generator **84** may be loaded by processor **80** into the pacer timing and control module to control the operation of the escape interval counters therein and to define refractory periods during which detection of R-waves and P-waves is ineffective to restart the escape interval counters.

If IMD **16** is configured to generate and deliver defibrillation shocks to heart **12,** stimulation generator **84** may include a high voltage charge circuit and a high-voltage output circuit. In the event that generation of a cardioversion or defibrillation shock is required, processor **80** may employ the escape interval counter to control timing of such cardioversion and defibrillation shocks, as well as associated refractory periods. In response to the detection of atrial or ventricular fibrillation or tachyarrhythmia requiring a cardioversion pulse, processor **80** may activate a cardioversion/defibrillation control module, which may, like pacer timing and control module, be a hardware component of processor **80** and/or a firmware or software module executed by one or more hardware components of processor **80.** The cardioversion/defibrillation control module may initiate charging of the high voltage capacitors of the high voltage charge circuit of stimulation generator **84** under control of a high voltage charging control line.

Processor **80** may monitor the voltage on the high voltage capacitor, e.g., via a voltage charging and potential (VCAP) line. In response to the voltage on the high voltage capacitor reaching a predetermined value set by processor **80,** processor **80** may generate a logic signal that terminates charging. Thereafter, timing of the delivery of the defibrillation or cardioversion pulse by stimulation generator **84** is controlled by the cardioversion/defibrillation control module of processor **80.** Following delivery of the fibrillation or tachycardia therapy, processor **80** may return stimulation generator **84** to a cardiac pacing function and await the next successive interrupt due to pacing or the occurrence of a sensed atrial or ventricular depolarization.

Stimulation generator **84** may deliver cardioversion or defibrillation shocks with the aid of an output circuit that determines whether a monophasic or biphasic pulse is delivered, whether housing electrode **58** serves as cathode or anode, and which electrodes are involved in delivery of the cardioversion or defibrillation shocks. Such functionality may be provided by one or more switches or a switching module of stimulation generator **84.** Stimulation generator **84** is configured to generate electrical stimulation (e.g., pulses) to the His bundle.

Telemetry module **88** includes any suitable hardware, firmware, software or any combination thereof for communicating with another device, such as programmer **24 (****FIG. 2A****).** Under the control of processor **80,** telemetry module **88** may receive downlink telemetry from and send uplink telemetry to programmer **24** with the aid of an antenna, which may be internal and/or external. Processor **80** may provide the data to be uplinked to programmer **24** and the control signals for the telemetry circuit within telemetry module **88,** e.g., via an address/data bus. In some examples, telemetry module **88** may provide received data to processor **80** via a multiplexer.

In some examples, processor **80** may transmit atrial and ventricular heart signals (e.g., electrocardiogram signals) produced by atrial and ventricular sense amplifier circuits within sensing module **86** to programmer **24.** Programmer **24** may interrogate IMD **16** to receive the heart signals. Processor **80** may store heart signals within memory **82** and may retrieve stored heart signals from memory **82.** Processor **80** may also generate and store marker codes indicative of different cardiac episodes detected by sensing module **86** and may transmit the marker codes to programmer **24.** An example pacemaker with marker-channel capability is described in U.S. Patent No. 4,374,382 to Markowitz, entitled, "MARKER CHANNEL TELEMETRY SYSTEM FOR A MEDICAL DEVICE," which issued on February 15, 1983 and is incorporated herein by reference in its entirety.

The various components of IMD **16** are coupled to power source **90,** which may include a rechargeable or non-rechargeable battery. A non-rechargeable battery may be selected to last for several years, while a rechargeable battery may be inductively charged from an external device, e.g., on a daily or weekly basis.

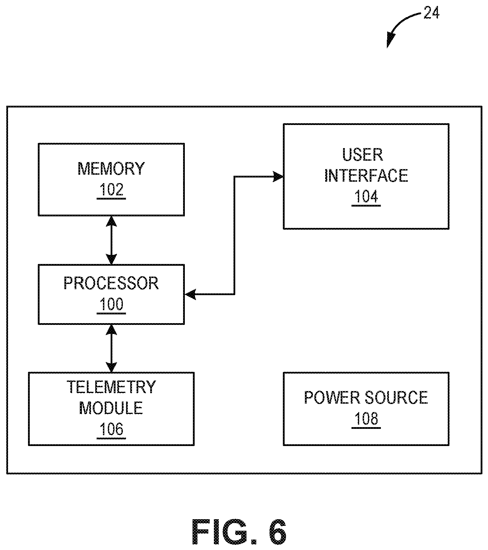

**FIG. 6** is block diagram of illustrative programmer **24.** As shown in **FIG. 5****,** programmer **24** includes processor **100,** memory **102,** user interface **104,** telemetry module **106** (e.g., communication interface), and power source **108.** Programmer **24** may be a dedicated hardware device with dedicated software for programming of IMD **16.** Alternatively, programmer **24** may be an off-the-shelf computing device running an application that enables programmer **24** to program IMD **16.**

Programmer **24** may be used to select therapy programs (e.g., sets of stimulation parameters), generate new therapy programs, modify therapy programs through individual or global adjustments, and/or transmit the new programs to a medical device, such as IMD **16 (****FIGS. 2A-B).** A clinician may interact with programmer **24** via user interface **104,** which may include display to present graphical user interface to a user, and a keypad or another mechanism for receiving input from a user.

Processor **100** can take the form one or more microprocessors, DSPs, ASICs, FPGAs, programmable logic circuitry, or the like, and the functions attributed to processor **100** herein may be embodied as hardware, firmware, software or any combination thereof. Memory **102** may store instructions that cause processor **100** to provide the functionality ascribed to programmer **24** herein, and information used by processor **100** to provide the functionality ascribed to programmer **24** herein. Memory **102** may include any fixed or removable magnetic, optical, or electrical media, such as RAM, ROM, CD-ROM, hard or floppy magnetic disks, EEPROM, or the like. Memory **102** may also include a removable memory portion that may be used to provide memory updates or increases in memory capacities. A removable memory portion may also allow patient data to be easily transferred to another computing device, or to be removed before programmer **24** is used to program therapy for another patient. Memory **102** may also store information that controls therapy delivery by IMD **16,** such as stimulation parameter values.

Programmer **24** may communicate wirelessly with IMD **16,** such as using RF communication or proximal inductive interaction. This wireless communication may be possible through telemetry module **106,** which may be coupled to an internal antenna or an external antenna. An external antenna that is coupled to programmer **24** may correspond to the programming head that may be placed over heart **12,** as described above with reference to **FIGS. 2A-B.** Telemetry module **106** may be similar to telemetry module **88** of IMD **16 (****FIG. 5****).**

Telemetry module **106** may also be configured to communicate with another computing device via wireless communication techniques, or direct communication through a wired connection. Examples of local wireless communication techniques that may be employed to facilitate communication between programmer **24** and another computing device include RF communication according to the IEEE 802.11 or BLUETOOTH^{™} specification sets, infrared communication, e.g., according to the IrDA standard, or other standard or proprietary telemetry protocols. In this manner, other external devices may be capable of communicating with programmer **24.**

Power source **108** delivers operating power to the components of programmer **24.** Power source **108** may include a battery and a power generation circuit to produce the operating power. In some embodiments, the battery may be rechargeable to allow extended operation. Recharging may be accomplished by electrically coupling power source **108** to a cradle or plug that is connected to an alternating current (AC) outlet. In addition, or alternatively, recharging may be accomplished through proximal inductive interaction between an external charger and an inductive charging coil within programmer **24.** In other embodiments, traditional batteries (e.g., nickel cadmium or lithium ion batteries) may be used. In addition, programmer **24** may be directly coupled to an alternating current outlet to power programmer **24.** Power source **108** may include circuitry to monitor power remaining within a battery. In this manner, user interface **104** may provide a current battery level indicator or low battery level indicator when the battery needs to be replaced or recharged. In some cases, power source **108** may be capable of estimating the remaining time of operation using the current battery.

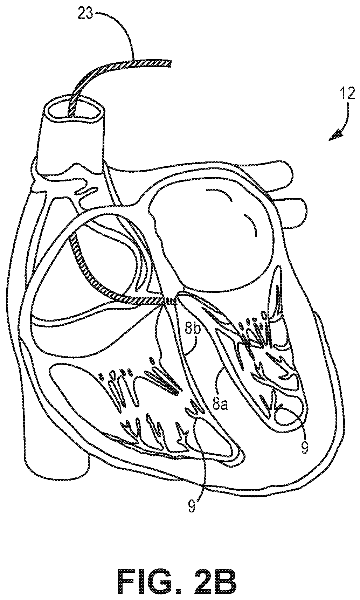

Referring again to **FIG. 5****,** processor **80** of IMD **16** may detect a tachyarrhythmia episode, such as a ventricular fibrillation, ventricular tachycardia, fast ventricular tachyarrhythmia episode, or an NST episode, based on electrocardiographic activity of heart **12** monitored via sensing module **86.** For example, sensing module **86,** with the aid of at least some of the electrodes **40, 42, 44, 46, 48, 50, 58, 61, 62, 64,** and **66** (shown in **FIGS. 2A-B** and **3A-B),** may generate one or more electrical signals, such as electrocardiogram (ECG) or electrogram (EGM) signals, that indicate the electrocardiographic activity. Alternatively, sensing module **86** may be coupled to sense electrodes that are separate from the stimulation electrodes that deliver electrical stimulation to heart **12** (shown in **FIGS. 2-4****)** and may be coupled to one or more different leads than leads **18, 20, 22** (shown in **FIGS. 2-3****).** The ECG or EGM signal may be indicative of the depolarization of heart **12.**

For example, as previously described, in some examples, processor **80** may identify the presence of a tachyarrhythmia episode by detecting a threshold number of tachyarrhythmia events (e.g., R-R or P-P intervals having a duration less than or equal to a threshold). In some examples, processor **80** may also identify the presence of the tachyarrhythmia episode by detecting a variable coupling interval between the R-waves of the heart signal.

The distal end of the lead **18** or lead **23** may be configured to be positioned near or in the His bundle or one or both bundle branches and the IMD 16 may be configured to deliver pacing to stimulate the His bundle or one or both bundle branches during bundle pacing, via electrodes **40, 42,** for example.

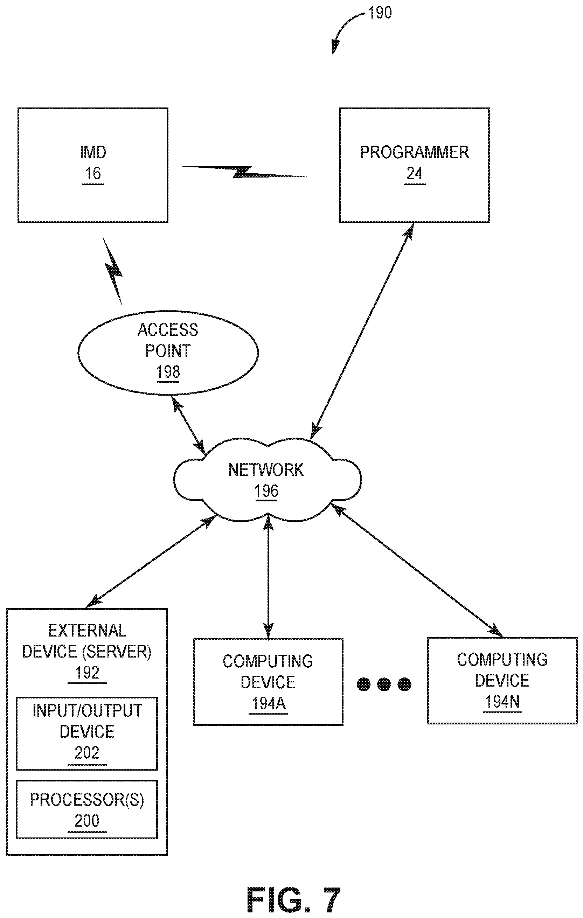

**FIG. 7** is a block diagram of illustrative system **190** that includes external device **132,** such as a server, and one or more computing devices **194A-194N** that are coupled to IMD **16** (see **FIG. 5****)** and programmer **24** (see **FIG. 6****)** via network **196,** according to one embodiment. In this embodiment, IMD **16** uses telemetry module **88** to communicate with programmer **24** via a first wireless connection, and to communicate with access point **198** via a second wireless connection. In the example of **FIG. 7****,** access point **198,** programmer **24,** external device **192,** and computing devices **194A-194N** are interconnected, and able to communicate with each other, through network **196.** In some cases, one or more of access point **198,** programmer **24,** external device **192,** and computing devices **194A-194N** may be coupled to network **196** through one or more wireless connections. IMD **16,** programmer **24,** external device **192,** and computing devices **194A-194N** may each include one or more processors, such as one or more microprocessors, DSPs, ASICs, FPGAs, programmable logic circuitry, or the like, that may perform various functions and operations, such as those described herein.

Access point **198** may include a device that connects to network **196** via any of a variety of connections, such as telephone dial-up, digital subscriber line (DSL), or cable modem connections. In other examples, access point **198** may be coupled to network **196** through different forms of connections, including wired or wireless connections. In some examples, access point **198** may communicate with programmer **24** and/or IMD **16.** Access point **198** may be co-located with patient **14** (e.g., within the same room or within the same site as patient **14)** or may be remotely located from patient **14.** For example, access point **198** may be a home monitor that is located in the patient's home or is portable for carrying with patient **14.**

During operation, IMD **16** may collect, measure, and store various forms of diagnostic data. In certain cases, IMD **16** may directly analyze collected diagnostic data and generate any corresponding reports or alerts. In some cases, however, IMD **16** may send diagnostic data to programmer **24,** access point **198,** and/or external device **192,** either wirelessly or via access point **198** and network **196,** for remote processing and analysis.

In some cases, IMD **16** and/or programmer **24** may combine all the diagnostic data into a single displayable lead integrity report, which may be displayed on programmer **24.** The lead integrity report contains diagnostic information concerning one or more electrode leads that are coupled to IMD **16,** such as leads **18, 20, 22** or **23.** A clinician or other trained professional may review and/or annotate the lead integrity report, and possibly identify any lead-related conditions.

In another example, IMD **16** may provide external device **192** with collected diagnostic data via access point **198** and network **196.** External device **192** includes one or more processors **200.** In some cases, external device **192** may request such data, and in some cases, IMD **16** may automatically or periodically provide such data to external device **192.** Upon receipt of the diagnostic data via input/output device **202,** external device **192** is capable of analyzing the data and generating reports or alerts upon determination that there may be a possible condition with one or more of leads **18, 20, 22, 23.** For example, one or more of leads **18, 20, 22, 23** may experience a condition related to a lead fracture or an insulation breach.

In one embodiment, external device **192** may combine the diagnostic data into a lead integrity report. One or more of computing devices **194A-194N** may access the report through network **196** and display the report to users of computing devices **194A-194N.** In some cases, external device **192** may automatically send the report via input/output device **202** to one or more of computing devices **194A-194N** as an alert, such as an audio or visual alert. In some cases, external device **192** may send the report to another device, such as programmer **24,** either automatically or upon request. In some cases, external device **192** may display the report to a user via input/output device **202.**

In one embodiment, external device **192** may include a secure storage site for diagnostic information that has been collected from IMD **16** and/or programmer **24.** In this embodiment, network **196** may include an Internet network, and trained professionals, such as clinicians, may use computing devices **194A-194N** to securely access stored diagnostic data on external device **192.** For example, the trained professionals may need to enter usernames and passwords to access the stored information on external device **192.** In one embodiment, external device **192** may be a CARELINK^{™} server provided by Medtronic, Inc., of Minneapolis, Minnesota.

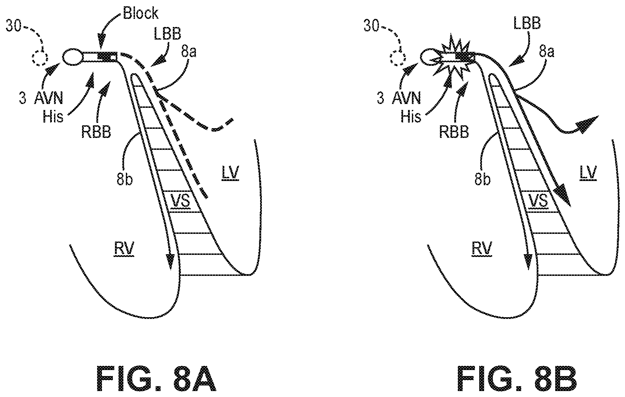

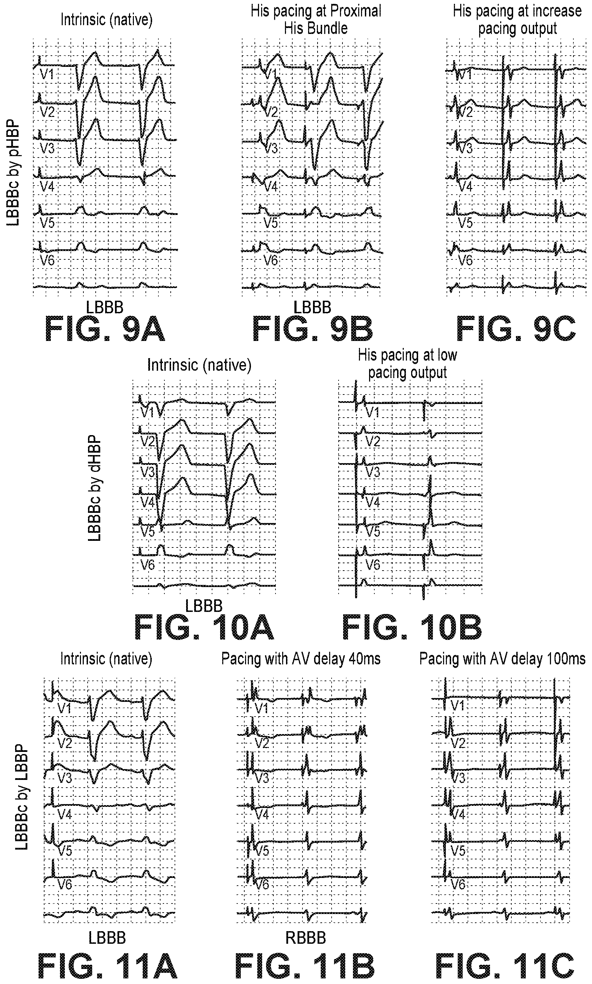

**FIGS. 8A-8D** are schematic diagrams of mechanisms of left bundle branch block correction using bundle pacing. **FIGS. 9-11** graphically depict ECG signals associated with each mechanism of LBBB/RBBB that is associated **FIGS. 8A-8D****.**

**FIGS. 8A-8D** show a block near AV node **3,** and right and left bundle branches **8a, 8b** extending through the left and right ventricles. **FIG. 8A** is a schematic diagram of an incident of left bundle branch block (LBBB) that occurs during an intrinsic rhythm when no pacing is applied. Illustrative intrinsic rhythm is graphically shown in **FIG. 9A** where the right bundle branch **8b** is not blocked.

**FIG. 8B** is a schematic diagram of LBBB correction by proximal His bundle pacing. Proximal His bundle pacing involves delivery of electrical stimuli at a position proximal to the block as opposed to delivering pacing stimuli distal to the block. As shown in this example, the proximal position of the block is closer to AV node **3** while the distal end of the block is further away from AV node **3** and closer to the ventricles. Pacing at the proximal His bundle initiates His bundle pacing without correction of LBBB, which may be similar to traditional His bundle pacing. **FIG. 9B** graphically depicts the rhythm acquired during bundle pacing at a proximal His bundle that appears to be more effective as compared to the intrinsic rhythm (native conduction) graphically shown in **FIG. 9A****.** As shown, LBBB is still present during the intrinsic rhythm **(****FIG. 9A****)** or during bundle pacing at proximal His bundle **(****FIG. 9B****).** Once the pacing output is significantly increased **(****FIG. 9C****),** bundle pacing can reach the left bundle branch and correct left bundle branch block. In one example, bundle pacing output increasing to 2.5 V from 0.5 V may allow the current, or simulation, to reach the whole area to correct for LBBB.

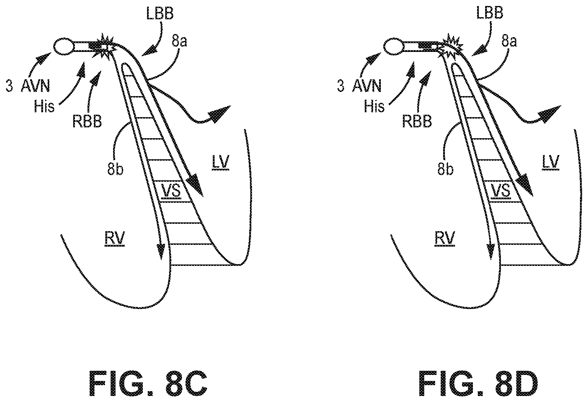

**FIG. 8C** is a schematic diagram of correction of left bundle branch block using distal His bundle pacing, which locates pacing in a distal portion of the His bundle, for example, distal to a His bundle block. **FIG. 10A** graphically depicts the intrinsic rhythm when LBBB is present and pacing has not yet begun to be delivered to the His bundle. **FIG. 10B** graphically depicts distal His bundle pacing at low pacing output. Pacing at distal His bundle captures fibers that may conduct through both left and right bundle branches and may facilitate left bundle branch block correction at a low pacing capture output.

His-bundle or bundle-branch pacing, which may utilize the natural His bundle-Purkinje conduction system, may provide optimal physiological pacing and synchronized ventricular contraction. However, bundle pacing lead **23** implantation may change conduction properties of the His bundle, which may be manifest as conduction block, including either complete or bundle branch block. Additionally, when His bundle pacing is used for correction of bundle branch block (either LBBB or RBBB), it usually requires a large pacing output because the pacing lead may be placed in the proximal His bundle (or proximal block region, see **FIG. 8B****).** In order to achieve a low and stable pacing output for correcting bundle branch block, bundle pacing lead **23** may be placed in the distal His bundle **(****FIG. 8C****)** or even in the bundle branch **(****FIG. 8D****).**

**FIG. 8D** is a schematic diagram of correction of left bundle branch block using left bundle branch pacing. **FIG. 11A** graphically depicts the intrinsic rhythm when LBBB is present and pacing has not yet begun to be delivered to the left bundle branch (LBB). Low-output pacing at left bundle branch (or fibers destined for left bundle branch) may correct left bundle branch block with a trait of right bundle branch block. By adjusting atrial to His-bundle or bundle-branch pacing delay, right bundle branch block can be corrected. The atrial to His-bundle or bundle-branch pacing delay may be defined as the time when an electrical impulse, which may represent depolarization or contraction, is sensed or detected from the atrium and the time in which the pacing pulse is generated from the pulse generator. As shown in **FIG. 11B** in which an AV delay of 40 ms was employed, LBBB is not present but RBBB remains. Increasing the pacing AV delay interval from 40 ms to 100 ms, as shown in **FIG. 11C****,** neither LBBB or RBBB are present.

Thus, while His bundle pacing may be optimal, sometimes undesired effects may be manifested due to bundle pacing lead implantation or pre-existing situations. By adjusting timing of delivering His-bundle or bundle-branch pacing, the present disclosure may re-synchronize His-bundle or bundle-branch pacing with activation from the atrium (pacing or intrinsic) in order to achieve synchronized ventricular activation via both left and right bundle branches and hence contraction, thereby improving pacing therapy. In the event of complete AV block, one bundle branch pacing can be synchronized with the pacing on the other ventricular chamber, for example, if pacing at the left bundle branch, it can be synchronized with the pacing in the right ventricle to avoid right bundle branch block or vice versa. In some embodiments, the bundle pacing lead may be placed in the left bundle branch to stimulate only the left bundle branch, while at the same time the pacing may be synchronized with intrinsic conduction to the RV, with the RV pacing (e.g., intrinsic or paced RV activation) or with the right bundle branch delay.

The illustrative methods disclosed herein automatically adjust delivery of bundle pacing in a cardiac pacing system to achieve synchronized ventricular activation via both left and right bundle branches and/or correction of right or left bundle branch block. Each method **300-500 (****FIGS. 12-14****)** disclosed herein can be automatically performed in an entirely closed-loop therapy system 10. There are many different therapy system embodiments that can deliver His-bundle or bundle-branch pacing in accordance with the methods described herein. For example, IMD 16 can be a dual chamber pacemaker that can be used if atrial fibrillation (AF) is not present or not detected by processor **80.** Bundle pacing lead **23** can be connected to ventricular (RV) connection port of connector block **34** of the dual chamber pacemaker. Bundle pacing lead **23** can be properly positioned in His bundle or bundle branch region. In one or more other embodiments, if persistent or chronic AF is detected by processor **80,** bundle pacing lead **23** can be connected to the device atrial connection port while RV lead **18** in the RV can be connected to ventricular connection port of connector block **34** of IMD **16.** Bundle pacing lead **23** may be placed in the His bundle region or bundle branch region.

In one or more other embodiments, therapy system **10** includes IMD **16** such as an ICD with a RV lead, and a bundle pacing lead extending therefrom. The RV lead may be positioned in the RV as is known to one having ordinary skill in the art or shown herein. The RV lead may be connected to the RV connection port of connector block **34** for IMD **16.** The RV shock lead is generally kept in RV. The bundle branch lead may be connected to the atrial port of connector block **34** for IMD 16. Bundle branch lead **23** may then be placed in the His bundle region or bundle branch region. In one or more embodiments, the bundle branch region includes the bundle branch and a volume within 1 mm from the bundle branch.

In one or more other embodiments, a therapy system **10** includes IMD **16** (e.g., CRT-ICD etc.), a bundle pacing lead, and an RV lead. The RV lead is connected to the device RV connection port of connector block **34.** The RV lead is positioned in the RV. The RV lead is connected with atrial connection port of connector block **34.** In the case of CRT-ICD, RV lead (the shocking lead) is always connected to the device RV connection port, and the bundle pacing lead can be connected to the atrial port if there is persistent/chronic AF or to the LV port if there is a sinus rhythm. If no AF is present in the patient, the lead configuration may include the atrial lead being positioned in the right atrium, and the bundle pacing lead connected with LV connection port of connector block **34.** If persistent or chronic AF is detected, the lead configuration may include the bundle pacing lead connected to the device atrial connection port, the RV lead connected to the RV connection port, and the LV lead connected to LV connection port of connector block **34.**

In one or more other embodiments, therapy system **10** includes IMD **16,** such as a CRT-P, an atrial lead, and a bundle pacing lead. If AF is not present, the lead configuration may include the atrial lead being connected to the atrial connection port. The atrial lead is positioned in the right atrium. The bundle pacing lead may be connected to the device RV connection port in some embodiments. The LV lead, connected to LV connection port, is positioned in the LV cardiac vein.

If persistent or chronic AF is present, IMD **16** lead configuration may include the bundle pacing lead being connected to the atrial connection port, the RV lead being connected to RV connection port, and the LV lead being connected to LV connection port of connector block **34** of IMD **16.**

In one or more embodiments, a VVI (e.g., single chamber) pacemaker can be configured with a single bundle pacing lead (e.g., His bundle lead or bundle branch pacing lead) that is connected to the RV connection port. For example, using IMD **16** configured for VVI pacing (e.g., single chamber device), there may be only one connection port, either for atrial pacing or RV pacing in a known way. For bundle pacing, the bundle pacing lead (the only lead) is connected to the only device connection port. If the bundle pacing lead is located in the His bundle, in one or both bundle branches, or is proximate thereto, the bundle pacing lead can sense atrial electrical activity, and bundle pacing can be delivered with an approximated A to bundle-pacing delay (e.g., about 50-80 ms after atrial sensing). Such a case can happen in patients with atrioventricular block.

In another embodiment, a subcutaneous device (SD) could send a control signal to an IMD, such as a leadless pacing device (LPD) (e.g., system **610** of **FIG. 15****),** to initiate bundle pacing. For example, the LPD may be configured to deliver His-bundle or bundle-branch pacing pulses in response to signals received from the SD. The signals may represent timing for delivering pacing pulses, such as an atrial-activation to bundle-pacing interval.

The LPD could sense a cardiac signal (e.g., an electrical signal) from the heart of the patient. Based on the cardiac signal, the LPD could determine whether to deliver bundle pacing to the heart from the LPD and at what timing interval. For example, the LPD, based on the second electrical signal, could determine that bundle pacing is not necessary and terminate bundle pacing. The LPD could consider whether sensed data meets a pre-specified threshold. For instance, if the QRS width does not exceed about 120 ms, the LPD may withhold the delivery of bundle pacing therapy (e.g., the LPD could then signal the SD that bundle pacing should not be delivered based upon the cardiac signal. The SD can be configured to perform a more detailed analysis in which at least one or more parameters (such as at least two parameters) are evaluated. The SD could then send another command signal that confirms, denies, or overrides the LPD.

In another embodiment, the LPD could sense a cardiac signal that indicates a switch between one pacing mode to another pacing mode should occur and would signal the SD. The SD could be configured to send an override signal to the LPD unless certain conditions are met.

In another embodiment, the SD transmits a control signal to the LPD to initiate bundle pacing. The LPD senses a cardiac signal (e.g., an electrical signal) from the heart of the patient. Based on the cardiac signal, the LPD could determine whether to deliver bundle pacing or the type of bundle pacing to deliver to the heart from the LPD. In one or more embodiments, the LPD, based on the second electrical signal, could initially determine that bundle pacing is not necessary. The initial determination by the LPD could be made by using tests having thresholds of one or more parameters. In one or more embodiments, the SD could perform a more detailed analysis as to whether bundle pacing should be delivered and when to be delivered. Using the sensed data from the LPD and/or SD, the SD could generate another signal to the LPD that either confirms, denies or overrides the LPDs initial determination.

In yet another embodiment, the LPD could determine that bundle pacing should be used in contravention to the SD communication. In one embodiment, the LPD would deliver bundle pacing. In one or more other embodiments, the LPD could determine that bundle pacing should be used over other pacing modes in contravention to the SD communication. In this scenario, the LPD could deliver bundle pacing.

In yet another embodiment, the LPD alone delivers bundle pacing without any communication with a SD. The LPD includes the computer instructions that employs any of the methods (e.g., **FIGS. 12-14****)** disclosed herein. The LPD includes a pair of electrodes that senses heart activity to determine when the pacing electrode should pace the His bundle or bundle branch tissue. The LPD can also communicate with one or more sensing electrodes in the RA such that an amplifier is used to amplify the sensed signals from the sensing electrodes. The sensing electrodes are associated with an implantable device.

In yet another embodiment, the LPD can be further configured to receive a signal from one or more subcutaneous electrodes in a heart chamber. The signal from the subcutaneous electrodes can be amplified by an amplifier located in a subcutaneous device. In one embodiment, the amplifier is configured to be up to 2 times larger than existing amplifiers, such as the amplifier located in Boston Scientific's Emblem S-ICD device. In one embodiment, the amplifier is configured to be up to 1.75 times larger than the amplifier located in Boston Scientific's Emblem S-ICD device. In one embodiment, the amplifier is configured to be up to 1.5 times larger than the amplifier located in Boston Scientific's Emblem S-ICD device. In one embodiment, the amplifier is configured to be up to 1.25 times larger than the amplifier located in Boston Scientific's Emblem S-ICD device. Boston Scientific User's Manual EMBLEM S-ICD EMBLEM MRI S-ICD SUBCUTANEOUS IMPLANTABLE DEFIBRILATOR REF A209, A219, located on Boston Scientific's website is incorporated by reference in its entirety herein and can be modified to employ methods **300-500.** For example, the SD may be used to sense far-field electrical signals (such as ECG) to determine QRS duration, or QRSd and an atrial signal, particularly when the LPD cannot sense such far-field electrical signals. Based on QRS timing, QRS duration, and atrial timing, the SD can communicate to the LPD a time to deliver His-bundle or bundle-branch pacing at an appropriate atrial-bundle pacing interval.

In some embodiments, the SD may be configured to deliver electrical pulses to cardiac tissue. The electrical pulses may be related to a different therapy than the therapy provided by the LPD. For example, the SD may deliver electrical pulses for cardiac resynchronization therapy.

In some embodiments, the SD may be employed to sense electrical signals from the patient's heart to determining the timings of atrial and/or ventricular activations.

The process flow diagram of **FIG. 12** illustrates an illustrative method that can be performed by IMD **16.** Illustrative implantable medical devices that can optimize pacing parameters such as A-bundle pacing delay include pacemakers, implantable cardioverter defibrillators, leadless pacemakers (e.g., MICRA^{™}), leadless pacemakers in communication with a subcutaneous device shown in **FIGS. 15-17** and described in the accompanying text. Medtronic MICRATM MC1VR01 MR Conditional single chamber transcatheter pacing system with SureScanA^{™} technology (VVIR) manual is incorporated herein by reference in its entirety. As described in **FIG. 12****,** bundle pacing may be delivered in synchronization with intrinsic activation or paced activation. In the example of **FIG. 12****,** the right atrial lead may be positioned in the right atrium, the bundle pacing lead may be positioned in the His bundle region or in the bundle branch region, which may be in, or in proximity to, the left bundle branch and/or the right bundle branch. The RV lead may be positioned within the right ventricle.

Method **300** begins at block **302** in which heart activity is acquired by IMD **16** via sensing module **86** receiving signals from electrode(s) located in or near the His bundle or one or both bundle branches and/or other electrodes located in any of the other embodiments shown in **FIGS. 2-4** and **15-17,** or otherwise incorporated by reference.

The sensed data may be stored into memory **82** of the IMD **16.** Sensed heart activity may be used to determine, or may include, one or more of: an intraventricular interval, a QRS duration, a QRS morphology, a QRS vector, an R-wave timing, a bundle-pacing to left- or right-ventricular-activation interval, and an atrial-sensing to left- or right-ventricular-activation interval. Examples of information acquired from sensed data (e.g., EGMs) include QRS duration, QRS morphology, R-wave timing, pacing-RV or -LV sensing, and/or VV interval. Pacing-RV or -LV sensing means the time interval from bundle pacing to the RV- or LV- sensing. Bundle pacing may synchronize both right and left ventricles, e.g., bundle-pacing to RV-sensing interval and bundle-pacing to LV-sensing interval are almost equal (e.g., meaning both ventricles are activated at almost same time, synchronized at the same time or with a delay).

The QRS complex is the combination of three deflections or QRS waves. The Q, R, and S waves occur in rapid succession and reflect a single event and are usually considered together. A Q wave is any downward deflection after the P wave. An R-wave follows as an upward deflection, and the S wave is any downward deflection after the R-wave. The QRS duration is measured when the Q wave is first detected until the end of the S wave. QRS morphology may be used to determine RV or LV activation time.

At decision block **304,** the processor **80** of the IMD **16** may use the data, acquired from the sensed heart activity (e.g., recording of EGMs, etc.) in block **302,** to determine whether one or more pacing parameters should be adjusted, such as bundle pacing timing. For example, processor **80** can determine whether QRS parameter or activation interval is greater than or equal to a threshold (e.g., 120 ms). Examples of QRS parameters or activation intervals may include, but are not limited to QRS duration, AV interval, VV interval, QRS morphology, etc. VV (V to V) interval may be representative of ventricular synchrony. When the heart rate changes, atrioventricular conduction time may change, and bundle pacing time may be adjusted with a new appropriate AV delay. Another determination can be made as to whether QRS duration is less than a threshold, such as about 120 ms. By including two different conditions that check whether QRS duration is greater than or equal to the threshold and another condition of checking whether QRS duration is less than the same or different threshold, the logic of the flow diagram causes the AV delay to converge to an optimal AV delay by gradually adjusting the AV delay until a certain number of times are looped through the flow diagram.

If processor **80** determines that one or more pacing parameters do not need to be adjusted, the NO path from block **304** to block **302** may return to continue sensing heart activity. In addition, the NO path may continue to allow bundle pacing to be delivered without requiring adjustment of pacing parameter(s). In contrast, if processor **80** of IMD **16** determines that one or more pacing parameters should be adjusted, then the YES path may continue to decision block **306** to determine whether the pacing stimuli (e.g., pulses) should be delivered earlier or later than the pacing stimuli that IMD **16** is currently set to deliver. One illustrative evaluation that can be performed by processor **80** is whether a bundle-pacing to ventricular-activation (e.g., V sensing) interval is less than a second threshold (e.g., 80 ms). In some embodiments, if the bundle pacing to ventricular activation interval is less than the second threshold, then the pacing stimuli may be delivered later, or otherwise, the pacing stimuli may be delivered earlier.

If processor **80** determines that the pacing stimuli should be delivered later, then method **300** may go to block **308** and may increase (e.g., up to about 5, 10, or 15 ms) the AV delay. Otherwise, method **300** may go to block **310** and processor **80** may decrease (e.g., up to about 5, 10, or 15 ms) the AV delay to deliver pacing stimuli earlier to the later depolarizing ventricle, in particular earlier than IMD **16** previously delivered pacing pulses thereto.

After decreasing the AV delay and storing the new AV delay into memory at block **310,** then method **300** may continue to blocks **312, 314** to cause processor **80** to perform a counting operation that ensures that sufficient sensed data over a period of time has been acquired and checked in order to find an optimum AV delay via method **300.** Block **312** may determine whether *N* = *N +* 1. N can be initialized at 1 (or 0) when method **300** is performed. N can be re-initialized to "1" by processor **80** when method **300** is re-started after a certain time period has expired (e.g., about 10 minutes, about 1 hour, about 1 day etc.).

Each time the logic of method **300** loops, or iterates, through block **312,** then N may be increased by "1" by processor **80** according to the equation of *N* = *N +* 1. At decision block **314,** processor **80** may determine whether *N* = *P* where P is a pre-defined integer that provides a maximum number of times (e.g., up to 10 times) that sensed data is acquired via block **302** to determine if the pacing parameters should deliver pacing pulses earlier or later than the present pacing parameters stored in memory **82** of the IMD **16.** Block **314** may return to block **304** in which a determination is made by processor **80** as to whether one or more pacing parameters need to be adjusted. One example of determining whether pacing parameters need to be adjusted may be based on whether a QRS duration exceeds a threshold.

The YES path from block **314** may continue to block **316** in which the optimal atrial-activation to bundle-pacing delay (e.g., AV delay or interval) may be selected from a set of atrial-activation to bundle-pacing intervals by processor **80** that were acquired while IMD 16 performed method **300** and that were stored in the IMD memory **82.** In one or more embodiments, the IMD **16** will be set such that a minimal duration is employed. For example, after five cycles of AV delay at about 80, 90, 100, 110, and 120 ms, QRS duration may be correspondingly reduced to about 120, 100, 95, 93, and 99 ms, respectively. The optimal QRS duration may be determined to be 93 ms, which corresponds to an AV delay 110 ms, and as such, the 110 ms AV delay may be stored in the memory.

In another example, whether bundle pacing timing should be adjusted may be based on dyssynchrony, or a measure of dyssynchrony. Non-limiting examples of detecting dyssynchrony include using: a comparison of a bundle-pacing to RV-sensing interval with a bundle-pacing to LV-sensing interval, a QRS morphology change, or both. Using a QRS morphology change may include comparing a current QRS morphology to a predetermined QRS morphology representative of an RBBB or LBBB pattern in the patient's heart. In some embodiments, in response to determining that bundle-pacing timing should be adjusted using dyssynchrony, the method may determine that bundle-pacing output should be adjusted.

At block **318,** the atrial-activation to bundle-pacing interval may be set to employ the optimized interval for bundle pacing. At block **320,** the IMD **16** may be configured to pace the His bundle or bundle branch tissue. In particular, processor **80** may control an electrical pulse generator to deliver a bundle pace on expiration of a defined AV interval, which may follow atrial activation, or upon expiration of a bundle-pacing to ventricular-activation interval, which may follow ventricular activation.

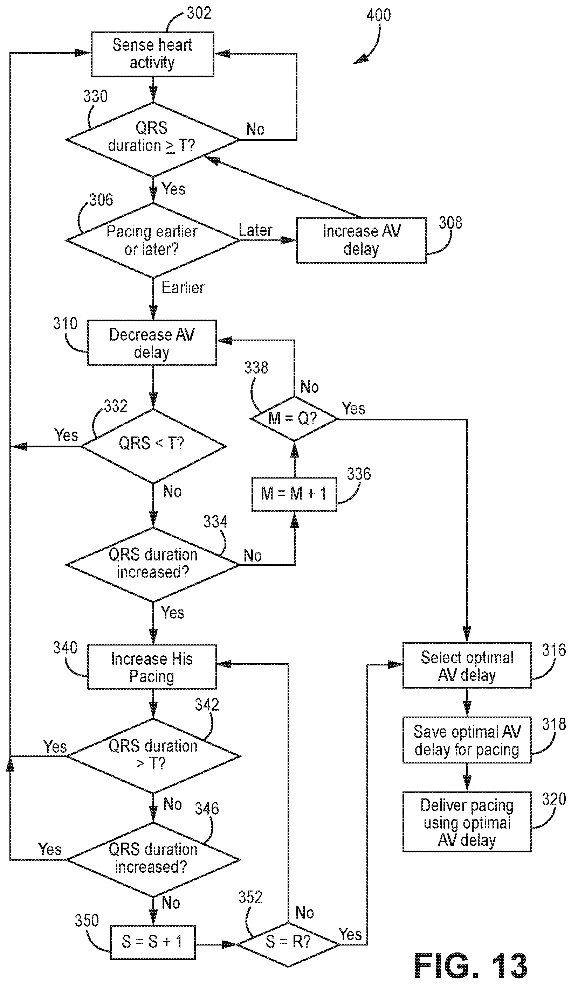

**FIG. 13** is a flowchart of left bundle branch pacing in synchronization with (back-up) RV pacing during left bundle branch block. As described in **FIG. 13****,** left bundle branch pacing in synchronization with (back-up) RV pacing during left bundle branch block (LBBB) may be utilized during atrial fibrillation, in which reliable atrial sensing/pacing is unavailable, after AV node ablation, or after there is right bundle branch injury during bundle pacing lead implantation. In the example of **FIG. 13****,** the bundle pacing lead may be located in the His bundle or bundle branch region, the RV lead may be positioned in the right ventricle. By adjusting bundle pacing time in reference with RV pacing time, a narrow QRS duration can be achieved.

In **FIGS. 12** and **13****,** a variable can trigger adjustment of atrial-to-bundle pacing interval. In some embodiments, the variable used for triggering bundle pacing is QRS duration. Other variables for triggering bundle pacing adjustment can include a change in the interval from the time of bundle-pacing to RV-sensing, to LV-sensing, or to far-field R-wave, or even a QRS morphology that differentiates the RV and LV activation time. For example, if bundle-pacing to RV-sensing interval is 80-100 ms longer than bundle-pacing to far-field R-wave, bundle pacing should be delayed in steps of 10 ms until bundle-pacing to RV-sensing interval is equal to or slightly longer than bundle-pacing to far-field R-wave interval (e.g., if intrinsic activation is available) or initiate RV pacing at an interval equal to or slightly longer than bundle-pacing to far-field R-wave interval.

In **FIG. 12****,** the "bundle pacing to RV sensing < 80 ms" can be a function of AV (or PR) interval, such as 40-60% of AV (or PR) interval, for example, if bundle-pacing to RV-sensing interval is less than 40% of AV or PR interval, the A-bundle pacing interval can be delayed by 10 ms (e.g., see the delay mechanism in **FIGS. 12** and **13****).** The "PR" interval refers to the interval between the P-wave and the R-wave in the QRS complex.

Additionally, the algorithm can distinguish between the LBBB pattern or the RBBB pattern, so the bundle pacing can be better synchronized with either intrinsic activation or ventricular pacing. For example, when the bundle pacing is used to correct LBBB and the RV sensing is delayed near the end of the detected far-field ECG, which may suggest the presence of delayed RV activation (RBBB), the bundle pacing may be delayed with a longer atrial-His pacing interval for bundle pacing to be synchronized with activation from atria, or RV pacing may be delivered earlier so that paced RV activation can be better synchronized with bundle pacing, leading to narrowed QRS duration. In general, bundle pacing may be adapted to achieve synchrony of the patient's heart in a manner manifested in a plurality of far-field electrical signals as a narrowed QRS duration or as a correction of LBBB or RBBB patterns in QRS morphology.

Similarly, if RV sensing occurs too early at the very beginning of a far-field EGM (or far before LV sensing), which may suggest bad synchronization, bundle pacing may be delivered earlier or RV pacing may be delayed, so that paced RV activation can be better synchronized with bundle pacing, leading to narrowed QRS duration.

While in some embodiments the time adjustment of bundle pacing is based on the QRS duration, it can also be based on the interval of bundle-pacing to RV- or LV-sensing, or even based on the detection of the presence of RBBB or LBBB (as described above) in case the device does not, or cannot, measure QRS duration. Additionally, the morphology of far- and near-field EGM and/or surface ECG may be used to estimate the activation delay in one or both ventricles. For example, in RV lead recording, if the EGM morphology of the RV tip-ring (e.g., the tip towards LV) is biphasic from a positive direction to a negative direction, meaning the propagation is from LV to RV, then the left bundle pacing may be delayed until a positive monophasic waveform followed with a relatively long small negative waveform is recorded. A similar method can be used to detect the activation propagation in the left ventricle.

Various methods may be used to detect dyssynchrony in the patient's heart for use in His bundle or bundle branch pacing. In some embodiments, a change between time intervals may be detected when an RV lead and/or an LV lead is available or used. For example, an increase in the difference between the pacing to RV-sensing interval and the pacing to LV-sensing interval or an increase in QRS duration may be detected based on electrical signals measured using the RV lead and/or the LV lead. The IMD may then be able to correct for the change in time intervals by adjusting the pacing timing for RV or for LV, or adjusting the timing of His pacing or bundle branch pacing, or adjusting the pacing output, according to techniques described herein.

In some other embodiments, when an RV lead and/or an LV lead is not available or used, a method may be used to calibrate an IMD to facilitate detection of an RBBB condition and/or an LBBB condition. The method may include pacing the LBB. For example, an intracardiac IMD, or leadless pacing device (LPD), may be configured to pace the LBB and the RBB. In response to pacing the LBB, an RBBB pattern may be observed, or detected, by the IMD using a far-field electrical signal, such as a far-field EGM. The IMD may determine and store the QRS morphology that corresponds to the detected RBBB pattern in the far-field electrical signal, or EGM. The IMD may also pace the RBB. In response to pacing the RBB, an LBBB pattern may be observed, or detected, using a far-field electrical signal, such as a far-field EGM. The IMD may determine and store the QRS morphology that corresponds to the detected LBBB pattern in the far-field electrical signal, or EGM. After calibration, the stored QRS morphologies corresponding to RBBB and LBBB patterns may be used to detect RBBB or LBBB conditions using the IMD. The IMD may then be able to correct for RBBB or LBBB by pacing the RBB earlier or LBB earlier according to techniques described herein. In some embodiments, an SD may be used to detect far-field electrical signals to detect dyssynchrony.

In some further embodiments, when a patient is known to have an existing LBBB or RBBB condition, the IMD may be used to memorize the corresponding LBBB or RBBB patterns of the condition using far-field electrical signals detected by the IMD. If the condition returns, which may be detected by comparing the memorized LBBB or RBBB patterns to measured far-field electrical signals, the IMD may operate to correct for LBBB or RBBB by pacing the RBB or the LBB earlier according to the techniques described herein.

In patients with pacing leads in RV and LV, the time difference between an RV sensed signal and an LV sensed signal may be used to represent the dyssynchrony between RV and LV. If the time difference increases, then dyssynchrony may be present, and pacing time in RV or LV may be adjusted or pacing time in LBB or RBB may be adjusted. In addition, the time of the greatest R-wave in a far-field EGM usually represents LV activation time. Thus, if the patient has only RV electrode (no LV electrode), then the EGM R-wave time may be compared with RV local time to determine the difference. If the difference increases, it may represent the presence of dyssynchrony.

**FIG. 13** shows a flowchart of one example of method **400,** which incorporates herein by reference method **300,** including all the same blocks shown and described in **FIG. 12****.** Method **400** may begin with block **302** by sensing heart activity and storing the data into memory **82.** At block **330,** a determination may be made as to whether QRS parameter, or activation interval, is greater than or equal to a threshold (T), such as 110, 120, 130, or 140 ms. One example of a QRS parameter is QRS duration. One example of an activation interval is a bundle-pacing to ventricular-activation interval.

If the QRS parameter is not greater than or equal to T, then heart activity may continue to be sensed at block **302.** The YES path from block **330** indicates the QRS parameter is greater or equal to T and method **400** may continue to adjust a time interval for bundle pacing. In particular, method **400** may continue to decision block **306** in which a determination is made as to whether to deliver pacing pulses earlier or later than the current timing. The time interval for bundle pacing may include, but is not limited to, an atrial-activation to bundle-pacing interval or a bundle-pacing to an LV- or RV-activation interval.

If a determination is made that the pacing pulses should be delivered later, the path may continue to block **308** in which the AV delay is increased (e.g., up to 10 ms steps) and may return to decision block **330;** otherwise, when pacing should be delivered earlier, the path may continue to block **310** to decrease the AV delay (e.g., up to 10 ms steps). Block **308** may return to block **330.**

After block **310,** decision block **332** may determine whether a QRS parameter or activation interval is less than a second threshold, such as 110, 120, 130, or 140 ms. The YES path from block **332** may return to sensing heart activity at block **302,** for example, because decreasing the AV delay in block **310** corrected the QRS parameter or activation interval. The NO path from block **332** may continue to decision block **334** in which a determination is made as to whether the QRS duration has increased.

In response to block **334,** if the QRS duration has not increased, method **400** may continue to block **336,** in which a counter M is increased by "1". At block **338,** if *M* = *Q*, where Q indicates a maximum number of iterations, then method **400** may continue to block **316.** Q may be an integer that is typically 10 or less and may ensure a number of loops are made to decrease the AV delay. If *M* ≠ *Q*, then method **400** may return to block **310** to continue decreasing AV delay. For example, QRS duration may decrease from 150 ms to 135 ms (e.g., a 15 ms step) such that the decrease in AV delay is in the right direction but may still be greater than 120 ms, so there may be a need to continue to decrease AV delay until QRS duration is less than or equal to 120 ms or adjustment is exhausted (e.g., M=Q). If the QRS duration has increased, method **400** may continue to block **340,** in which bundle pacing output (e.g., His pacing output or bundle branch pacing output) is increased.

At block **342,** a determination may be made as to whether QRS duration is less than a threshold (e.g., 120 ms). The YES path from block **342** may return to block **302** to continue sensing heart activity. In contrast, the NO path may continue to decision block **346** to determine whether QRS duration increased. The NO path may continue such that counter S is increased by "1" in which *S* = *S +* 1 at block **350** and then may continue to decision block **352** to determine whether *S* = *R* in which R is an integer of less than 10. If *S* = *R*, then method **400** may continue to block **316** in which the optimal AV delay is selected. The optimal AV delay may be automatically selected. Alternatively, a user may select an AV delay from the set of AV delays presented to the user on a graphical user interface presented through programmer **24** or a cell phone controlled by the user. The optimal AV delay for pacing may be saved in block **318.** Pacing may be delivered using optimal AV delay in block **320.**

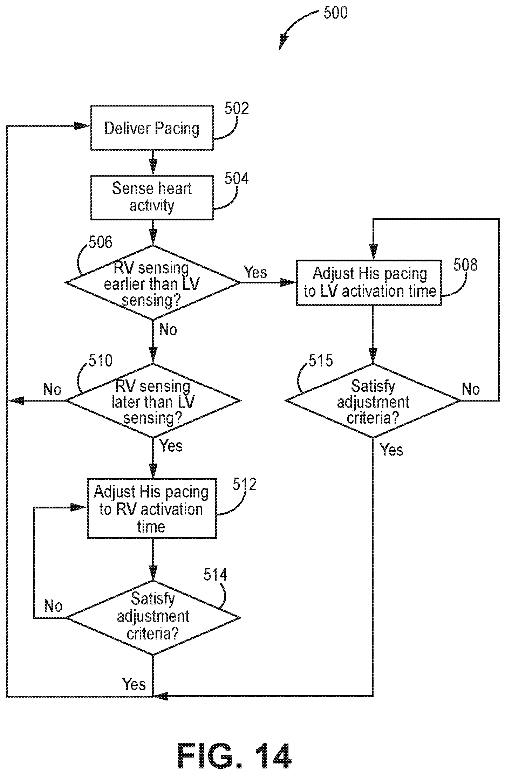

**FIG. 14** shows a flowchart of an example of method **500,** which may optimize VV delay for pacing of the His bundle or one or both bundle branches. At block **502,** the IMD **16** may deliver electrical stimuli (e.g., pacing pulses) to the His bundle or one or both bundle branches. At block **504,** heart activity may be sensed in the same or similar manner as described with respect to block **302.** At decision block **506,** a determination may be made as to whether RV sensing is occurring earlier than LV sensing (e.g., earlier than or at the same time). For example, during His or bundle branch pacing that corrects LBBB, if RV sensing is 50 ms earlier than LV sensing, this may mean that LV activation by bundle pacing is occurring later, so it may be beneficial to initiate bundle branch pacing for LBBB correction at an appropriate (earlier) time in synchronization with RV sensing. In another example, when an LV lead is available, if bundle-pacing to RV-sensing interval is 40 ms, and bundle-pacing to LV-sensing interval is 85 ms, then bundle branch pacing may be delivered and LV pacing with the LV lead may be adjusted to be delivered 40-50 ms earlier than bundle pacing, as a result, RV and LV activation may occur at almost the same time.

The YES path from block **506** may continue to block **508** in which bundle pacing may be adjusted to LV pacing timing (or LV activation), for example, when an LV lead is available. The NO path from block **506** may continue to decision block **510** in which a determination may be made as to whether RV sensing occurs later than LV sensing (e.g., later than or at the same time). The NO path from decision block **510** may return to delivering pacing at block **502** to the His bundle or one or both bundle branches and then sensing heart activity in response to the delivered pacing pulses at block **504.** The YES path from block **510** may continue to block **512** in which bundle pacing may be adjusted to RV pacing timing (or RV activation), for example, when an RV lead is available.

The path may continue to decision block **514** in which a determination may be made as to whether adjustment criteria are satisfied. One example of determining whether adjustment criteria are satisfied may include determining whether the timing of RV sensing and LV sensing are almost the same, such as less than or equal to about 40, 30, 20, or 10 ms. Another example of determining whether adjustment criteria are satisfied may include determining whether the RV-sensing to LV-sensing interval is optimal. The optimal the interval from RV-sensing to LV-sensing may be predetermined by monitoring, for example, using an echocardiogram, to find the optimal interval at which the cardiac output is the most, or highest, or mechanical and/or electrical synchronization is best, or optimal. Alternatively, the optimal interval from RV-sensing to LV-sensing can be predetermined using a 12-lead body surface ECG in which the interval generates the shortest QRS duration. Further, the optimal interval from RV-sensing to LV-sensing may be predetermined using an ECG belt (e.g., multiple-channel body surface mapping) that shows the most synchronized pattern at a particular interval from RV-sensing to LV-sensing. This predetermined interval may typically vary from about -20 ms to +40 ms, or around 0 ms, which may depend on where the RV and LV pacing leads are located in the ventricles.

The YES path from decision block **514** may continue to deliver pacing at block **502.** The NO path from decision block **514** may return to **512** to adjust the bundle pacing to RV activation timing.

The path from block **508** may continue to decision block **515** in which a determination may be made as to whether adjustment criteria are satisfied. The determination as to whether adjustment criteria are satisfied may be made in the same or similar manner as described with respect to block **514** (e.g., using the same adjustment criteria). The YES path from block **515** may continue to deliver pacing at block **502.** The NO path from decision block **515** may return to block **508** to adjust bundle pacing to LV activation timing.

While **FIGS. 12-14** are described relative to a particular pacing parameter (e.g., QRS parameters, such as QRS duration) to initiate the adjustment of an atrial-to-bundle pacing interval, other variables can also be used to adjust atrial-to-bundle pacing interval. Other illustrative pacing parameters that can be used include a change in the interval of bundle-pacing to RV- and/or LV-sensing or bundle-pacing to far-field R-wave. For example, if a bundle-pacing to RV-sensing interval is 80-100 milliseconds longer than a bundle-pacing to far-field R-wave interval, bundle pacing may be delayed at block **308** by a certain amount of time (e.g., 10 milliseconds etc.) until the bundle-pacing to RV-sensing interval is equal to or slightly longer than the bundle-pacing to far-field R-wave interval (if intrinsic activation available) or RV pacing may be initiated at an interval equal to or slightly longer than bundle-pacing to far-field R-wave interval.

In general, QRS parameters may include, but are not necessarily limited to, QRS duration, QRS morphology change, QRS vector change, or QRS pattern (e.g., RBBB or LBBB pattern).

In **FIG. 12****,** at block **306,** the "bundle pacing to RV sensing < 80 ms" can be a function of AV (or PR) interval, such as 40-70% of AV (or PR) interval, for example, if bundle pacing to RV sensing interval is less than 40% of AV or PR interval, the atrial to bundle-pacing interval by 10 ms may be increased (e.g., see the delay mechanism in **FIGS. 12** and **13****).** Additionally, the algorithm can distinguish the pattern of LBBB or RBBB so the bundle pacing can be better synchronized with either intrinsic activation or ventricular pacing. For example, when the bundle pacing is used to correct LBBB and the RV sensing is delayed near the end of the detected far-field ECG, suggesting the presence of delayed RV activation (RBBB), the bundle pacing should be delayed with a longer atrial to bundle-pacing interval for bundle pacing to be synchronized with activation from atria, or RV pacing should be delivered earlier so paced RV activation can be better synchronized with bundle pacing. Similarly, if RV sensing occurs too early at the very beginning of the far-field EGM (or far before LV sensing), suggesting bad synchronization, in such a case, bundle pacing may occur earlier, or RV pacing may be delayed. While the time adjustment of bundle pacing may be based on the QRS duration in some embodiments, it can also be based on the interval of bundle-pacing to RV- or LV-sensing or based on the detection of the presence of RBBB or LBBB (as described above) in case the device does not or cannot measure QRS duration. Additionally, the morphology of far- and near-field EGM and surface ECG can be used to estimate the activation delay in which ventricle. For example, in RV lead recording, if the EGM morphology of the RV tip-ring (the tip towards LV) is biphasic from the positive direction to the negative direction, meaning the propagation of the signal is from LV to RV. Consequently, the left bundle pacing should be delayed until a positive monophasic waveform followed with a relatively long small negative waveform is recorded. A similar method can be used to detect the activation propagation in the left ventricle.

The algorithm may also distinguish an LBBB pattern from an RBBB pattern using only far-field electrical signals. For example, the morphology of far-field EGM with LBBB (or RBBB) can be obtained during implantation by applying right bundle branch pacing (causing LBBB morphology) or left bundle branch pacing (causing RBBB morphology). If the patient already has an existing LBBB, then the device EGM can memorize the morphology of EGM when no pacing is applied. Different patients can have different EGM morphology for LBBB or RBBB, which may be obtained and personalized after implantation with left or right bundle branch pacing or by using existing LBBB/RBBB information.

The implantable medical device used in various embodiments of the present disclosure may be a leadless pacing device (LPD), which may communicate with another implantable medical device, such as a subcutaneous device.

**FIG. 15** shows an example system **610** that includes a subcutaneous device (SD) as IMD **630** (e.g., SICD, loop recorder (e.g., REVEAL^{®}), etc.) implanted exterior to a rib cage of patient **14** and leadless pacing device (LPD) **616** implanted within right atrium **26** of patient **14.** IMD **630** can be implanted external to a rib cage and within the vasculature. Additionally, or alternatively, an implantable medical device can be implanted substernally/retrosternally, as described in U.S. Patent Application 61/819,946, entitled "IMPLANTABLE MEDICAL DEVICE SYSTEM HAVING IMPLANTABLE CARDIAC DEFIBRILLATOR SYSTEM AND SUBSTERNAL LEADLESS PACING DEVICE" filed May 6, 2013, incorporated by reference in its entirety. In the example of **FIG. 15****,** system **610** includes LPD **616** and IMD **630.** External programmer **670** may be configured to communicate with one or both of LPD **616** and IMD **630.** Generally, there are no wires or other direct electrical (e.g., hardwired) connections between IMD **630** and LPD **616.** In this manner, any communication between IMD **630** and LPD **616** may be described as "wireless" communication. Patient **14** is ordinarily, but not necessarily, a human patient.

Illustrative IMD **630** includes housing **632** configured to be subcutaneously implanted outside the rib cage of patient **14.** The subcutaneous implantation location may be anterior to the cardiac notch, for example. In addition, housing **632** may carry three subcutaneous electrodes **634A-634C** (collectively "electrodes **634").** In other examples, housing **632** may carry fewer or greater than three electrodes. Lead **636** may be configured to couple to housing **632** and extend from housing **632** to a different subcutaneous location within patient **14.** For example, lead **636** may be tunneled laterally and posteriorly to the back of patient **14** at a location adjacent to a portion of a latissimus dorsi muscle. Lead **636** may carry electrode coil **638** along a length of lead **636** and sensing electrode **640** at a distal end of lead **636.** IMD **630** may be configured such that heart **12** may be disposed at least partially between housing **632** and electrode coil **638** of lead **636.** In some examples, lead **636** may carry two or more electrode coils **638** and/or two or more sensing electrodes **640.**

IMD **630** may contain, within housing **632,** signal processing and therapy delivery circuitry to detect cardiac conditions (e.g., ventricular dyssynchrony, arrhythmias such as bradycardia and tachycardia conditions etc.) and to communicate with LPD **616** to apply appropriate electrical stimuli (e.g. pacing and/or anti-tachyarrhythmia shock therapy (e.g., defibrillation or cardioversion shocking pulses)) to heart **12.** IMD **630** also may be configured to apply pacing pulses via one or more electrodes **634.** IMD **630** may be configured to apply the anti-tachyarrhythmia shock pulses between coil electrode **638** and one or more of electrodes **634** and/or electrically conductive housing **632** (e.g., an additional can electrode) of IMD **630.** IMD **630** may be configured to communicate with programmer **670** via an RF communication link, inductive coupling, or some other wireless communication protocol.

In some embodiments, IMD **630** differs from previously used ICDs in that housing **632** may be larger in size than the housing of some existing ICDs to accommodate larger capacity batteries, for example. In addition, IMD **630** may be implanted subcutaneously whereas some existing ICDs may be implanted under muscle or deeper within patient **14.** In other examples, housing **632** may be shaped or sized differently to be implanted subcutaneously instead of under a muscle or within deep tissue. Moreover, IMD **630** may not include leads configured to be placed in the bloodstream (e.g., endocardial or epicardial leads). Instead, IMD **630** may be configured to carry one or more electrodes (e.g., electrodes **634)** on housing **632** together with one or more subcutaneous leads (e.g., lead **636)** that carry defibrillation coil electrode **638** and sensing electrode **640.** In other examples, lead **636** may include additional electrodes. These subcutaneously implanted electrodes of IMD **630** may be used to provide therapies similar to that of existing ICDs without invasive vascular leads. In other examples, the exact configuration, shape, and size of IMD **630** may be varied for different applications or patients. Although IMD **630** is generally described as including one or more electrodes, IMD **630** may typically include at least two electrodes to deliver electrical stimulation (e.g., therapy) and/or provide at least one sensing vector. Other illustrative SDs, or IMD **630,** can be used in combination with LPD **616.** For example, IMD **630** may include, or be, an intravenously implanted device (IID), an ICD, a pacemaker, or any other suitable device.

System **610** also includes one or more LPDs, such as LPD **616.** LPD **616** may be, for example, an implantable leadless pacing device (e.g., a pacemaker, cardioverter, and/or defibrillator) that provides electrical stimulation to heart **12** via electrodes carried on the housing of LPD **616.** In the example of **FIG. 15****,** LPD **616** is implanted within left ventricle **32** of heart **12** to sense electrical activity of heart **12** and/or deliver electrical stimulation, e.g., CRT such as fusion pacing, to heart **12.** Fusion pacing involves left ventricle (LV) **32** only pacing with an electrode on the LPD **616** in coordination with intrinsic right ventricle (RV) activation. Alternatively, fusion pacing can involve pacing RV **28** with an electrode on LPD **616** in coordination with intrinsic LV activation. In this scenario, LPD **616** is placed within the RV **28.**

LPD **616** is schematically shown in **FIG. 15** attached to a wall of right atrium (RA) **26** via one or more fixation elements (e.g. tines, helix etc.) that penetrate the tissue. These fixation elements may secure LPD **616** to the cardiac tissue and retain an electrode (e.g., a cathode or an anode) in contact with the cardiac tissue. One or more electrodes may be positioned to stimulate the tissue of RA **26,** LV **32,** or both RA **26** and LV **32.** LPD **616** may also include one or more motion sensors (e.g., accelerometers) configured to detect and/or confirm cardiac conditions (e.g., ventricular dyssynchrony, tachyarrhythmias etc.) from mechanical motions of heart **12.** Since LPD **616** may include two or more electrodes carried on the exterior housing of LPD **616,** no other leads or structures may need to reside in other chambers of heart **12.** However, in other examples, system **610** may include additional LPDs within respective chambers of heart **12** (e.g., left atrium (LA) **33,** RV **28,** and/or LV **32).**

Using the electrodes carried on the housing of LPD **616,** LPD **616** may be capable sensing intrinsic electrical signals, e.g., an electrocardiogram (ECG). IMD **630** may similarly sense intrinsic electrical signals from the sensing vectors of electrodes **634, 638,** and **640.** These intrinsic signals may be electrical signals generated by cardiac muscle and indicative of depolarizations and repolarizations of heart **12** at various times during the cardiac cycle. LPD **616** may generate electrograms from these cardiac signals that may be used by LPD **616** to detect cardiac conditions (e.g. ventricular dyssynchrony, arrhythmias, such as tachyarrhythmias), or identify other cardiac events, e.g., ventricle depolarizations or atrium depolarizations. LPD **616** may also measure impedances of the carried electrodes and/or determine capture thresholds of those electrodes intended to be in contact with cardiac tissue. In addition, LPD **616** may be configured to communicate with external programmer **670.** The configurations of electrodes used by LPD **616** for sensing and pacing may be typically considered bipolar but unipolar may also be used.

External programmer **670** may be configured to communicate with one or both of IMD **630** and LPD **616.** In examples which external programmer **24** only communicates with one of IMD **630** and LPD **616,** the non-communicative device may receive instructions from or transmit data to the device in communication with programmer **670.** In some examples, programmer **670** includes a handheld computing device, computer workstation, or networked computing device. Programmer **670** may include a user interface that receives input from a user. In other examples, the user may also interact with programmer **670** remotely via a networked computing device. The user may interact with programmer **670** to communicate with LPD **616** and/or IMD **630.** For example, the user may interact with programmer **670** to send an interrogation request and retrieve therapy delivery data, update therapy parameters that define therapy, manage communication between LPD **616** and/or IMD **630,** or perform any other activities with respect to LPD **616** and/or IMD **630.** Although the user may be a physician, technician, surgeon, electrophysiologist, or other healthcare professional, the user may be patient **14** in some examples.

Programmer **670** may also allow the user to define how LPD **616** and/or IMD **630** senses electrical signals (e.g., ECGs), detects cardiac conditions (e.g. ventricular dyssynchrony, arrhythmias etc.), delivers therapy, and communicates with other devices of system **610.** For example, programmer **670** may be used to change detection parameters. In another example, programmer **670** may be used to manage therapy parameters that define therapies such as CRT. Moreover, programmer **670** may be used to alter communication protocols between LPD **616** and IMD **630.** For example, programmer **670** may instruct LPD **616** and/or IMD **630** to switch between one-way and two-way communication and/or change which of LPD **616** and/or IMD **630** are tasked with initial detection of a cardiac condition.

Programmer **670** may communicate with LPD **616** and/or IMD **630** via wireless communication using any techniques known in the art. Examples of communication techniques may include, for example, radiofrequency (RF) telemetry, but other techniques are also contemplated. In some examples, programmer **670** may include a programming head that may be placed proximate to the patient's body near the LPD **616** and/or IMD **630** implant site in order to improve the quality or security of communication between LPD **616** and/or IMD **630** and programmer **670.**

LPD **616** and IMD **630** may engage in communication to facilitate appropriate detection of ventricular dyssynchrony and/or delivery of CRT. The communication may include one-way communication in which one device is configured to transmit communication messages and the other device is configured to receive those messages. The communication may include two-way communication in which each device is configured to transmit and receive communication messages. LPD **616** and IMD **630** may be configured to communicate with each other provide alternative electrical stimulation therapies.

Although LPD **616** may at least partially determine whether or not LPD **616** delivers CRT or another therapy to patient **14,** LPD **616** may perform one or more functions in response to receiving a request from IMD **630** and without any further analysis by LPD **616.** In this manner, IMD **630** may act as a master device and LPD **616** may act as a "slave" device. In this configuration, LPD **616** passively senses. Specifically, a mode may be employed as a trigger mode to pace in synchrony (e.g., a WT mode). In one or more embodiments, LPD **616** may be configured to actively sense.

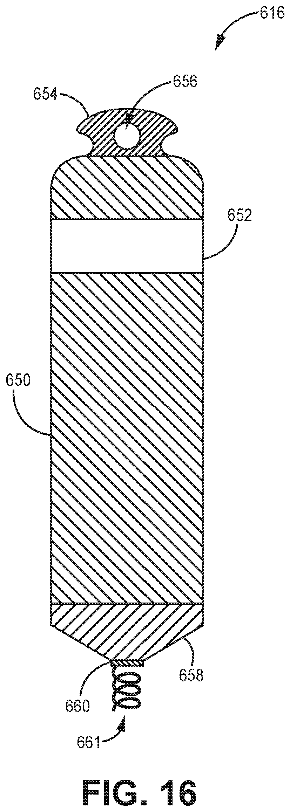

**FIG. 16** shows one example of LPD **616** of **FIG. 15****.** As shown in **FIG. 16****,** LPD **616** includes case **650,** cap **658,** electrode **660,** electrode **652,** tissue-piercing electrode assembly **661** (which may also function as a fixation mechanism (e.g., with a helix shape)), flange **654,** and opening **656.** In general, the tissue-piercing electrode assembly **661** extends axially from a housing of LPD **616.** Together, case **650** and cap **658** may be considered the housing of LPD **616.** In this manner, case **650** and cap **658** may enclose and protect the various electrical components within LPD **616.** Case **650** may enclose substantially all the electrical components, and cap **658** may seal case **650** and create the hermetically sealed housing of LPD **616.** Although LPD **616** is generally described as including one or more electrodes, LPD **616** may typically include at least two electrodes (e.g., electrodes **652** and **660**) to deliver an electrical signal (e.g., therapy such as CRT) and/or provide at least one sensing vector. Electrodes **652** and **660** are carried on the housing created by case **650** and cap **658.** In this manner, electrodes **652** and **660** may be considered leadless electrodes. In the example of **FIG. 15****,** electrode **660** is disposed on the exterior surface of cap **658.** Electrode **660** may be a circular electrode positioned to contact cardiac tissue upon implantation. Electrode **652** may be a ring or cylindrical electrode disposed on the exterior surface of case **650.** Both case **650** and cap **658** may be electrically insulating. Electrode **660** may be used as a cathode and electrode **652** may be used as an anode, or vice versa, for delivering CRT or other appropriate cardiac therapy (ATP, shock, etc.). However, electrodes **652** and **660** may be used in any stimulation configuration. In addition, electrodes **652** and **660** may be used to detect intrinsic electrical signals from cardiac muscle. In other examples, LPD **616** may include three or more electrodes, where each electrode may deliver therapy and/or detect intrinsic signals. CRT delivered by LPD **616** may be considered to be "painless" to patient **14** or even undetectable by patient **14** since, e.g., the electrical stimulation occurs very close to or at cardiac muscle and at relatively low energy levels compared with alternative devices.

One or more fixation mechanisms of tissue-piercing electrode assembly **661** may attach LPD **616** to cardiac tissue. The fixation mechanisms may include a helix (as illustrated), active fixation tines, screws, clamps, adhesive members, or any other types of attaching a device to tissue. In some embodiments, fixation mechanisms may be constructed of a memory material that retains a preformed shape. During implantation, fixation mechanisms may be flexed forward to pierce tissue and allowed to flex back towards case **650.** In this manner, fixation mechanisms may be embedded within the target tissue.

Flange **654** may be provided on one end of case **650** to enable tethering or extraction of LPD **616.** For example, a suture or other device may be inserted around flange **654** and/or through opening **656** and attached to tissue. In this manner, flange **654** may provide a secondary attachment structure to tether or retain LPD **616** within heart **12** if fixation mechanisms fail. Flange **654** and/or opening **656** may also be used to extract LPD **616** if explantation (or removal) from patient **14** is desired.

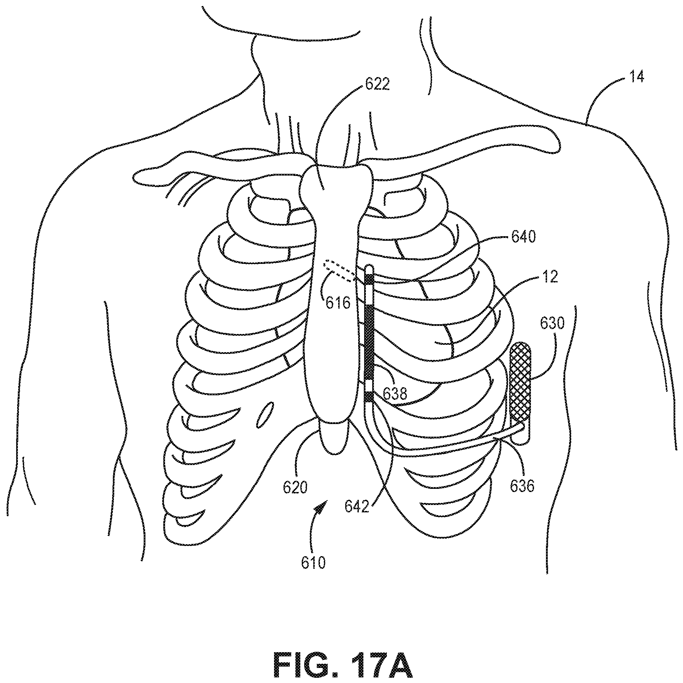

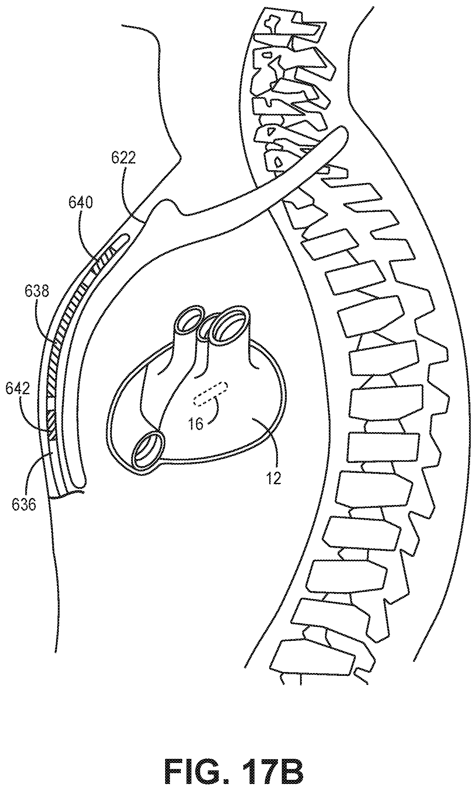

**FIGS. 17A-17B** are conceptual diagrams of patient **14** implanted with implantable cardiac system **610** that includes LPD **616** inside the heart in order to deliver bundle pacing to His bundle or bundle branch tissue. Implantable cardiac system **610** that contains a substernal/subcutaneous device (SD), or IMD **630,** can implement method **300-500** as described herein. **FIG. 17A** is a front view of patient **14** implanted with implantable cardiac system **610.** **FIG. 17B** is a side view patient **14** with implantable cardiac system **610.**

Implantable cardiac system **610** includes an implantable medical device (IMD) **630** such as an implantable cardiac defibrillator (ICD) or pacemaker connected to a defibrillation lead **636.** In the example illustrated in **FIGS. 17A-B**-, IMD **630** is implanted subcutaneously on the left midaxiallary of patient **14.** IMD **630** may, however, be implanted at other subcutaneous locations on patient **14** as described herein.

Defibrillation lead **636** includes a proximal end that is connected to IMD **630** and a distal end that includes one or more electrodes. Defibrillation lead **636** extends subcutaneously from IMD **630** toward xiphoid process **620.** At a location near xiphoid process **620,** defibrillation lead **636** bends or turns and extends subcutaneously superiorly, substantially parallel to sternum **622.** The distal end of defibrillation lead **636** may be positioned near the second or third rib of patient **14.** However, the distal end of defibrillation lead **636** may be positioned further superior or inferior depending on the location of IMD **630** and other factors. Although illustrated as being offset laterally from and extending substantially parallel to sternum **622** in the example of **FIGS. 17A-B,** defibrillation lead **636** may be implanted over sternum **622,** offset from sternum **622,** but not parallel to sternum **622** (e.g., angled lateral from sternum **622** at either the proximal or distal end).

Defibrillation lead **636** includes defibrillation electrode **638,** which may be an elongated coil electrode, toward the distal end of defibrillation lead **636.** Defibrillation lead **636** is placed such that a therapy vector between defibrillation electrode **638** and a housing or can electrode of IMD **630** is substantially across the ventricle of heart **12.**

Defibrillation lead **636** may also include sensing and/or pacing electrodes **640** and **642** located toward the distal end of defibrillation lead **636.** In the example illustrated in **FIGS. 17A-B,** sensing electrode **640** and **642** are separated from one another by defibrillation electrode **638.** IMD **630** may sense electrical activity of heart **12** via a combination of sensing vectors that include combinations of electrodes **640** and **642** and the housing or can electrode of IMD **630.** For example, IMD **630** may obtain electrical signals sensed using a sensing vector between electrodes **640** and **642,** obtain electrical signals sensed using a sensing vector between electrode **640** and the conductive housing or can electrode of IMD **630,** obtain electrical signals sensed using a sensing vector between electrode **642** and the conductive housing or can electrode of IMD **630,** or a combination thereof. In some instances, IMD **630** may even sense cardiac electrical signals using a sensing vector that includes defibrillation electrode **638.**

IMD **630,** which may be an ICD, may analyze the sensed electrical signals from one or more of the sensing vectors of defibrillation lead **636** to detect ventricular dyssynchrony (e.g., widening QRS duration, time difference between RV and LV activation components in ECG vectors, and atrial signal of P wave in ECG) and/or other cardiac conditions (e.g., tachycardia, fibrillation). In response to detecting the ventricular dyssynchrony, IMD **630** may communicate with LPD **616** to initiate bundle pacing at a particular timing for resynchronization and/or cardiac resynchronization therapy pacing in an attempt to terminate the ventricular dyssynchrony. The means of communication between LPD **616** and IMD **630** is the same or similar as that which is described herein.

LPD **616** is implanted in the His bundle or bundle branch region and communicatively coupled to IMD device **630.** LPD **616** and IMD device **630** may, for example, both include a communication module via which the devices exchange wireless communications. LPD **616** and IMD device **630** may, for example, be coupled via inductive coupling, RF coupling, tissue conductance communication, or other wireless communication mechanism.

As indicated above, LPD **616** may be implanted in the His bundle region or bundle branch region. LPD **616** is configured to include a housing, electrodes coupled to the housing or formed by the housing, and a fixation mechanism (e.g., helix) to attach LPD **616** at a His bundle location.

LPD **616** may sense electrical activity of heart **12** via electrodes and provide pacing pulses to heart **12** via the same or different electrodes. The pacing pulses provided to heart **12** may be responsive to sensed electrical signals of the heart sensed either via electrodes of LPD **616** or sensed via one or more electrode combinations of defibrillation lead **636.** LPD **616** may generate and deliver pacing pulses with any of a number of amplitudes and pulse widths to capture heart **12.**

LPD **616** may also analyze the sensed electrical signals from one or more of the sensing vectors of LPD **616** and/or from the IMD to detect ventricular dyssynchrony. LPD **616** may not deliver pacing therapy until LPD **616** receives communication from IMD **630** indicating detection of ventricular dyssynchrony by IMD **630.**

The configuration described above in **FIGS. 17A-B** is directed to providing ventricular pacing via LPD **616** positioned near the His bundle or one or both bundles. In some instances, more than one LPD **616** may be utilized for multi-chamber pacing, e.g., with one LPD **616** providing atrial pacing and another LPD **616** providing ventricle pacing. Alternatively, LPD **616** may be positioned over the ventricle and include a small tether extending up to the atrium with an electrode on the tether. LPD **616** could sense and/or pace via the electrode on the tether. In yet further embodiments, LPD **616** may be used in combination with a pacing lead implanted substernally to provide dual chamber pacing.

IMD **630** may include a housing that forms a hermetic seal that protects components of IMD **630.** The housing of IMD **630** may be formed of a conductive material, such as titanium. IMD **630** may also include a connector assembly (also referred to as a connector block or header) that includes electrical feedthroughs through which electrical connections are made between conductors within the lead **636** and electronic components included within the housing. As will be described in further detail herein, housing may house one or more processors, memories, transmitters, receivers, sensors, sensing circuitry, therapy circuitry and other appropriate components. The housing is configured to be implanted in a patient, such as patient **14.**

As described herein, lead **636** includes a lead body that may include electrodes **638, 640, 642** located near the distal lead end or elsewhere along the length of the lead body. The lead bodies of lead **636** also contain one or more elongated electrical conductors (not illustrated) that extend through the lead body from a connector assembly of IMD **630** provided at a proximal lead end to one or more electrodes of lead **636.** The lead bodies of lead **636** may be formed from a non-conductive material, including silicone, polyurethane, fluoropolymers, mixtures thereof, and other appropriate materials, and shaped to form one or more lumens within which the one or more conductors extend. However, the techniques are not limited to such constructions.

The one or more elongated electrical conductors contained within the lead bodies of lead **636** may engage with respective ones of electrodes **638, 640, 642.** In one example, each of electrodes **638, 640, 642** is electrically coupled to a respective conductor within its associated lead body. The respective conductors may electrically couple to circuitry, such as a therapy module or a sensing module, of IMD **630** via connections in connector assembly, including associated feedthroughs. The electrical conductors transmit therapy from a therapy module within IMD **630** to one or more of electrodes **638, 640, 642** and transmit sensed electrical signals from one or more of electrodes **638, 640, 642** to the sensing module within IMD **630.**

The examples illustrated in **FIGS. 17A-B** are illustrative in nature and should not be considered limiting of the techniques described in this disclosure. In other examples, IMD **630** and defibrillation lead **636** may be implanted at other locations. For example, IMD **630** may be implanted in a subcutaneous pocket in the right chest. In this example, defibrillation lead **636** may be extend subcutaneously from the device toward the manubrium of the sternum and bend or turn and extend subcutaneously inferiorly from the manubrium of the sternum, substantially parallel with the sternum.

System **610** may be further described as an IMD system that provides, but the techniques may be applicable to other cardiac systems, including cardiac resynchronization therapy defibrillator (CRT-D) systems, cardioverter systems, or combinations thereof.

The substernal/retrosternal IMD system **610** can be configured to deliver bundle pacing to synchronize the ventricles with each other. Bundle pacing includes pacing the His bundle or one or both bundles with one or more pacing electrodes. Pacing electrodes can be found on the bundle pacing lead or leadless pacing device. In addition, or alternatively, electrodes **640** and **642** of IMD **630** can be configured to deliver pacing pulse to the LV. The LV and/or RV is paced by separate electrodes.

Bundle pacing may be delivered while another type of pacing may be delivered to another tissue site. Illustrative cardiac pacing system that may employ both bundle pacing to a His bundle or bundle branch tissue site and another pacing therapy delivered to another pacing site (e.g., left ventricle or right ventricle) is disclosed in U.S. Patent No. 9,789,319, entitled SYSTEMS AND METHODS FOR LEADLESS CARDIAC RESYNCHRONIZATION THERAPY that issued October 17, 2017, which is incorporated herein by reference in its entirety. U.S. Patent No. 9,789,319 discloses, for example, a subcutaneous device and LPD in communication with each other. As applied to the present disclosure, the leadless pacing device (LPD) would be configured to deliver bundle pacing using an elongated helical tip that is similar or the same as the helical tip, for example, on the Medtronic 3830 lead.

The techniques described in this disclosure, including those attributed to an IMD, such as LPD **616,** programmer **670,** or various constituent components, may be implemented, at least in part, in hardware, software, firmware, or any combination thereof. For example, various aspects of the techniques may be implemented within one or more processors, including one or more microprocessors, DSPs, ASICs, FPGAs, or any other equivalent integrated or discrete logic circuitry, as well as any combinations of such components, embodied in programmers, such as physician or patient programmers, stimulators, image processing devices, or other devices. The term "processor" or "processing circuitry" may generally refer to any of the foregoing logic circuitry, alone or in combination with other logic circuitry, or any other equivalent circuitry.

Such hardware, software, and/or firmware may be implemented within the same device or within separate devices to support the various operations and functions described in this disclosure. In addition, any of the described units, modules or components may be implemented together or separately as discrete but interoperable logic devices. Depiction of different features as modules or units is intended to highlight different functional aspects and does not necessarily imply that such modules or units must be realized by separate hardware or software components. Rather, functionality associated with one or more modules or units may be performed by separate hardware or software components, or integrated within common or separate hardware or software components.

To achieve the best pacing output, bundle pacing may be synchronized with either intrinsic activation or either RV or LV pacing as described herein. In some embodiments, bundle pacing may be terminated only when a normal ventricular pattern with appropriate atrioventricular conduction interval is detected.

When implemented in software, the functionality ascribed to the systems, devices, and techniques described in this disclosure may be embodied as instructions on a computer-readable medium such as RAM, ROM, NVRAM, EEPROM, FLASH memory, magnetic data storage media, optical data storage media, or the like. The instructions may be executed by one or more processors to support one or more aspects of the functionality described in this disclosure.

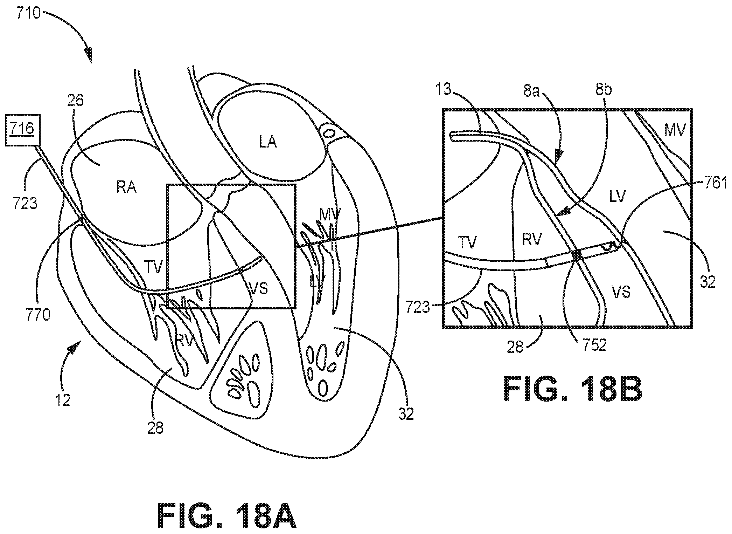

**FIGS. 18A-B** show patient's heart **12** implanted with IMD **716** operably coupled to implantable medical electrical lead **723** to deliver bundle branch pacing according to one example of an IMD system **710.** **FIG. 18B** **is** a close-up view of lead **723** in the patient's heart **12** of **FIG. 18A****.** In some embodiments, electrical lead **723** may be the only lead implanted in the patient's heart **12.**

In particular, lead **723** may be configured for dual bundle branch pacing. Lead **723** may be the same as or similar to lead **23** (**FIGS. 2A-B**), except lead **723** is implanted near the bundle branches instead of, for example, the His bundle **13.** As illustrated, lead **723** is implanted in the septal wall from RV **28** toward LV **32.** Lead **723** may not pierce through the wall of LV **32** or extend into the LV chamber. Electrode **752** and tissue-piercing electrode assembly **761** may be disposed on a distal end portion of lead **723,** which may also be described as a shaft. Electrode **752** and tissue-piercing electrode assembly **761** may be the same as or similar to electrode **652** and tissue-piercing electrode assembly **661** (**FIG. 16**), except electrode **752** is configured as a cathode electrode to sense or pace the RBB and electrode assembly **761** is configured to sense or pace the LBB, for example, during dual bundle branch pacing. Accordingly, electrode **752** may be implanted near RBB **8b,** and electrode assembly **761** may be implanted near LBB **8a.**

Electrode assembly **761** may be described as a unipolar cathode electrode, which may be implanted on the left side of the patient's septum. Electrode **752** may be described as a unipolar cathode electrode, which may be implanted on the right side of the patient's septum.

During dual bundle branch pacing, both electrode **752** and electrode assembly **761** (which also includes an electrode) may each deliver a cathodal pulse to achieve synchronized activation, or excitation, of RBB **8b** and LBB **8a,** which may result in synchronized activation of RV **28** and LV **32.** In some embodiments, the pulses may be delivered at the same time to achieve synchrony. In other embodiments, the pulses may be delivered with a delay to achieve synchrony.

Lead **723** may include electrode **770** disposed more proximal to the electrode **752** and electrode assembly **761.** Electrode **770** may be positioned in or near RA **26** and may function as an anode for cathodal pulses from electrode **752** and/or electrode assembly **761.**

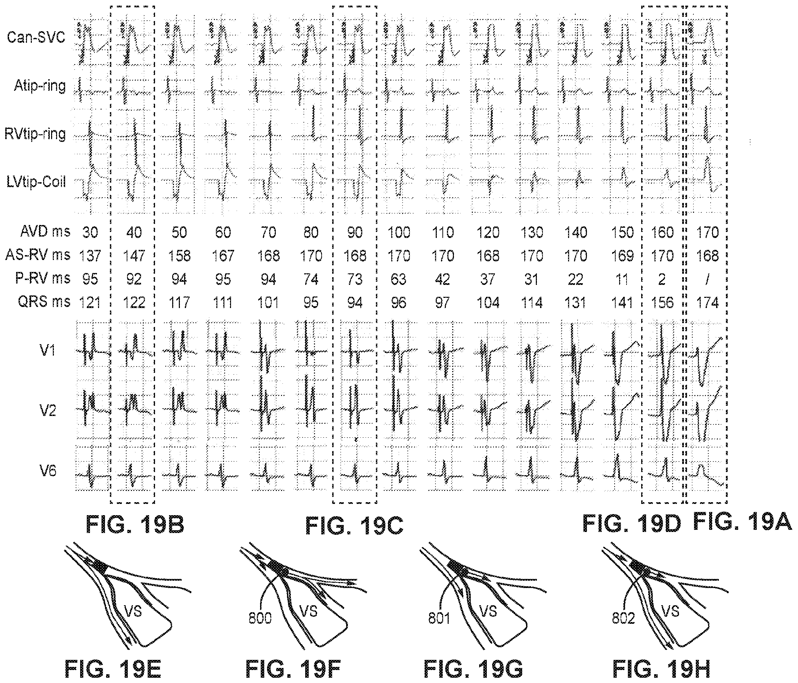

**FIGS. 19A-H** show electrical signals resulting from various pacing configurations applied to a patient with an LBBB. The electrical signals shown include electrogram (EGM) and electrocardiogram (ECG) information. **FIG. 19E** is similar to **FIG. 8A** in that a LBBB is shown without pacing. **FIGS. 19F-H** show various pacing configurations **800, 801, 802** at a distal His bundle pacing site similar to **FIG. 8C****.** In particular, **FIG. 19F** shows a pacing configuration **800** that uses an AV delay of 40 ms, which results in the LV activating earlier than the RV as shown in the electrical signals of **FIG. 19B. FIG. 19G** shows pacing configuration **801** that uses an AV delay of 90 ms, which results in the LV activating simultaneously with the RV as shown in the electrical signals of **FIG. 19C. FIG. 19H** shows pacing configuration **802** that uses an AV delay of 160 ms, which results in the LV activating later than the RV as shown in the electrical signals of **FIG. 19D. FIG. 19A** shows resulting electrical signals of a pacing configuration (not shown) that uses an AV delay of 170 ms and looks like a full LBBB pattern, similar to intrinsic LBBB without pacing.

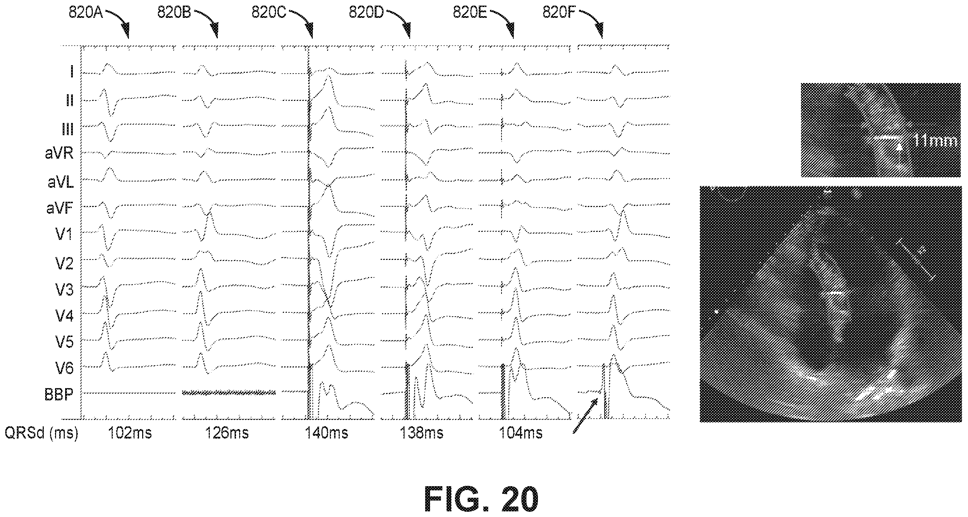

**FIG. 20** shows electrical signals resulting from positioning a bundle branch pacing lead (see, for example, **FIGS. 18A-B**) at various locations in a patient with an LBBB and the resulting QRS duration (QRSd) in milliseconds (ms). In particular, electrical signals correspond to various locations as the bundle branch pacing lead advances from the right side to the left side of the bundle branches. Electrical signals **820A** correspond to no pacing lead in the patient's heart (intrinsic) and a resulting QRSd of 102 ms. Electrical signals **820B** correspond to the lead near the LBB without pacing resulting in a QRSd of 126 ms. Electrical signals **820C** correspond to pacing near the RBB resulting in a QRSd of 140 ms, which may be characterized as a wide QRSd. Electrical signals **820D** correspond to pacing in the middle of the RBB and LBB resulting in a QRSd of 138 ms. Electrical signals **820E** correspond to pacing near the LBB resulting in a QRSd of 104 ms. Electrical signals **820F** correspond to the lead near the LBB without pacing while recording bundle branch potentials, which looks similar to electrical signals **820B.**

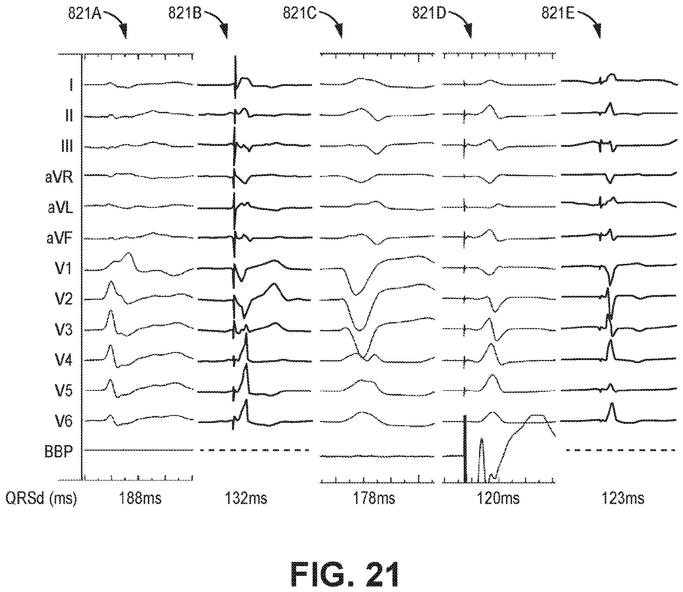

**FIG. 21** shows electrical signals resulting from positioning a bundle branch pacing lead (see, for example, **FIGS. 18A-B**) at various locations in various patients with an RBBB or an LBBB and the resulting QRSd in milliseconds (ms). Electrical signals **821A, 821B** correspond to a patient having an RBBB. Electrical signals **821A** correspond to no pacing lead in the patient's heart and a resulting QRSd of 188 ms. Electrical signals **821B** correspond to pacing near the RBB resulting in a QRSd of 132 ms, which corrects the RBBB. Electrical signals **821C** to **821E** correspond to a patient having an LBBB. Electrical signals **821C** correspond to no pacing lead in the patient's heart (intrinsic) resulting in a QRSd of 178 ms. Electrical signals **821D** correspond to pacing near the LBB resulting in a QRSd of 120 ms, which is a narrow QRS. Electrical signals **821E** correspond to recording after implantation during LBB pacing and a resulting QRSd of 123 ms.

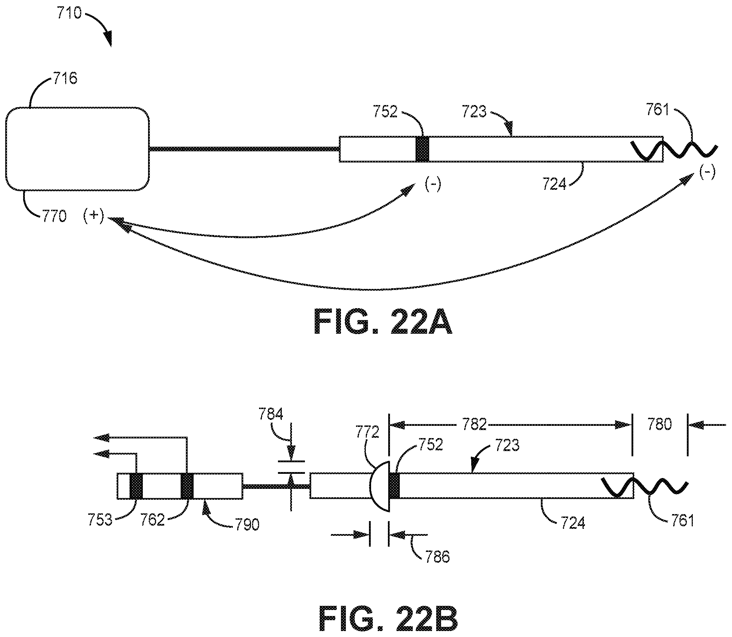

**FIGS. 22A-B** show various aspects of IMD system **710** including implantable medical lead **723** that may be used for bundle pacing, particularly dual bundle branch pacing. As illustrated in **FIG. 22A****,** medical lead **723** may be directly coupled to IMD **716.** IMD **716** may function as an electrode **770,** for example, using a discrete electrode or a housing of IMD **716.** As illustrated in **FIG. 22B****,** lead **723** may include lead connector **790** disposed on a proximal end portion. Lead connector **790** may include contacts **753, 762** operably coupled to electrode **752** and electrode assembly **761,** respectively, to operably couple with electronic circuitry of IMD **716,** for example, when lead connector **790** is inserted into IMD **716.**

Lead **723** may also include protrusion **772** (e.g., a shield or stopper) that may prevent electrode assembly **761** of lead **723** from advancing too far into cardiac tissue. In particular, protrusion **722** may prevent electrode assembly **761** of lead **723** from perforating the LV wall and extending into the LV. Protrusion **722** may extend laterally from a nominal surface of lead **723** to engage a wall of cardiac tissue when being implanted. Protrusion **722** may have a biased shape that facilitates the ease of retracting lead **723** while still preventing further advancement of lead **723** when protrusion **722** engages cardiac tissue. Protrusion **722** may be formed of any suitable material to facilitate its functionality. Protrusion **722** may be formed of a flexible, soft, and/or expansible material.

Protrusion **722** may have a height, or width, **784** extending orthogonally from the nominal surface of lead **723,** such as the surface of distal end portion **724** of lead **723.** Height **784** may be any suitable range to facilitate preventing further advancement of lead **723** when engaging cardiac tissue while also being small enough to facilitate ease of implantation. In some embodiments, height **784** may be greater than or equal to about 0.5, 1, 1.5, 2, 2.5, or 3 mm. In some embodiments, height **784** may be less than about 3, 2.5, 2, 1.5, 1, or 0.5 mm.

Protrusion **722** may have length **786** extending along, or parallel to, the nominal surface of lead **723,** for example, in a longitudinal direction. In some embodiments, length **786** may be greater than or equal to about 0.5, 1, 1.5, 2, 2.5, or 3 mm. In some embodiments, length **786** may be less than or equal to about 3, 2.5, 2, 1.5, 1, or 0.5 mm.

Distal end portion **724** of lead **723** may have length **782** in any suitable range to facilitate positioning electrode assembly **761** near a His bundle or bundle branch region. In some embodiments, length **782** may be greater than or equal to about 4, 5, 6, 7, 8, 9, 10, 11, 12, 13, 14, 15, 16, 17, or 18 mm. In some embodiments, length **782** may be less than about 4, 5, 6, 7, 8, 9, 10, 11, 12, 13, 14, 15, 16, 17, or 18 mm.

Electrode assembly **761** may have length **780** extending out from an end of distal end portion **724** in any suitable range to facilitate positioning electrode assembly **761** near the His bundle or bundle branch region. Some or all of electrode assembly **761** may function as an electrode. In some embodiments, length **780** may be greater than or equal to about 0.5, 1, 1.5, 2, 2.5, 3, 3.5, 3.75, 4, or 10 mm. In some embodiments, length **780** may be less than about 0.5, 1, 1.5, 2, 2.5, 3, 3.5, 3.75, 4, or 10 mm.

In general, lengths of protrusion **722,** distal end portion **724,** and/or electrode assembly **761** may be selected to facilitate positioning electrode **752,** electrode assembly **761,** or both electrode **752** and electrode assembly **761** near the His bundle or one or both bundle branches. The various lengths may be determined, for example, based on a septum thickness. Septum thickness may be determined using an echocardiogram.

AV delay may be programmed using sensing from an atrial electrode, such as sensing from electrode **770** of **FIG. 18A****,** which may be used only for sensing in some embodiments. The atrial electrode may be positioned, for example, about 5 to 10 cm proximal to the distal end of lead **723** or electrode assembly **761.** In other words, the atrial electrode may be spaced 5 cm or more from one or both cathode electrodes. The AV delay, or time from atrial activation to bundle pacing, may be appropriately set using the atrial electrode.

One embodiment of lead implantation method may incorporate ECG and impedance guided lead placement employing the lead shown in **FIG. 22A****.** In general, the lead may include an ECG transition during implantation that can be used to determine where the pacing lead is located. For example, the ECG QRS morphology may change, which represents a transition from an LBBB pattern or delay to an RBBB pattern or delay, as the pacing lead is used to intermittently pace during implantation from the right side of the ventricular septum to the left side. If the ECG morphology indicates the tip is near the endocardium of the ventricular septum, the lead insertion may be stopped. During the lead insertion through the septum, the pacing impedance may be monitored. If a sudden change in pacing impedance is detected (such as drop by 10-30%), the pacing lead may be pulled back by rotating the lead, for example, counterclockwise about 1-3 turns. As the left bundle branch is located beneath the endocardium, use of these two assessments can avoid the lead perforation into the LV. Once the pacing lead is in place, electrical output may be applied to one or both right and left bundle branches in a synchronized manner, which may excite the ventricles simultaneously, almost simultaneously, or using a short delay.

Synchronized pacing of one or both bundle branches may be based on an atrial event, a ventricular event, or both. In some embodiments, synchronized pacing may deliver two or more pacing pulses within the same cardiac cycle, for example, one to each ventricle.

In some embodiments, a lead including two or more pacing electrodes may be used to deliver differently paced pulses to the bundle of His or one or both bundle branches. Differently paced pulses are not delivered simultaneously or sequentially to the His bundle or bundle branch tissue. Instead, a pulse to a first tissue may be paced at a first time, and a pulse to a second tissue may be pulsed at a second time. A delay may be used between the first pulse and the second pulse.

If the lead shown in **FIG. 22B** is used, then echocardiography may be performed before implantation to assess the thickness of the ventricular septum. A pacing lead may be selected or chosen with an appropriate distance from electrode assembly, or distal end portion or tip of the lead, to the proximal cathode electrode. The selected pacing lead may also include a stopper to prevent perforation of the LV. Pacing output may be applied to one or both bundle branches in a synchronized manner, simultaneously (or synchronized) so both ventricles can be excited in a synchronized manner, which may excite the ventricles simultaneously, almost simultaneously, or using a short delay.

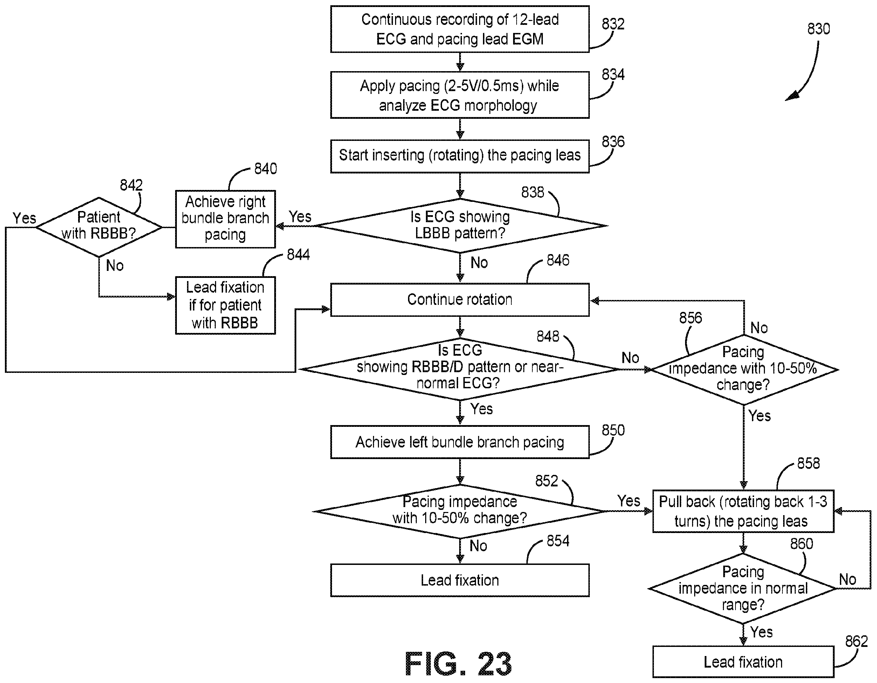

**FIG. 23** is a flow diagram that shows an example of a method of lead implantation for bundle branch pacing for a leaded or leadless IMD. Method **830** may include one or more of the blocks illustrated. Method **830** may begin, for example, once the implantable medical lead makes contact with the septal wall. Method **830** may include block **832** to continuously record a 12-lead ECG and a pacing lead EGM. Method **830** may include block **834** to apply pacing from the pacing lead while analyzing ECG morphology. In some embodiments, the pacing output amplitude may be about 2 to 5 V and a pulse width duration may be about 0.5 ms.

In block **836,** the lead may be inserted into the septal wall, which may include rotating the lead. In block **838,** the ECG may be analyzed to determine whether an LBBB pattern is present. If the answer to block **838** is YES, method **830** may continue to block **840,** whereas if the answer is NO, method **830** may continue to block **846.**

In block **840,** method **830** may determine that an RBB pacing location has been achieved, for example, based on detecting the LBBB pattern in block **838.** In block **842,** method **830** may determine whether the patient has RBBB. If the answer to block **842** is NO, then the lead may be fixed in the RBB location in block **844,** whereas if the answer is YES, then the lead may continue to be rotated in block **846.** For example, in block **846,** the lead may advance a short distance, for example, by rotating 1-3 times in a clockwise direction.

After rotation, method **830** may determine whether the ECG shows an RBBB pattern, RBB delay pattern, or a near-normal ECG. If the answer to block **848** is NO, method **830** may continue to block **856,** whereas if the answer to block **848** is YES, method **830** may continue to block **850.** In block **856,** method **830** may determine whether pacing lead impedance has changed greater than or equal to about 10, 20, 30, 40, 50, or 60 % compared to a previously measured impedance of the pacing lead. A large change in pacing lead impedance may indicate that the lead has perforated into the LV. If the answer to block **856** is NO, method **830** may return to continuing rotation of the lead in block **846,** whereas if the answer is YES, method **830** may continue to block **858.**

In block **850,** method **830** may determine that an LBB pacing location has been achieved, for example, based on detecting the RBBB pattern, RBB delay pattern, or near-normal ECG. In block **852,** method **830** may determine whether pacing lead impedance has changed greater than or equal to about 10, 20, 30, 40, 50, or 60 % compared to a previously measured impedance of the pacing lead. If the answer to block **852** is NO, the impedance change may be within a normal range, and the lead may be fixed in the LBB location in block **854,** whereas if the answer is YES, method **830** may continue to block **858.**

In block **858,** method **830** may pull back or retract the lead a short distance, for example, by rotating 1-3 times in the counterclockwise direction. In block **860,** method **830** may determine whether that the pacing impedance is in a normal range. If the answer to block **860** is YES, the lead may be fixed in block **862,** whereas if the answer is NO, method **830** may return to block **858** to continue pulling back or retracting the lead a short distance.

During method **830,** the IMD may memorize the pattern of LBBB or RBBB/D (e.g., RBBB or RBB delay). Post implantation, the device may adjust pacing to the LBB or RBB, for example, if LBBB or RBBB/D is detected.

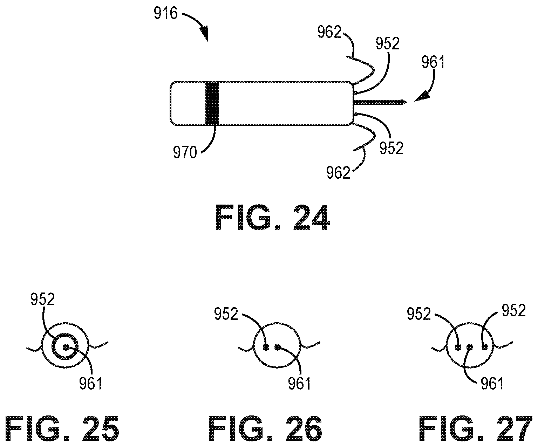

**FIG. 24** shows various aspects of one example of LPD **916** for His bundle or bundle pacing, which may be similar to LPD 616 (**FIG. 16**), except instead of a helical electrode assembly, LPD **916** may include a relatively straight, needle-like, or dart-type electrode assembly **961** for piercing tissue. In other embodiments, electrode assembly **961** may be a screw or helix-type. Electrode assembly **961** may be described as a cathode electrode for pacing on the left side of the patient's septum. LPD **916** may include electrode **952,** which may be described as a cathode electrode for pacing on the right side of the patient's septum. LPD **916** may include an electrode **970,** which may be described as an anode electrode.

LPD **916** may include fixation elements **962** that are different, separate, or independent from electrode assembly **961.** Fixation elements **962** may attach LPD **916** to cardiac tissue. Fixation elements **962** may include any suitable mechanisms, such as active fixation tines, screws, clamps, adhesive members, or any other types of attaching a device to tissue. As shown, fixation elements **962** may be constructed of a memory material that retains a preformed shape. During implantation, fixation elements **962** may be flexed forward to pierce tissue and allowed to flex back towards a case of housing of LPD **916.** In this manner, fixation elements **962** may be embedded within the target tissue.

**FIGS. 25-27** show various locations to position electrode **952.** **FIG. 25** shows electrode **952** as a ring electrode around electrode assembly **961.** **FIG. 26** shows electrode **952** as a discrete contact adjacent to electrode assembly **961.** **FIG. 27** shows electrode **952** as multiple discrete contacts adjacent to electrode assembly **961,** such as two discrete contacts on opposite sides of electrode assembly **961.** Electrode **952** may be described as leadlessly coupled to the housing. Electrode assembly **961** may also be described as being, or including, an electrode leadlessly coupled to a piercing element.

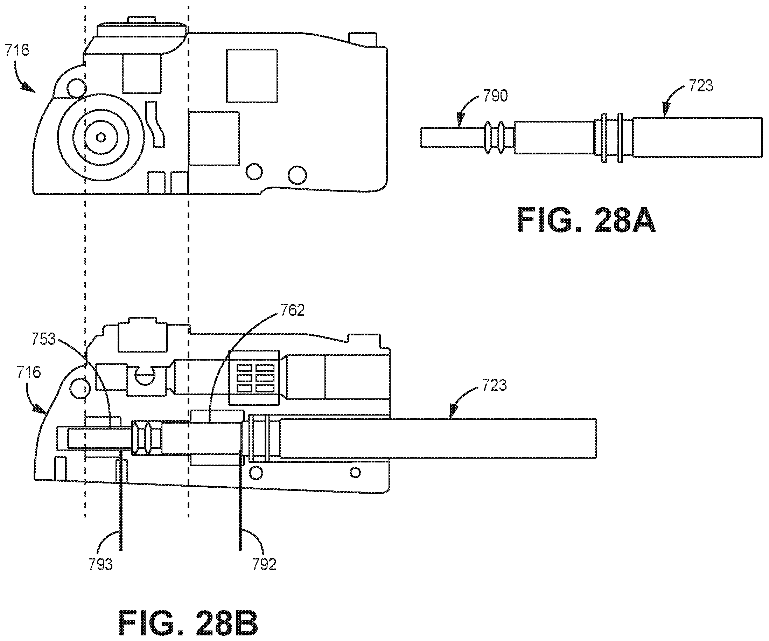

**FIGS. 28A-B** show a lead, such as implantable medical lead **723** (**FIG. 22B**), having a lead connector **790** with contacts **753, 762** operably coupled to cathode electrodes on lead **723.** IMD **716** may be configured to receive lead connector **790** in a lead connector receptacle. One example of IMD **716** includes a Medtronic ADAPTA^{™} MRI SureScan. When inserted, contacts **753, 762** may operably couple to cathode connections **793, 792** of IMD **716,** respectively, for sensing or electrical pulse delivery.

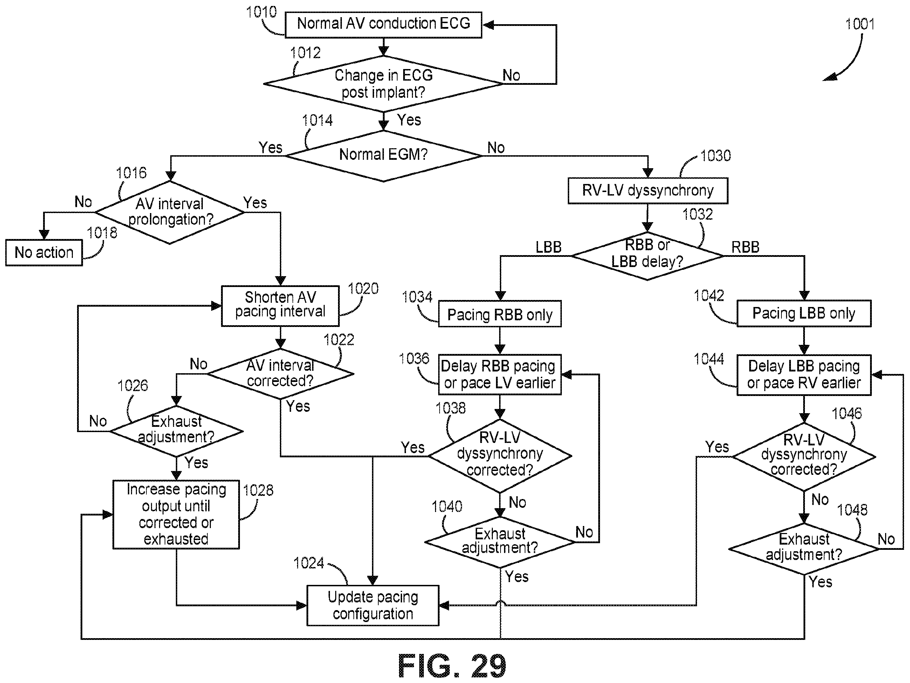

**FIGS. 29-31** show various methods for updating pacing configurations based on ECG and/or EGM information. In general, if measured AV interval prolongation is detected, the methods may shorten AV pacing intervals until measured AV interval prolongation is corrected. Output pacing amplitude may be adjusted, for example, if AV pacing intervals adjustment cannot correct the prolongation. Further, in general, if an LBBB pattern or LBB delay is detected, AV pacing intervals and/or pacing output amplitude may be adjusted in a similar manner.

In some embodiments, upon detecting dyssynchrony during a patient's intrinsic rhythm or during pacing, delivery of His-bundle or bundle-branch stimulation pulses may be adjusted based on the dyssynchrony detected. RV-LV dyssynchrony, such as an LBB or RBB delay or LBBB or RBBB pattern, may be the result of one bundle branch not being activated by bundle pacing. Increasing pacing output may reactivate both bundle branches at an appropriate AV interval. In some embodiments, if an RBB delay or RBBB pattern is detected, pacing output amplitude may be adjusted.

**FIG. 29** shows one example of method **1001** for updating a current pacing configuration when an ECG changes post implantation in block **1012** after achieving normal AV conduction in an ECG upon implantation in block **1010.** Upon detecting a change in an ECG post implantation, method **1001** may determine whether an EGM is normal in block **1014.** If the EGM is normal, method **1001** may continue to block **1016.** Otherwise, if the EGM is abnormal, method **1001** may continue to block **1030.**

In block **1016,** method **1001** may determine whether a measured AV interval is prolongated. If NO, method **1001** may continue to block **1018** to take no action to change the current pacing configuration. If YES, method **1001** may continue to block **1020** to shorten an AV pacing interval.

In block **1022,** method **1001** may determine whether a measured AV interval shows correction in response to the shortened AV pacing interval. If YES, method **1001** may continue to block **1024** to update the pacing configuration based on the corresponding shortened AV pacing interval. If NO, method **1001** may continue to block **1026.**

In block **1026,** method **1001** may determine whether adjustments to the AV pacing interval have been exhausted. If NO, method **1001** may return to block **1020** to continue shortening the AV pacing interval, for example, in an iterative manner. If YES, method **1001** may continue to block **1028** to increase a pacing output amplitude (e.g., voltage) until the measured AV interval is corrected or output pacing adjustments have been exhausted, upon which method **1001** may continue to block **1024** to update the pacing configuration after correcting or exhausting the pacing output adjustments based on, for example, an optimal AV pacing interval and/or optimal pacing output.

In block 1030, after determining that an EGM is not normal in block **1014,** method **1001** may determine whether an RBB or LBB delay is represented in the EGM. If there is an LBB delay, method **1001** may continue to block **1034,** whereas if there is an RBB delay, method **1001** may continue to block **1042.**

In block **1034,** method **1001** may determine that only the RBB is being paced by the implanted IMD. In block **1036,** pacing timing may be adjusted such that RBB pacing may be delayed (e.g., later) or LV pacing may be advanced (e.g., earlier). Method **1001** may continue to determine whether RV-LV dyssynchrony has been corrected in block **1038.** If YES, method **1001** may continue to block **1024** to update the pacing configuration. If NO, method **1001** may continue to block **1040.**

In block **1040,** method **1001** may determine whether adjustments to RBB pacing or LV pacing timing have been exhausted. If NO, method **1001** may return to block **1036** to continue adjusting pacing timing, for example, in an iterative manner. If YES, method **1001** may continue to block **1028** to increase pacing output amplitude (e.g., voltage) until RV-LV dyssynchrony is corrected or output pacing adjustments have been exhausted. Method **1001** may continue to block **1024** to update the pacing configuration based on, for example, an optimal AV pacing interval and/or optimal pacing output.

In block **1042,** method **1001** may determine that only the LBB is being paced by the implanted IMD. In block **1044,** pacing timing may be adjusted such that LBB pacing may be delayed (e.g., later) or RV pacing may be advanced (e.g., earlier). Method 1001 may continue to determine whether RV-LV dyssynchrony has been corrected in block **1046.** If YES, method may continue to block **1024** to update the pacing configuration. If NO, method **1001** may continue to block **1048.**

In block **1048,** method **1001** may determine whether adjustments to LBB pacing or RV pacing timing have been exhausted. If NO, method **1001** may return to block **1044** to continue adjusting pacing timing, for example, in an iterative manner. If YES, method **1001** may continue to block **1028** to increase pacing output amplitude (e.g., voltage) until RV-LV dyssynchrony is corrected or output pacing adjustments have been exhausted. Method **1001** may continue to block **1024** to update the pacing configuration after correcting or exhausting the pacing output adjustments based on, for example, an optimal AV pacing interval and/or optimal pacing output.

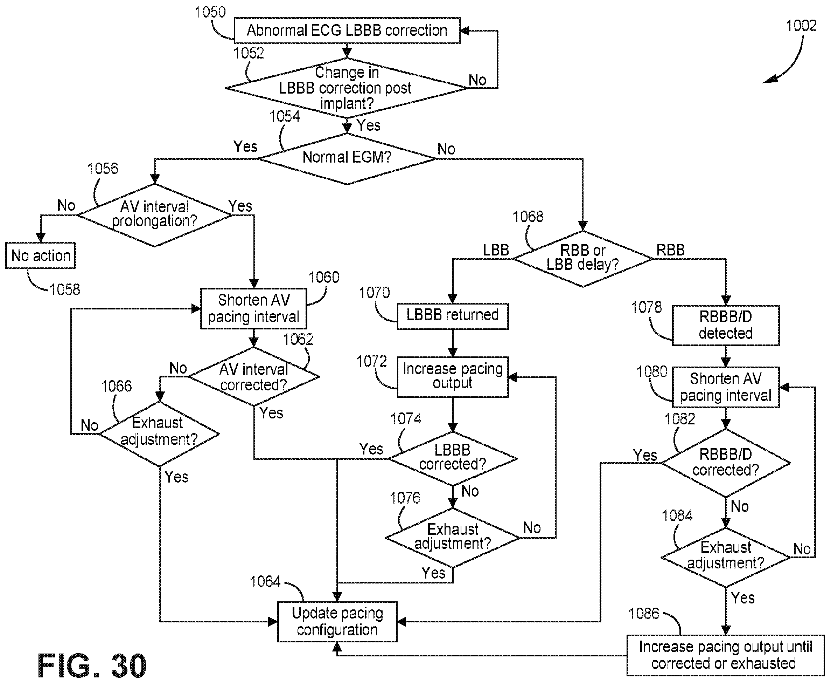

**FIG. 30** shows one example of method **1002** for updating a current pacing configuration when an ECG changes post implantation in block **1052** after achieving correction of an abnormal LBBB ECG upon implantation in block **1050.** Upon detecting a change in ECG post implantation, method **1002** may determine whether an EGM is normal in block **1054.** If the EGM is normal, method **1002** may continue to block **1056.** Otherwise, if the EGM is abnormal, method **1002** may continue to block **1068.**

In block **1056,** method **1002** may determine whether a measured AV interval is prolongated. If NO, method **1002** may continue to block **1058** to take no action to change the current pacing configuration. If YES, method **1002** may continue to block **1060** to shorten an AV pacing interval.

In block **1062,** method **1002** may determine whether a measured AV interval shows correction in response to the shortened AV pacing interval. If YES, method **1002** may continue to block **1064** to update the pacing configuration based on the corresponding shortened AV pacing interval. If NO, method **1002** may continue to block **1066.**

In block **1066,** method **1002** may determine whether adjustments to the AV pacing interval have been exhausted. If NO, method **1002** may return to block **1060** to continue shortening the AV pacing interval, for example, in an iterative manner. If YES, method **1002** may continue to update pacing configuration in block **1064** based on, for example, an optimal AV pacing interval and/or optimal pacing output.

In block **1068,** after determining that an EGM is not normal in block **1054,** method **1002** may determine whether an RBB or LBB delay is represented in the EGM. If there is an LBB delay, method **1002** may continue to block **1070,** whereas if there is an RBB delay, method **1002** may continue to block **1078.**

In block **1070,** method **1002** may determine that LBBB has returned. In block **1072,** pacing output amplitude (e.g., voltage) may be increased. In block **1074,** method **1002** may determine whether LBBB has been corrected in response to the increased pacing output. If YES, method **1002** may continue to block **1064.** If NO, method **1002** may continue to block **1076.**

In block **1076,** method **1002** may determine whether pacing output adjustments have been exhausted. If YES, method **1002** may continue to block **1064** to update the pacing configuration based on, for example, an optimal AV pacing interval and/or optimal pacing output. If NO, method **1002** may return to block **1072** to continue increasing pacing output, for example, in an iterative manner.

In block **1078,** after determining that an RBB delay is represented in the EGM in block **1068,** method **1002** may determine that an RBBB pattern or RBB delay has been detected. In block **1080,** method **1002** may shorten an AV pacing interval. Method **1002** may determine whether the RBBB pattern or RBB delay has been corrected in response to shortening the AV pacing interval in block **1082.** If YES, method **1002** may continue to block **1064** to update the pacing configuration based on, for example, the optimal AV pacing interval and/or optimal pacing output. If NO, method **1002** may continue to block **1084.**

In block **1084,** method **1002** may determine whether AV pacing interval adjustments have been exhausted. If NO, method **1002** may return to block **1080** to continue shortening the AV pacing interval, for example, in an iterative manner. If YES, method **1002** may continue to block **1086** to increase pacing output until the RBBB pattern or RBB has been corrected or output pacing adjustments have been exhausted. Method **1002** may continue to block **1064** to update the pacing configuration based on, for example, an optimal AV pacing interval and/or optimal pacing output.

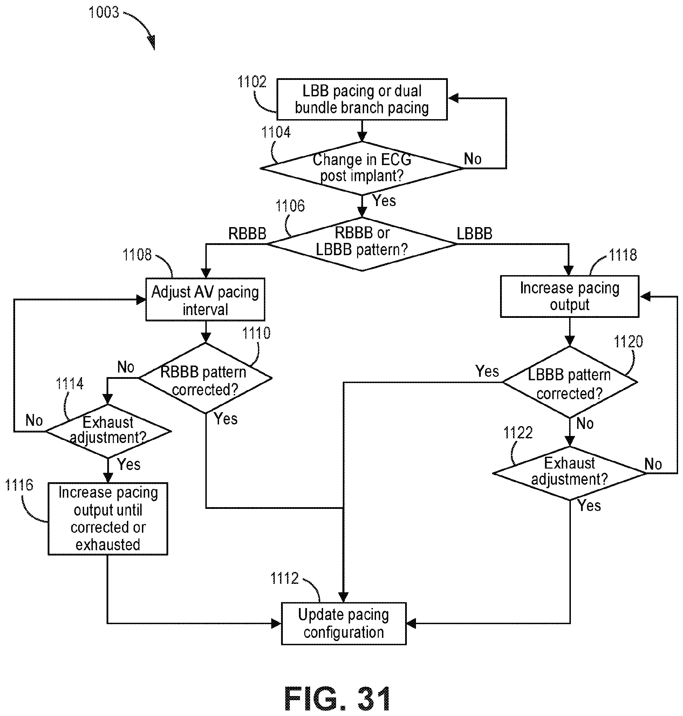

**FIG. 31** shows one example of method **1003** for updating a current pacing configuration when an ECG changes in block **1104** after implanting an IMD for LBB or dual bundle branch pacing in **1102.** Method **1003** may include determining whether an RBBB or LBBB pattern is represented in an EGM. If an RBBB pattern is represented, method **1003** may continue to block **1108.** If an LBBB pattern is represented, method **1003** may continue to block **1118.**

In block **1108,** method **1003** may adjust an AV pacing interval. In block **1110,** method **1003** may determine whether the RBBB pattern has been corrected in response to adjusting the AV pacing interval. If YES, method **1003** may continue to block **1112** to update the pacing configuration based on, for example, an optimal AV pacing interval and/or optimal pacing output. If NO, method **1003** may continue to block **1114.**

In block **1114,** method **1003** may determine whether AV pacing interval adjustments have been exhausted. If NO, method **1003** may return to block **1108** to adjust AV pacing interval, for example, in an iterative manner. If YES, method **1003** may continue to block **1116** to increase pacing output until the RBBB pattern is corrected or AV pacing interval adjustments have been exhausted. Method **1003** may continue to block **112** to update the current pacing configuration based on, for example, an optimal AV pacing interval and/or an optimal pacing output.

In block **1118,** after determining that an LBBB pattern is represented in block **1106,** method **1003** may increase pacing output amplitude (e.g., voltage). Method **1003** may determine whether the LBBB pattern has been corrected in block **1120.** If YES, method **1003** may continue to block **1112.** If NO, method may continue to block **1122.**

In block **1122,** method **1003** may determine whether pacing output adjustments have been exhausted. If NO, method **1003** may return to block **1118** to continue increasing pacing output, for example, in an iterative manner. If YES, method **1003** may continue to block **1112** to update the pacing configuration based on, for example, an optimal AV pacing interval and/or an optimal pacing output.

Various examples have been described. These and other examples are within the scope of the following claims. For example, a single chamber, dual chamber, or triple chamber pacemakers (e.g., CRT-P) or ICDs (e.g., CRT-D) devices can be used to implement the illustrative methods described herein.

### ILLUSTRATIVE EMBODIMENTS

While the present disclosure is not so limited, an appreciation of various aspects of the disclosure will be gained through a discussion of the specific illustrative embodiments provided below, which provide alloys with superior mechanical and corrosion properties. Various modifications of the illustrative embodiments, as well as additional embodiments of the disclosure, will become apparent herein.

Embodiment A1 is a method of using a pacemaker for His-bundle or bundle-branch pacing of a patient's heart comprising sensing heart activity using a set of electrodes associated with the pacemaker; using a processor in the pacemaker to determine whether one of a QRS parameter and an activation interval acquired from the sensed heart activity is greater than a first threshold; in response to determining whether the QRS parameter or activation interval is greater than the first threshold, using the processor to determine whether bundle pacing should be delivered earlier or later; and in response to determining whether bundle pacing should be delivered earlier or later, using the processor to adjust a time interval for bundle pacing.

Embodiment A2 is a method of any A embodiment, wherein using the processor to determine whether bundle pacing should be delivered earlier or later comprises determining whether a bundle-pacing to ventricular-activation interval reaches a second threshold.

Embodiment A3 is a method of any A embodiment, wherein using the processor to adjust the time interval for bundle pacing comprises adjusting an atrial-activation to bundle-pacing interval or adjusting a bundle-pacing to left- or right-ventricular-activation interval.

Embodiment A4 is a method of any A embodiment, wherein the sensed heart activity comprises one or more of: an intraventricular interval, a QRS duration, a QRS morphology, a QRS vector, an R-wave timing, a bundle-pacing to left- or right-ventricular-activation interval, and an atrial-sensing to left- or right-ventricular-activation interval.

Embodiment A5 is a method of any A embodiment, wherein the processor is contained in a housing of the pacemaker.

Embodiment A6 is a method of any A embodiment, further comprising: using the processor to determine whether another QRS parameter acquired from the sensed heart activity is greater than the first threshold; and in response to determining that the another QRS parameter is greater than or equal to the first threshold, using the processor to determine whether the another QRS parameter is greater than the QRS parameter.

Embodiment A7 is a method of embodiment A6, further comprising: in response to determining that the another QRS parameter is greater than the QRS parameter, using the processor to increase a bundle pacing output level.

Embodiment A8 is a method of embodiment A7, further comprising: using the processor to determine whether yet another QRS parameter acquired from the sensed heart activity is greater than the first threshold; and in response to determining that the yet another QRS parameter is greater than the another QRS parameter, using the processor to select an optimal time interval or an optimal pacing output level for bundle pacing corresponding to a minimal QRS parameter.

Embodiment A9 is a method of any A embodiment, wherein the first threshold is equal to or less than 130 ms.

Embodiment A10 is a method of any A embodiment, wherein the time interval for bundle pacing is adjusted by increasing up to 10 ms or decreasing down to 10 ms.

Embodiment A11 is a method of using an implantable medical device having a processor and an electrical pulse generator comprising: using the processor to control the electrical pulse generator to adjust delivery of bundle pacing. The processor is configured to: measure a QRS parameter and determine whether the QRS parameter is larger than a threshold; in response to determining that the QRS parameter is greater than or equal to the threshold, adjust a bundle-pacing to ventricular-activation interval or a bundle pacing output level; and deliver bundle pacing using the adjusted bundle-pacing to ventricular-activation interval or using the adjusted bundle pacing output level.

Embodiment A12 is an implantable medical device for His-bundle or bundle-branch pacing of a patient's heart comprising: a sensing circuit configured to sense an atrial event (As) and a ventricular event (Vs); an electrical pulse generator configured to generate and deliver electrical His-bundle or bundle-branch stimulation pulses (Vp); a processor coupled to the sensing circuit and the electrical pulse generator and configured to control the electrical pulse generator to deliver said Vp on expiration of a defined AV interval or Vp-Vs interval following a said As or Vs. The processor is further configured to: determine whether Vp timing should be adjusted; in response to determining that the Vp timing should be adjusted, determine whether Vp should be delivered earlier or later; in response to determining whether the Vp should be delivered earlier or later, decrease an AV delay if the Vp should be delivered earlier or increase the AV delay in response to determining Vp should be delivered later; generate a set of AV delays that are stored in memory based on iterating through decreases or increases in the AV delay; select an optimal AV delay from the set of AV delays stored in memory; and use the optimal AV delay when delivering His-bundle or bundle-branch pacing.

Embodiment A13 is a device of any A embodiment, wherein the processor is further configured to: in response to determining that the Vp timing should be adjusted, advance or delay Vp in response to whether Vp should be delivered earlier or later to adjust Vp timing.

Embodiment A14 is a device of any A embodiment, wherein the processor is further configured to determine whether Vp timing should be adjusted based on dyssynchrony.

Embodiment A15 is a device of embodiment A14, wherein the processor is further configured to detect dyssynchrony using one or both of: a comparison of a Vp to RV-sensing interval with a Vp to LV-sensing interval, and a QRS morphology change.

Embodiment A16 is a device of embodiment A15, wherein to detect dyssynchrony using a QRS morphology change comprises comparing a current QRS morphology to a predetermined QRS morphology representative of an RBBB or LBBB pattern.

Embodiment A17 is a device of any of embodiments A14 to A16, wherein the processor is further configured to: in response to determining that the Vp timing should be adjusted using dyssynchrony, determining that the Vp output should be adjusted.

Embodiment A18 is a device of any A embodiment, wherein to determine whether Vp timing should be adjusted is based on one or more criteria comprising one or both of a QRS parameter and an activation interval.

Embodiment A19 is a device of embodiment A18, wherein the criterion comprises one or more of: a QRS duration, a QRS morphology change, a QRS vector change, an RBBB pattern, an LBBB pattern, and a bundle-pacing to RV- or LV-activation interval.

Embodiment A20 is a device of embodiment A18 or A19, wherein to determine whether the Vp timing should be adjusted is based on a QRS parameter being greater than a threshold.

Embodiment A21 is a device of any A embodiment, wherein the implantable medical device comprises one or more of: a pacemaker with a medical lead, an implantable cardioverter-defibrillator (ICD), an intracardiac device, a leadless pacing device (LPD), a subcutaneous ICD, and a subcutaneous medical device.

Embodiment A22 is a device of any A embodiment, wherein the implantable medical device comprises a triple chamber device, a dual chamber device, or a single chamber device.

Embodiment A23 is a device of embodiment A22, wherein the single chamber device only employs a bundle pacing lead.

Embodiment A24 is a device of embodiment A22 or A23, wherein the dual chamber device requires at least a bundle pacing lead disposed in the His bundle or bundle branch region and another lead in one of the right ventricle, left ventricle, or right atrium.

Embodiment A25 is a device of any of embodiments A22 to A24, further comprising: a housing for containing the sensing circuit, the electrical pulse generator, and the processor; and a connector interface coupled to the housing, the connector interface comprising a set of ports for receiving a set of leads. The set of ports includes: a right atrial port for receiving a right atrial lead, and a left ventricular port for receiving a left ventricular lead, wherein the bundle pacing lead is configured to be coupled to the right ventricular port.

Embodiment A26 is a device of embodiment A25, wherein when the patient is experiencing atrial fibrillation, the bundle pacing lead is inserted through the device right atrial port, the right ventricular lead is inserted through the device right ventricular port, and the left ventricular lead is inserted through the device left ventricular port.

Embodiment A27 is a device of embodiment A25 or A26, wherein when a patient is not experiencing atrial fibrillation, the bundle pacing lead is inserted through either the right or left ventricular port of the device, and the right or left ventricular lead is inserted through the other of the right or left ventricular port of the device.

Embodiment A28 is a device of any of embodiments A25 to A27, wherein the right atrial port is configured to receive a Y-adaptor, the Y-adaptor configured to receive connections to the tip and ring electrodes of the bundle pacing lead.

Embodiment A29 is a device of embodiment A28, wherein the right ventricular lead is inserted through the right ventricular port of the device and the left ventricular lead is inserted through the left ventricular port of the device.

Embodiment A30 is a device of any of embodiments A25 to A29, further comprising at least two electrodes on the housing.

Embodiment A31 is a device of embodiment A30, wherein the implantable medical device comprises an intracardiac device.

Embodiment A32 is an implantable medical device for His-bundle or bundle-branch pacing of a patient's heart comprising: a sensing circuit configured to sense an atrial event (As) and a ventricular event (Vs); an electrical pulse generator configured to generate and deliver electrical His-bundle or bundle-branch stimulation pulses (Vp); a processor coupled to the sensing circuit and the electrical pulse generator and configured to control the electrical pulse generator to deliver said Vp on expiration of a defined AV interval or Vp-Vs interval following a said As or Vs. The processor is further configured to: determine whether RV activation occurs earlier than LV activation; and in response to determining RV activation occurs earlier than LV activation, adjust Vp timing to deliver His-bundle or bundle-branch pacing based on an LV activation time.

Embodiment A33 is an implantable medical device for His-bundle or bundle-branch pacing of a patient's heart comprising: a sensing circuit configured to sense an atrial event (As) and a ventricular event (Vs); an electrical pulse generator configured to generate and deliver electrical His-bundle or bundle-branch stimulation pulses (Vp); and a processor coupled to the sensing circuit and the electrical pulse generator and configured to control the electrical pulse generator to deliver said Vp on expiration of a defined AV interval or Vp-Vs interval following a said As or Vs. The processor is further configured to: determine whether RV activation occurs earlier than LV activation; in response to determining that RV activation does not occur earlier than LV activation, determine whether RV activation occurs later than LV activation; and in response to determining RV activation occurs later than LV activation, adjust Vp timing to deliver His-bundle or bundle-branch pacing based on an RV activation time.

Embodiment A34 is a method of using a pacemaker for His-bundle or bundle-branch pacing of a patient's heart comprising: sensing electrical activity of the patient's heart including an atrial event (As) and a ventricular event (Vs); detecting dyssynchrony based on the sensed electrical activity of the patient's heart during the patient's intrinsic rhythm or during pacing; and adjusting delivery of His-bundle or bundle-branch stimulation pulses (Vp) based on the dyssynchrony detected.

Embodiment A35 is a method of any A embodiment, further comprising: in response to detecting dyssynchrony in the sensed electrical activity, determining whether the sensed electrical activity represents a right bundle branch (RBB) delay, and in response to determining that the sensed electrical activity represents an RBB delay, delivering Vp to the left bundle branch (LBB) of the patient's heart earlier or deliver right ventricular (RV) pacing earlier.

Embodiment A36 is a method of any A embodiment, further comprising: in response to detecting dyssynchrony in the sensed electrical activity, determining whether the sensed electrical activity represents a right bundle branch (RBB) delay; and in response to determining that the sensed electrical activity does not represent an RBB delay, delivering Vp to the right bundle branch (RBB) of the patient's heart earlier or delivering left ventricular (LV) pacing earlier.

Embodiment A37 is a method of any A embodiment, further comprising: in response to detecting dyssynchrony in the sensed electrical activity, determining whether the sensed electrical activity represents a right bundle branch (RBB) delay; and in response to determining that the sensed electrical activity represents an RBB delay, adjusting an As-Vp interval.

Embodiment A38 is a method of any of embodiments A34 to A37, further comprising: in response to detecting dyssynchrony in the sensed electrical activity, determining whether the sensed electrical activity represents a right bundle branch (RBB) delay; and in response to determining that the sensed electrical activity does not represent an RBB delay, increasing Vp output.

Embodiment A39 is a method of using a pacemaker for His-bundle or bundle-branch pacing of a patient's heart comprising sensing electrical activity of the patient's heart including an atrial event (As) and a ventricular event (Vs); detecting a right bundle branch block (RBBB) pattern or left bundle branch block (LBBB) pattern in the sensed electrical activity of the patient's heart during the patient's intrinsic rhythm or during pacing; and adjusting the delivery of His-bundle or bundle-branch stimulation pulses (Vp) based on the detected bundle-branch-block pattern.

Embodiment A40 is a method of any A embodiment, further comprising in response to detecting an RBBB pattern in the sensed electrical activity, adjusting an As-Vp interval.

Embodiment A41 is a method of any A embodiment, further comprising in response to detecting an LBBB pattern in the sensed electrical activity, increasing Vp output.

Embodiment B1 is an implantable medical device for bundle-branch pacing of a patient's heart comprising a plurality of electrodes. The plurality of electrodes a left bundle branch (LBB) cathode electrode positionable, when implanted, on a left side of the septum of the patient's heart proximate to the LBB, and a right bundle branch (RBB) cathode electrode positionable, when implanted, implantable on a right side of the septum of the patient's heart proximate to the RBB. The device includes an electrical pulse generating circuit coupled to the plurality of electrodes to generate and deliver electrical bundle-branch stimulation pulses (Vp); and a processor coupled to the electrical pulse generating circuit, the processor configured to control the electrical pulse generating circuit to deliver synchronized left and right bundle-branch Vp using the plurality of electrodes based on one or both of an atrial event (As) and a ventricular event (Vs).

Embodiment B2 is a device of any B embodiment, further comprising a sensing circuit operably coupled to the processor and the plurality of electrodes to sense electrical activity of the patient's heart including As or Vs using at least one of the plurality of electrodes.

Embodiment B3 is a device of any B embodiment, wherein the LBB cathode electrode does not pierce or extend into the left ventricle.

Embodiment B4 is a device of any B embodiment, wherein the synchronized left and right bundle-branch Vp are delivered simultaneously.

Embodiment B5 is a device of any B embodiment, wherein the synchronized left and right bundle-branch Vp are delivered sequentially or with a delay between the left bundle-branch Vp and the right bundle-branch Vp.

Embodiment B6 is a device of any B embodiment, further comprising a medical electrical lead to deliver Vp, the medical electrical lead comprising a lead body, the plurality of electrodes disposed on the lead body to deliver pacing pulses within a single cardiac cycle.

Embodiment B7 is a device of embodiment B6, wherein the lead body comprises a shaft extending longitudinally and a stopper protruding radially outward from the shaft configured to prevent the left bundle-branch electrode from perforating into the left ventricle.

Embodiment B8 is a device of embodiment B6 or B7, wherein the plurality of electrodes comprises an atrial electrode coupled to the lead body and implantable in the patient's heart to sense As.

Embodiment B9 is a device of embodiment B8, wherein the atrial electrode is spaced at least 5 cm from one of the bundle-branch cathode electrodes.

Embodiment B10 is a device of any B embodiment, wherein the implantable medical device is a leadless pacing device (LPD) comprising a housing and at least one of the plurality of electrodes is leadlessly connected to the housing.

Embodiment B11 is a device of embodiment B10, wherein the at least one electrode leadlessly connected to the housing is in the shape of a ring electrode.

Embodiment B12 is a device of any B embodiment, wherein the implantable medical device is a leadless pacing device (LPD) comprising a housing and at least one of the plurality of electrodes is leadlessly connected to a piercing element extending axially from the housing.

Embodiment B13 is a device of embodiment B12, further comprising a fixation element different than the piercing element extending axially from the housing.

Embodiment B14 is a method of using a pacemaker for bundle-branch pacing of a patient's heart comprising: sensing electrical activity of the patient's heart including an atrial event (As) and a ventricular event (Vs); and delivering synchronized left and right bundle-branch stimulation pulses (Vp) using left and right bundle-branch cathode electrodes based on one or both of As and Vs, wherein the left bundle branch (LBB) cathode electrode is implantable on a left side of the septum of the patient's heart proximate to the LBB of the patient's heart, wherein the right bundle branch (RBB) cathode electrode is implantable on a right side of the septum of the patient's heart proximate to the RBB.

Embodiment B15 is a method of positioning a bundle pacing electrode in a patient's heart comprising: monitoring electrical activity of the patient's heart using one or both of an external electrode and a bundle pacing electrode on a bundle pacing lead or leadless device; rotating or advancing the bundle pacing lead or leadless device toward the His bundle or bundle branch of the patient's heart while intermittently applying pacing with the bundle pacing electrode; determining whether an RBBB pattern is present based on the monitored electrical activity; determining whether the patient's heart has a right bundle branch block (RBBB); and in response to determining that an RBBB pattern is present during applied pacing and that the patient's heart does not have a RBBB, fixing the bundle pacing lead or leadless device for left bundle branch pacing.

Embodiment B16 is a method of any B embodiment, further comprising in response to determining that an LBBB pattern is present and the patient's heart has a RBBB, continuing to rotate or advance the bundle pacing lead or leadless device toward the left bundle branch of the patient's heart.

Embodiment B17 is a method of embodiment B16, further comprising: in response to continuing to rotate or advance the bundle pacing lead or leadless device, determining whether the monitored electrical activity represents an RBBB pattern, RBB delay, or near-normal electrocardiogram (ECG); and in response to determining that an RBBB pattern, RBB delay, or near-normal ECG is present, fixing the bundle pacing lead or leadless device.

Embodiment B18 is a method of embodiment B17, further comprising: determining whether an impedance change of the bundle pacing electrode falls below a threshold; in response to determining that the impedance change of the bundle pacing electrode does not fall below a threshold, continuing to rotate or advance the bundle pacing lead or leadless device or fixing the bundle pacing lead or leadless device; and in response to determining that the impedance change of the bundle pacing electrode does fall below the threshold, reversing rotation or advancement of the bundle pacing lead or leadless device.

Embodiment B19 is a method of embodiment B18, wherein the threshold is less than 50% of a previously measured impedance of the bundle pacing electrode.

Embodiment B20 is a method of embodiment B18 or B19, further comprising fixing the bundle pacing lead or leadless device in response to an impedance of the bundle pacing electrode being within a normal range.

Embodiment C1 is a leadless pacing device (LPD) for His-bundle or bundle-branch pacing in a patient's heart comprising: an intracardiac housing; a plurality of electrodes comprising a bundle pacing electrode leadlessly connected to the housing and implantable proximate to or in the His bundle or bundle branch of the patient's heart; a sensing circuit operably coupled to the plurality of electrodes and configured to sense one or both of an atrial event (As) and a ventricular event (Vs) using at least one of the plurality of electrodes; and an electrical pulse generator coupled to the bundle pacing electrode, the electrical pulse generator configured to generate and deliver electrical His-bundle or bundle-branch stimulation pulses (Vp) based on one or both of As and Vs to the patient's heart using the bundle pacing electrode.

Embodiment C2 is a device of any C embodiment, further comprising a processor coupled to the sensing circuit and the electrical pulse generator, the processor configured to adjust a time interval or a pacing output level for His-bundle or bundle-branch pacing based on one or both of As and Vs sensed by the sensing circuit.

Embodiment C3 is a device of any C embodiment, wherein when the plurality of electrodes is positioned proximate to the His bundle or bundle branch, wherein the plurality of electrodes is configured to not pierce or extend into the left ventricle.

Embodiment C4 is a device of any C embodiment, wherein the plurality of electrodes comprises an atrial cathode electrode leadlessly connected to the housing and implantable in the patient's heart to sense As.

Embodiment C5 is a device of any C embodiment, wherein the bundle pacing electrode is leadlessly connected to a piercing element extending axially from the housing.

Embodiment C6 is a device of embodiment C5, wherein the piercing element is also configured as a fixation element.

Embodiment C7 is a device of embodiment C5 or C6, further comprising a fixation element different than the piercing element extending axially from the housing.

Embodiment C8 is a leadless pacing device (LPD) for His-bundle or bundle-branch pacing in a patient's heart comprising: an intracardiac housing; a communication interface disposed in the housing configured to receive signals from a subcutaneously implanted device (SD); and a controller disposed in the housing operatively coupled to at least one electrode configured to deliver His-bundle or bundle-branch pacing pulses to the patient's heart in response to the received signals.

Embodiment C9 is an LPD of any C embodiment, wherein the controller is further configured to deliver His-bundle or bundle-branch pacing pulses adapted to achieve synchrony of the patient's heart in a manner manifested in a plurality of far-field electrical signals as: a narrowed QRS duration, or a correction of left branch bundle block (LBBB) or right branch bundle block (RBBB) patterns in QRS morphology.

Embodiment C10 is an LPD of any C embodiment, wherein the controller is further configured to adapt His-bundle or bundle-branch pacing pulse timing or pacing output level based on a detected A-V dyssynchrony.

Embodiment C11 is an LPD of any C embodiment, wherein the controller is further configured to adapt His-bundle or bundle-branch pacing pulse timing or pacing output level based on a detected V-V dyssynchrony.

Embodiment C12 is an LPD of any C embodiment, wherein the controller is further configured to adapt His-bundle or bundle-branch pacing pulse timing or pacing output level based on dyssynchrony detected by the SD.

Embodiment C13 is an LPD of any C embodiment, further comprising the SD, wherein the SD is configured to deliver electrical pulses to cardiac tissue, the electrical pulses being related to a different therapy than therapy provided by the LPD.

Embodiment C14 is an LPD of embodiment C13, wherein the different therapy comprises cardiac resynchronization therapy.

Embodiment C15 is an LPD of any C embodiment, wherein a subcutaneous electrode associated with the SD is employed to sense electrical signals from the patient's heart to determine the timings of atrial and ventricular activations.

Embodiment C16 is an LPD of any C embodiment, wherein the at least one electrode of the LPD is positioned within 10 mm of the His bundle or left bundle branch of the patient's heart.

Embodiment C17 is an LPD of any C embodiment, wherein the LPD comprises an elongated helical tip to extend proximate to the His bundle or bundle branch.

Embodiment C18 is an LPD of any C embodiment, wherein the LPD comprises an elongated needle to extend near the His bundle or bundle branch.

Embodiment C19 is an LPD of any C embodiment, wherein the LPD comprises a fixation element to attach the LPD to the patient's heart.

Embodiment C20 is an LPD of any C embodiment, wherein the LPD comprises a medical electrical lead, the lead comprising a stopper to prevent the at least one electrode of the LPD from perforating into the left ventricle of the patient's heart.

Embodiment C21 is a method of His-bundle or bundle-branch pacing comprising: using a subcutaneously implanted device (SD) to sense far-field electrical signals; determining a QRS parameter or activation based on the far-field electrical signals; and communicating a timing to a leadless pacing device (LPD) to deliver His-bundle or bundle-branch pacing using an atrial-activation to bundle-pacing interval.

Thus, various embodiments of HIS BUNDLE AND BUNDLE BRANCH PACING ADJUSTMENT are disclosed. It should be understood that various aspects disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e.g., all described acts or events may not be necessary to carry out the techniques). In addition, while certain aspects of this disclosure are described as being performed by a single module or unit for purposes of clarity, it should be understood that the techniques of this disclosure may be performed by a combination of units or modules associated with, for example, a medical device.

In one or more examples, the described techniques may be implemented in hardware, software, firmware, or any combination thereof. If implemented in software, the functions may be stored as one or more instructions or code on a computer-readable medium and executed by a hardware-based processing unit. Computer-readable media may include non-transitory computer-readable media, which corresponds to a tangible medium such as data storage media (e.g., RAM, ROM, EEPROM, flash memory, or any other medium that can be used to store desired program code in the form of instructions or data structures and that can be accessed by a computer).

Instructions may be executed by one or more processors, such as one or more digital signal processors (DSPs), general purpose microprocessors, application specific integrated circuits (ASICs), field programmable logic arrays (FPGAs), or other equivalent integrated or discrete logic circuitry. Accordingly, the term "processor" as used herein may refer to any of the foregoing structure or any other physical structure suitable for implementation of the described techniques. Also, the techniques could be fully implemented in one or more circuits or logic elements.

All references and publications cited herein are expressly incorporated herein by reference in their entirety for all purposes, except to the extent any aspect directly contradicts this disclosure.

All scientific and technical terms used herein have meanings commonly used in the art unless otherwise specified. The definitions provided herein are to facilitate understanding of certain terms used frequently herein and are not meant to limit the scope of the present disclosure.

Unless otherwise indicated, all numbers expressing feature sizes, amounts, and physical properties used in the specification and claims may be understood as being modified either by the term "exactly" or "about." Accordingly, unless indicated to the contrary, the numerical parameters set forth in the foregoing specification and attached claims are approximations that can vary depending upon the desired properties sought to be obtained by those skilled in the art utilizing the teachings disclosed herein or, for example, within typical ranges of experimental error.

The recitation of numerical ranges by endpoints includes all numbers subsumed within that range (e.g. 1 to 5 includes 1, 1.5, 2, 2.75, 3, 3.80, 4, and 5) and any range within that range. Herein, the terms "up to" or "no greater than" a number (e.g., up to 50) includes the number (e.g., 50), and the term "no less than" a number (e.g., no less than 5) includes the number (e.g., 5).

The terms "coupled" or "connected" refer to elements being attached to each other either directly (in direct contact with each other) or indirectly (having one or more elements between and attaching the two elements). Either term may be modified by "operatively" and "operably," which may be used interchangeably, to describe that the coupling or connection is configured to allow the components to interact to carry out at least some functionality (for example, a mobile user device may be operatively coupled to a cellular network transmit data to or receive data therefrom).

Terms related to orientation, such as "top," "bottom," "side," and "end," are used to describe relative positions of components and are not meant to limit the orientation of the embodiments contemplated. For example, an embodiment described as having a "top" and "bottom" also encompasses embodiments thereof rotated in various directions unless the content clearly dictates otherwise.

Reference to "one embodiment," "an embodiment," "certain embodiments," or "some embodiments," etc., means that a particular feature, configuration, composition, or characteristic described in connection with the embodiment is included in at least one embodiment of the disclosure. Thus, the appearances of such phrases in various places throughout are not necessarily referring to the same embodiment of the disclosure. Furthermore, the particular features, configurations, compositions, or characteristics may be combined in any suitable manner in one or more embodiments.

As used in this specification and the appended claims, the singular forms "a," "an," and "the" encompass embodiments having plural referents, unless the content clearly dictates otherwise. As used in this specification and the appended claims, the term "or" is generally employed in its sense including "and/or" unless the content clearly dictates otherwise.

As used herein, "have," "having," "include," "including," "comprise," "comprising" or the like are used in their open-ended sense, and generally mean "including, but not limited to." It will be understood that "consisting essentially of," "consisting of," and the like are subsumed in "comprising," and the like.

The term "and/or" means one or all of the listed elements or a combination of at least two of the listed elements.

The phrases "at least one of," "comprises at least one of," and "one or more of" followed by a list refers to any one of the items in the list and any combination of two or more items in the list.
Further disclosed herein is the subject-matter of the following clauses:
1. A method of using a pacemaker for His-bundle or bundle-branch pacing of a patient's heart, the method comprising:
   sensing heart activity using a set of electrodes associated with the pacemaker;
   using a processor in the pacemaker to determine whether one of a QRS parameter and an activation interval acquired from the sensed heart activity is greater than a first threshold;
   in response to determining whether the QRS parameter or activation interval is greater than the first threshold, using the processor to determine whether bundle pacing should be delivered earlier or later; and
   in response to determining whether bundle pacing should be delivered earlier or later, using the processor to adjust a time interval for bundle pacing.
2. The method of clause 1, wherein using the processor to determine whether bundle pacing should be delivered earlier or later comprises determining whether a bundle-pacing to ventricular-activation interval reaches a second threshold.
3. The method of clause 1 or 2, wherein using the processor to adjust the time interval for bundle pacing comprises adjusting an atrial-activation to bundle-pacing interval or adjusting a bundle-pacing to left- or right-ventricular-activation interval.
4. The method of any preceding clause , wherein the sensed heart activity comprises one or more of: an intraventricular interval, a QRS duration, a QRS morphology, a QRS vector, an R-wave timing, a bundle-pacing to left- or right-ventricular-activation interval, and an atrial-sensing to left- or right-ventricular-activation interval.
5. The method of any preceding clause , wherein the processor is contained in a housing of the pacemaker.
6. The method of any preceding clause , further comprising:
   using the processor to determine whether another QRS parameter acquired from the sensed heart activity is greater than the first threshold; and
   in response to determining that the another QRS parameter is greater than or equal to the first threshold, using the processor to determine whether the another QRS parameter is greater than the QRS parameter.
7. The method of clause 6, further comprising:
   in response to determining that the another QRS parameter is greater than the QRS parameter, using the processor to increase a bundle pacing output level.
8. The method of clause 7, further comprising:
   using the processor to determine whether yet another QRS parameter acquired from the sensed heart activity is greater than the first threshold; and
   in response to determining that the yet another QRS parameter is greater than the another QRS parameter, using the processor to select an optimal time interval or an optimal pacing output level for bundle pacing corresponding to a minimal QRS parameter.
9. The method of any preceding clause , wherein the first threshold is equal to or less than 130 ms.
10. The method of any preceding clause , wherein the time interval for bundle pacing is adjusted by increasing up to 10 ms or decreasing down to 10 ms.
11. A method of using an implantable medical device having a processor and an electrical pulse generator, the method comprising:
   using the processor to control the electrical pulse generator to adjust delivery of bundle pacing, the processor configured to:
   measure a QRS parameter and determine whether the QRS parameter is larger than a threshold;
   in response to determining that the QRS parameter is greater than or equal to the threshold, adjust a bundle-pacing to ventricular-activation interval or a bundle pacing output level; and
   deliver bundle pacing using the adjusted bundle-pacing to ventricular-activation interval or using the adjusted bundle pacing output level.
12. A method of using a pacemaker for His-bundle or bundle-branch pacing of a patient's heart, the method comprising:
   sensing electrical activity of the patient's heart including an atrial event (As) and a ventricular event (Vs);
   detecting dyssynchrony based on the sensed electrical activity of the patient's heart during the patient's intrinsic rhythm or during pacing; and
   adjusting delivery of His-bundle or bundle-branch stimulation pulses (Vp) based on the dyssynchrony detected.
13. The method of clause 12, further comprising:
   in response to detecting dyssynchrony in the sensed electrical activity, determining whether the sensed electrical activity represents a right bundle branch (RBB) delay, and
   in response to determining that the sensed electrical activity represents an RBB delay, delivering Vp to the left bundle branch (LBB) of the patient's heart earlier or deliver right ventricular (RV) pacing earlier.
14. The method of clause 12 or 13, further comprising:
   in response to detecting dyssynchrony in the sensed electrical activity, determining whether the sensed electrical activity represents a right bundle branch (RBB) delay; and
   in response to determining that the sensed electrical activity does not represent an RBB delay, delivering Vp to the right bundle branch (RBB) of the patient's heart earlier or delivering left ventricular (LV) pacing earlier.
15. The method of any of clause 12 to 14, further comprising:
   in response to detecting dyssynchrony in the sensed electrical activity, determining whether the sensed electrical activity represents a right bundle branch (RBB) delay; and
   in response to determining that the sensed electrical activity represents an RBB delay, adjusting an As-Vp interval.
16. The method of claim any of clause 12 to 15, further comprising:
   in response to detecting dyssynchrony in the sensed electrical activity, determining whether the sensed electrical activity represents a right bundle branch (RBB) delay; and
   in response to determining that the sensed electrical activity does not represent an RBB delay, increasing Vp output.
17. A method of using a pacemaker for His-bundle or bundle-branch pacing of a patient's heart, the method comprising:
   sensing electrical activity of the patient's heart including an atrial event (As) and a ventricular event (Vs);
   detecting a right bundle branch block (RBBB) pattern or left bundle branch block (LBBB) pattern in the sensed electrical activity of the patient's heart during the patient's intrinsic rhythm or during pacing; and
   adjusting the delivery of His-bundle or bundle-branch stimulation pulses (Vp) based on the detected bundle-branch-block pattern.
18. The method of clause 17, further comprising in response to detecting an RBBB pattern in the sensed electrical activity, adjusting an As-Vp interval.
19. The method of clause 17 or 18, further comprising in response to detecting an LBBB pattern in the sensed electrical activity, increasing Vp output.

## Claims

1. An implantable medical device for bundle-branch pacing of a patient's heart comprising:
a plurality of electrodes comprising a left bundle branch (LBB) cathode electrode positionable, when implanted, on a left side of the septum of the patient's heart proximate to the LBB, and a right bundle branch (RBB) cathode electrode positionable, when implanted, implantable on a right side of the septum of the patient's heart proximate to the RBB,
an electrical pulse generating circuit coupled to the plurality of electrodes to generate and deliver electrical bundle-branch stimulation pulses (Vp); and
a processor coupled to the electrical pulse generating circuit, wherein the processor is configured to control the electrical pulse generating circuit to deliver synchronized left and right bundle-branch Vp using the plurality of electrodes based on one or both of an atrial event (As) and a ventricular event (Vs).

2. The device of claim 1, further comprising a sensing circuit operably coupled to the processor and the plurality of electrodes to sense electrical activity of the patient's heart including As or Vs using at least one of the plurality of electrodes.

3. The device of claim 1 or 2, wherein the LBB cathode electrode does not pierce or extend into the left ventricle.

4. The device of any of the preceding claims, wherein the synchronized left and right bundle-branch Vp are delivered simultaneously.

5. The device of any of the preceding claims, wherein the synchronized left and right bundle-branch Vp are delivered sequentially or with a delay between the left bundle-branch Vp and the right bundle-branch Vp.

6. The device of any of the preceding claims, further comprising a medical electrical lead to deliver Vp, the medical electrical lead comprising a lead body, the plurality of electrodes disposed on the lead body to deliver pacing pulses within a single cardiac cycle.

7. The device of claim 6, wherein the lead body comprises a shaft extending longitudinally and a stopper protruding radially outward from the shaft configured to prevent the left bundle-branch electrode from perforating into the left ventricle.

8. The device of claim 6 or 7, wherein the plurality of electrodes comprises an atrial electrode coupled to the lead body and implantable in the patient's heart to sense As.

9. The device of claim 8, wherein the atrial electrode is spaced at least 5 cm from one of the bundle-branch cathode electrodes.

10. The device of any of the preceding claims, wherein the implantable medical device is a leadless pacing device (LPD) comprising a housing and at least one of the plurality of electrodes is leadlessly connected to the housing.

11. The device of claim 10, wherein the at least one electrode leadlessly connected to the housing is in the shape of a ring electrode.

12. The device of any of the preceding claims, wherein the implantable medical device is a leadless pacing device (LPD) comprising a housing and at least one of the plurality of electrodes is leadlessly connected to a piercing element extending axially from the housing.

13. The device of claim 12, further comprising a fixation element different than the piercing element extending axially from the housing.
